# EUROPEAN PATENT APPLICATION

(11) **EP 2 042 514 A1**
(43) Date of publication of application: **01.04.2009**
(21) Application number: 07018871.9
(22) Date of filing: 25.09.2007
(51) Int. Cl.: C07K 14/435

(54) **Maintenance of hair growth and treatment of hair-loss**

(71) Applicant: Life & Brain GmbH, 53127 Bonn (DE)
(72) Inventor: Betz, Regina, 53113 Bonn (DE); Nöthen, Markus, 53115 Bonn (DE); Pasternack, Sandra, 53111 Bonn (DE); Al Aboud, Khalid, M.D., P.O. Box 5440 Makkah (SA)
(74) Representative: Vossius & Partner

(57) **Abstract**

The present invention relates to a nucleic acid molecule, encoding a polypeptide having P2Y5 receptor function wherein said nucleic acid molecule comprises: (a) a nucleic acid molecule encoding a polypeptide having the amino acid sequence of SEQ ID NO:2; (b) a nucleic acid molecule having the DNA sequence of SEQ ID NO:1; (c) a nucleic acid molecule having the sequence of SEQ ID NO:1, wherein each thymidine is replaced by uridine; (d) a nucleic acid molecule that hybridizes under stringent conditions to the complementary strand of a nucleic acid molecule of (a), (b) or (c); (e) a nucleic acid molecule encoding a polypeptide having at least 80% sequence identity to the polypeptide of (a); or (f) a nucleic acid molecule that is degenerate with respect to the nucleic acid molecule of (b), (c) or (d); for the diagnosis, treatment and/or prevention of hair-loss and the diagnosis of a predisposition for hair-loss. Furthermore, the invention relates to compositions and uses for the diagnosis, treatment and/or prevention of hair-loss as well as to methods of identifying compounds useful in the treatment of hair-loss. The invention also relates to nucleic acid molecules carrying mutations that are causative and/or indicative of hair-loss, diagnostic compositions, kits and methods for testing for the presence of the mutant nucleic acid molecule.

## Description

The present invention relates to a nucleic acid molecule, encoding a polypeptide having P2Y5 receptor function wherein said nucleic acid molecule comprises: (a) a nucleic acid molecule encoding a polypeptide having the amino acid sequence of SEQ ID NO:2; (b) a nucleic acid molecule having the DNA sequence of SEQ ID NO:1; (c) a nucleic acid molecule having the sequence of SEQ ID NO:1, wherein each thymidine is replaced by uridine; (d) a nucleic acid molecule that hybridizes under stringent conditions to the complementary strand of a nucleic acid molecule of (a), (b) or (c); (e) a nucleic acid molecules encoding a polypeptide having at least 80% sequence identity to the polypeptide of (a); or (f) a nucleic acid molecule that is degenerate with respect to the nucleic acid molecule of (b), (c) or (d); for the diagnosis, treatment and/or prevention of hair-loss and the diagnosis of a predisposition for hair-loss. Furthermore, the invention relates to compositions and uses for the diagnosis, treatment and/or prevention of hair-loss as well as to methods of identifying compounds useful in the treatment of hair-loss. The invention also relates to nucleic acid molecules carrying mutations that are causative and/or indicative of hair-loss, diagnostic compositions, kits and methods for testing for the presence of the mutant nucleic acid molecule.

Throughout this specification, several documents are cited. The disclosure content of these documents is herewith incorporated by reference (including all product descriptions and manufacturers instructions).

G protein-coupled receptors (GPCRs) constitute a major class of proteins responsible for transducing a signal within a cell. GPCRs share a common structural organization characterized by an amino terminal extracellular domain, seven hydrophobic alpha helices putatively constituting transmembrane domains, three extracellular loops, three intracellular loops, and a carboxy terminal intracellular domain.
Upon binding of a ligand to an extracellular portion of a GPCR, a signal is transduced within the cell that results in a change in a biological or physiological property of the cell. GPCRs, along with heterotrimeric, guanine nucleotide binding G proteins and effectors (intracellular enzymes and channels modulated by G proteins), are the components of a modular signaling system that connects the state of intracellular second messengers to extracellular inputs.
G protein-coupled receptor proteins are present on the cell surface of each functional cell and organ in the body, and they play important physiological roles as targets of the molecules that regulate their functions, e.g., hormones, neurotransmitters, physiologically active substances and the like. GPCR gene-products and its ligands are related to the occurrence of numerous metabolic and genetic disorders. Mechanistically, approximately 50-60% of all clinically relevant drugs act by modulating the functions of various GPCRs.

The P2Y5 receptor is an orphan G protein-coupled receptor and contains seven predicted hydrophobic transmembrane regions, a structural feature of G protein-coupled receptors. P2Y5 was originally reported to bind extracellular nucleotides as ligands (T. E. Webb et al., Biochem Biophys Res Commun 219, 105 (1996)), however, further experiments were not able to substantiate that these extracellular nucleotides are indeed P2Y5 ligands (Q. Li et al., Biochem Biophys Res Commun 236, 455 (1997); I. von KOgelgen, Pharmacol Ther 110, 415 (2006)). The gene encoding the P2Y5 receptor, *P2RY5,* has been published under the accession number uc001 vcf.1 (database UCSC (University of California Santa Cruz), NCBI Build 36.1). It is localized in reverse orientation in intron 17 of the *RB1* (retinoblastoma) gene (Herzog et al., Genome Research 6, 858 (1996)). The gene consists of only one coding exon encoding 344 amino acids. Seven 5'-untranslated regions (UTR) and one 3'-untranslated region were given in the database (UCSC).

Hair loss is a common occurrence in humans and has a variety of causes. The causes include purely genetic factors, as in androgenetic alopecia, multifactorial factors i.e. genetic as well as external factors, as in alopecia areata, or mainly external factors as in drug-induced alopecia. Hair loss causes significant psychological distress in the majority of those affected. Currently available therapies are unsatisfactory and there is a demand for novel treatment strategies. A powerful approach to furthering our understanding of the pathophysiology of human hair loss is the identification of the genes underlying Mendelian isolated alopecias. investigation of this type of hair loss offers the unique opportunity of identifying factors that are not only necessary for, but also specific to hair growth.
A number of genes have already been identified using this approach.
Ahmad *et al.* identified a human homolog of the murine *hairless* gene on chromosome 8p12 (W. Ahmad et al., Science 279, 720 (1998). In individuals with alopecia universalis this gene was found to carry a missense mutation, which may affect the function of the gene, which encodes for a zinc finger transcription factor. Work by Levy-Nissenbaum *et al*. identified nonsense mutations in the gene encoding corneodesmosin (CDSN), a glycoprotein expressed in the epidermis and inner root sheath of hair follicles that act as a keratinocyte adhesion molecule (E. Levy-Nissenbaum et al., Nat Genet 34, 151 (2003)). These nonsense mutations result in premature stop codons in *CDSN* in individuals suffering from hypotrichosis simplex of the scalp, an autosomal dominant form of isolated alopecia, Kljuic *et al*. identified another gene, the cadherin family member *desmoglein 4*, that carries mutations in individuals with autosomal recessive hypotrichosis (LAH) (A. Kljuic et al., Cell 113, 249 (2003)).
Recent work by Kazantseva *et al.* identified the *lipase H* (*LIPH*) gene as a further gene involved in human hair growth and scalp hair loss (A. Kazantseva et al., Science 314, 982 (2006)) while work by Miller *et al.* suggests a role of the *vitamin D receptor* gene (J. Miller et al., J Invest Dermatol 117, 612 (20011)).
Despite significant progress in this research field, the complex pathophysiology of human hair growth is far from completely understood, and a major breakthrough in therapy has yet to be achieved.

The technical problem underlying the present invention was the provision of means and methods useful in the diagnosis of hair-loss or a predisposition thereto.

The solution to this problem is achieved by providing the embodiments as characterized in the claims.

Accordingly, the present invention relates to a nucleic acid molecule, encoding a polypeptide having P2Y5 receptor function wherein said nucleic acid molecule comprises (a) a nucleic acid molecule encoding a polypeptide having the amino acid sequence of SEQ ID NO:2; (b) a nucleic acid molecule having the DNA sequence of SEQ ID NO:1; (c) a nucleic acid molecule having the sequence of SEQ ID NO:1, wherein each thymidine is replaced by uridine; (d) a nucleic acid molecule that hybridizes under stringent conditions to the complementary strand of a nucleic acid molecule of (a), (b) or (c); (e) a nucleic acid molecule encoding a polypeptide having at least 80% sequence identity to the polypeptide of (a); or (f) a nucleic acid molecule that is degenerate with respect to the nucleic acid molecule of (b), (c) or (d); for the diagnosis, treatment and/or prevention of hair-loss and the diagnosis of a predisposition for hair-loss.

Nucleic acid molecules, in accordance with the present invention, include DNA, such as cDNA or genomic DNA, RNA (e.g. mRNA), also in synthetic or semisynthetic form, further synthetic or semisynthetic derivatives of DNA or RNA (e.g. PNA or phosphorothioates) and mixed polymers, both sense and antisense strands. They may contain additional non-natural or derivatized nucleotide bases, as will be readily appreciated by those skilled in the art. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art. For the purposes of the present invention, a peptide nucleic acid (PNA) is a polyamide type of DNA analog and the monomeric units for the derivatives of adenine, guanine, thymine and cytosine are available commercially (Perceptive Biosystems). Certain components of DNA, such as phosphorus, phosphorus oxides, or deoxyribose derivatives, are not present in PNAs. As disclosed by Nielsen et al., Science 254:1497 (1991); and Egholm et al., Nature 365:666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaiier probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

In a preferred embodiment the nucleic acid molecule is DNA.

The term "polypeptide", which is interchangeably used herein with the term "protein", in accordance with the present invention describes molecular chains of amino acids, including single chain proteins or their fragments, containing 30 or more amino acids. Preferably, these amino acid chains are linear. Polypeptides may further form multimers consisting of at least two identical or different molecules. The corresponding higher order structures of such multimers are correspondingly termed homo- or heterodimers, homo- or heterotrimers etc. Homodimers, trimers etc. of fusion proteins giving rise or corresponding to polypeptides of the present invention also fall under the definition of the term "polypeptide" or "protein". Those components of said fusion proteins, which are not P2Y5 protein sequences or fragments or variants thereof as defined herein above, include amino acid sequence which confer desired properties such as modified/enhanced stability, modified/enhanced solubility and/or the ability of targeting one or more specific cell types or could confer a different biological activity. For example, fusion proteins with antibodies specific for cell surface markers or with antigen-recognizing fragments of said antibodies are envisaged. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides/proteins where the modification is effected e.g. by glycosylation, acetylation, phosphorylation and similar modifications which are well known in the art.

It is also well known that polypeptides are not always entirely linear. For instance, polypeptides may be branched as a result of ubiquitination, and they may be circular, with or without branching, generally as a result of post-translation events, including natural processing event and events brought about by human manipulation which do not occur naturally. Circular, branched and branched circular polypeptides may be synthesized by non-translational natural processes and by synthetic methods.
The modifications can be a function of how the protein is made. For recombinant polypeptides, for example, the modifications will be determined by the host cells posttranslational modification capacity and the modification signals in the polypeptide amino acid sequence. Accordingly, when glycosylation is desired, a polypeptide should be expressed in a glycosylating host, generally an eukaryotic cell, for example Cos7, HELA or others. The same type of modification may be present in the same or varying degree at several sites in a given polypeptide. Also, a given polypeptide may contain more than one type of modification.

The term "P2Y5 receptor" in accordance with the present invention refers to the orphan G protein-coupled receptor (GPCR) P2Y5.
The coding sequence of the *P2RY5* gene is shown in SEQ ID NO:1 and the deduced 344 amino acid sequence is shown in SEQ ID NO:2. The genomic sequence of the *P2RY5* gene is represented in SEQ ID NO: 4 while the sequence corresponding the *P2RY5* mRNA (including 5' and 3' UTRs) is represented in SEQ ID NO:3. It is predicted that amino acids 1 to about 19 constitute the amino terminal extracellular domain, amino acids about 20-292 constitute the region spanning the transmembrane domain, and amino acids about 293-344 constitute the carboxy terminal intracellular domain. The transmembrane domain contains seven transmembrane segments, three extracellular loops and three intracellular loops. As used herein, the term "transmembrane segment" refers to a structural amino acid motif which includes a hydrophobic helix that spans the plasma membrane. The transmembrane segments are found from about amino acid 20 to about amino acid 46, from about amino acid 56 to about amino acid 79, from about amino acid 93 to about amino acid 113, from about amino acid 134 to about amino acid 154, from about amino acid 182 to about amino acid 209, from about amino acid 228 to about amino acid 253, and from about amino acid 273 to about amino acid 292. Within the region spanning the entire transmembrane domain are three intracellular and three extracellular loops. The three intracellular loops are found from about amino acid 47 to about amino acid 55, from about amino acid 114 to about amino acid 133, and from about amino acid 210 to about amino acid 227. The three extracellular loops are found at from about amino acid 80 to about amino acid 92, from about amino acid 155 to about amino acid 181, and from about amino acid 254 to about amino acid 272.
The nucleic acid molecules of any of (d) to (f) include nucleic acid molecules encoding polypeptides comprising or consisting of fragments of the amino acid sequence set forth in SEQ ID NO: 2. It is well known in the art that functional polypeptides may be cleaved to yield fragments with unaltered or substantially unaltered function. Such cleavage may include the removal of a given number of N-and/or C-terminal amino acids. Additionally or alternatively, a number of internal (non-terminal) amino acids may be removed, provided the obtained polypeptide has P2Y5 receptor activity. Said number of amino acids to be removed from the termini and/or internal regions may be one, two, three, four, five, six, seven, eight, nine, ten, 15, 20, 25, 30, 40, 50 or more than 50. Any other number between one and 50 is also deliberately envisaged. In particular, removals of amino acids which preserve sequence and boundaries of any conserved functional domain(s) or subsequences in the sequence of SEQ ID NO: 2 are particularly envisaged. Means and methods for determining such domains are well known in the art and include experimental and bioinformatic means. Experimental means include the systematic generation of deletion mutants and their assessment in assays for P2Y5 receptor activity known in the art and as described in the Examples enclosed herewith. Bioinformatic means include database searches. Suitable databases include protein sequence databases. In this case a multiple sequence alignment of significant hits is indicative of domain boundaries, wherein the domain(s) is/are comprised of the/those subsequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam).

Nucleic acid molecules of the invention comprising a nucleic acid molecule having the DNA sequence of SEQ ID NO:1 include, but are not limited to, nucleic acid molecules having the sequence of SEQ ID NO:1, nucleic acid molecules having the sequence of SEQ ID NO:1 and additional coding sequences, such as leader or secretory sequence as well as nucleic acid molecules having the sequence of SEQ ID NO:1 and additional non-coding sequences, e.g. 5' and 3' untranslated sequences (UTR) that are transcribed but not translated, such as for example SEQ ID NO:3. Such additional sequence may play a role in transcription, mRNA processing (including splicing and polyadenylation signals), ribosome binding and stability of mRNA. In addition, the nucleic acid molecule may be the entire genomic DNA representing the *P2RY5* gene (as represented in SEQ ID NO:4). Furthermore, the nucleic acid molecule may comprise the sequence of SEQ ID NO:1 fused to marker sequences encoding, for example, a peptide that facilitates purification of the encoded polypeptide, such as a V5- or poly-His-tag.

The term "hybridizes/hybridizing" as used herein refers to a pairing of a polynucleotide to a (partially) complementary strand of this polynucleotide which thereby form a hybrid.

It is well known in the art how to perform hybridization experiments with nucleic acid molecules. Correspondingly, the person skilled in the art knows what hybridization conditions she/he has to use to allow for a successful hybridization in accordance with item (i)(c), above. The establishment of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985).

"Stringent conditions" refers to hybridization conditions under which the polynucleotides that are capable of hybridizing to the polynucleotides of the invention or parts thereof hybridize to these target sequences to a detectably greater degree than to other sequences (e.g., at least 2-fold over background). Stringent conditions are sequence-dependent and will be different in different circumstances. By controlling the stringency of the hybridization and/or washing conditions, target sequences that have at least 90% sequence identity, more preferably 95%, such as 98% and more preferred 100% sequence identity to the probe can be identified (highly stringent hybridization conditions). Alternatively, stringency conditions can be adjusted to allow a higher degree of mismatching in sequences (low stringency conditions of hybridization). Such highly stringent and low stringent conditions for hybridization are well known to the person skilled in the art. For example, highly stringent conditions for hybridization comprise, e.g. an overnight incubation at 65°C in 4x SSC (600 mM NaCl, 60 mM sodium citrate) followed by washing at 65°C in 0.1x SSC for one hour. Alternatively, highly stringent hybridization conditions can comprise: an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing in e.g. 0.1-0.5x SSC at about 55-65°C for about 5 to 20 min. Also contemplated are nucleic acid molecules that hybridize to the polynucleotides of the invention at lower stringency hybridization conditions ("low stringency conditions for hybridization"). Changes in the stringency of hybridization are primarily accomplished through the manipulation of formamide concentration (lower percentages of formamide result in lowered stringency), salt conditions, or temperature. For example, lower stringency conditions include an overnight incubation at 50°C in 4x SSC or an overnight incubation at 37°C in a solution comprising 6x SSPE (20x SSPE = 3M NaCl; 0.2M NaH₂PO₄; 0.02M EDTA, pH 7.4), 0.5% SDS, 30% formamide, 100 mg/ml salmon sperm blocking DNA; followed by washes at 50°C with 1 x SSPE, 0.1% SDS. In addition, to achieve an even lower stringency, washes performed following stringent hybridization can be done at higher salt concentrations (e.g. 5x SSC). It is of note that variations in the above conditions may be accomplished through the inclusion and/or substitution of alternate blocking reagents. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. The embodiment recited herein above preferably refers to highly stringent conditions and alternatively to conditions of lower stringency. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed).

As stated herein above, preferred in accordance with the present invention are nucleic acid molecules which are capable of hybridizing to the nucleic acid molecules of the invention or parts thereof, under (highly) stringent hybridization conditions, i.e. which do not cross hybridize to nucleic acid molecules unrelated in nucleotide sequence. In accordance with item (d), above, nucleic acid molecules related but not identical in sequence with the nucleic acid molecules of items (a), (b) or (c) are also encompassed by the invention. In addition, the invention comprises according to item (d) fragments of the nucleic acid molecule of (a), (b) or (c). For all embodiments falling under item (d), it is essential in accordance with this embodiment, that the polypeptide encoded by this nucleic acid molecule retains or essentially retains P2Y5 receptor function.

In accordance with the present invention, P2Y5 receptor function is essentially retained, if at least 60% of the biological activity of the P2Y5 receptor activity are retained. Preferably, at least 75% or at least 80% of the P2Y5 receptor activity are retained. More preferred is that at least 90% such as at least 95%, even more preferred at least 98% such as at least 99% of the biological activity of the P2Y5 receptor are retained. Most preferred is that the biological activity is fully, i.e. to 100%, retained. Also in accordance with the invention are polypeptides having increased biological activity compared to P2Y5 receptor, i.e. more than 100% enzyme activity. Methods of assessing biological activity of a polypeptide are well known to the person skilled in the art and include without being limiting measuring receptor mediated changes in cAMP levels. Methods for determining cAMP levels are well known to the skilled person and are described, for example in George et al., Journal of 8iomolecular Screening 2, 235 (1997).

In accordance with the present invention the nucleic acid molecule can also encode a polypeptide having at least 80% sequence identity to the polypeptide of (a). More preferably, the nucleic acid molecule encodes a polypeptide that has at least 85%, even more preferably 90% sequence identity to the polypeptide of (a). Even more preferably the nucleic acid molecule can encode a polypeptide that has at least 95 sequence identity to the polypeptide of (a) and most preferably 98% sequence identity to the polypeptide of (a).

In accordance with the present invention, the term "% sequence identity" describes the number of matches ("hits") of identical nucleotides/amino acids of two or more aligned nucleic acid or amino acid sequences as compared to the number of nucleotides or amino acid residues making up the overall length of the nucleic acid or amino acid sequences (or the overall compared part thereof). In other terms, using an alignment, for two or more sequences or subsequences the percentage of amino acid residues or nucleotides that are the same (e.g., 80% or 85% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of a test sequence. Preferred nucleic acid molecules/polypeptides in accordance with the invention are those where the described identity exists over a region that is at least about 15 to 25 amino acids or nucleotides in length, more preferably, over a region that is at least about 50 to 100 amino acids or nucleotides in length. More preferred nucleic acid molecules/polypeptides in accordance with the present invention are those having the described sequence identity over the entire length of the nucleic acid molecule or polypeptide. Those having skill in the art will know how to determine percent sequence identity between/among sequences using, for example, algorithms such as those based on CLUSTALW computer program (Thompson Nucl. Acids Res. 2 (1994), 4673-4680) or FASTA (Pearson and Lipman, Proc. Natl. Acad. Sci., 1988, 85; 2444), as known in the art.
Although the FASTA algorithm typically does not consider internal non-matching deletions or additions in sequences, i.e., gaps, in its calculation, this can be corrected manually to avoid an overestimation of the % sequence identity. CLUSTALW, however, does take sequence gaps into account in its identity calculations. Also available to those having skill in this art are the BLAST and BLAST 2.0 algorithms (Altschul, Nucl. Acids Res., 1977, 25:3389). The BLASTN program for nucleic acid sequences uses as default a word length (W) of 11, an expectation (E) of 10, M=5, N=4, and a comparison of both strands. For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. All those programs may be used for the purposes of the present invention. Accordingly, all the nucleic acid molecules having the prescribed function and further having a sequence identity of at least 80% as determined with any of the above recited or further programmes available to the skilled person fall under the scope of the invention.

In accordance with the present invention the nucleic acid molecule encoding a polypeptide having P2Y5 receptor function may be degenerate with respect to the nucleic acid molecule of item (b), (c) or (d). When used in accordance with the present invention the term being "degenerate" means that due to the redundancy of the genetic code different nucleotide sequences code for the same amino acid.

The term "hair-loss" in accordance with the present invention and as used throughout the description relates to and includes all hair-loss conditions including, for example, androgenetic alopecia, alopecia areata, monogenic and syndromal forms of alopecia. Also included within the term "hair-loss" are all forms of hair-loss induced by, for example, drugs (chemotherapy) or mechanic stimuli.

Androgenetic alopecia, the most common form of hair-loss, is triggered by elevated androgene receptor level and by elevated dihydrotestosterone (DHT) level, a sex hormone, that can adversely affect the hair on the scalp. However, the mechanism by which DHT accomplishes this is not yet understood.

Alopecia areata (AA) is a common dermatologic disease with a lifetime prevalence of 1-2%, which causes round, patchy hair loss on the scalp. A classification in three subtypes is established referring to the amount of hair loss, ranging from patchy AA (one or more circumscribed patches of hair loss) to alopecia totalis (AT, 100 % loss of scalp hair without loss of body hair) and alopecia universalis (AU, 100 % loss of both scalp and body hair). The etiopathogenesis of AA is incompletely understood, but AA is thought to be a tissue-specific autoimmune disease directed against the hair follicle. Induced hair-loss refers to exogenic factors, as for example drugs (chemotherapy) or, mechanic stimuli.

Monogenic forms of hair-loss are characterised by a single gene being responsible for the pathogenic cause of hair loss. Examples include, but are not limited to, Hypotrichosis simplex of the scalp (HSS), hypotrichosis simplex, generalised form (HSG), alopecia universalis congenitalis, papular atrichia, hypotrichosis Marie Unna and monilethrix.

The present inventors surprisingly found that the orphan G protein coupled receptor P2Y5 mediates signalling pathways that are crucial for the maintenance of hair growth. Dysregulation of the P2Y5 receptor, on the other hand, was shown to be associated with diseases such as hair-loss. To the applicants best knowledge, these are the first data that implicate a G protein-coupled receptor as being essential for and specific to the maintenance of human hair growth. Due to these findings it is now possible to develop new diagnostic tools to predict an individuals predispositions for diseases mediated by the P2Y5 receptor as well as diagnostic and therapeutic approaches for individuals affected by diseases mediated by the P2Y5 receptor, in particular hair-loss.

Experimentally, using a genome-wide linkage-analysis, the present inventors identified a genetic defect underlying an autosomal-recessive mode of inherited hypotrichosis simplex, in a Saudi-Arabian family. After sequencing of various genes in the candidate region, a premature stop-codon in the G protein-coupled receptor P2Y5 was detected. When screening other hypotrichosis cases it was found that a different *P2RY5*-Mutation in two further HSG-families from Saudi-Arabia, enclosing one and two affected individuals respectively, also resulting in a truncated, non-functional protein. A number of diverse functional studies, such as expression-profiling in humans (cDNA panels (BD), multiple tissue expression human array3 (BD)), 5'RACE (Rapid amplification of 5' complementary DNA ends) and Northern Blotting (mouse RNA), confirmed these results. Furthermore, the proteins were analyzed by Western blot, protein truncation tests (The TNT^{®} T7 Quick Coupled Transcription/Translation System) and immunofluorescence experiments.

In preferred embodiments of the invention, the hair-loss is selected from the group consisting of hypotrichosis simplex of the scalp (HSS), hypotrichosis simplex, generalised form (HSG), alopecia universalis congenitalis, papular atrichia, hypotrichosis Marie Unna and monilethrix.

Hypotrichosis simplex (HS; MIM 146520 and MIM 605389) is a rare form of non-syndromic alopecia that affects men and women equally. In most families the disorder is inherited as an autosomal dominant trait, although autosomal recessive inheritance has also been observed. The extent of scalp and body hair involvement is variable. Some families experience hypotrichosis that is confined to the scalp (hypotrichosis simplex of the scalp, HSS) while others experience loss of body hair in addition to scalp hair loss (hypotrichosis simplex, generalized form, HSG). The hair loss is diffuse and usually begins in early childhood and progresses until adulthood. Hypotrichosis from birth has also been reported. In hypotrichosis, the hair shaft characteristically shows no gross abnormality.

In a preferred embodiment, the nucleic acid molecule of the invention is contained in a vector.

The term "vector" in accordance with the present invention refers to a vector that is a plasmid, cosmid, virus, bacteriophage or another vector. Such vectors may be used e.g. in genetic engineering. Incorporation of the nucleic acid into a vector offers the possibility of introducing the nucleic acid molecule efficiently into the cells and preferably the DNA of a recipient. The recipient may be a single cell such as a cell from a cell line. Such a measure renders it possible to express, when expression vectors are chosen, the respective nucleic acid molecule in the recipient. Thus, incorporation of the nucleic acid molecule into an expression vector opens up the way to a permanently elevated level of the encoded protein in any cell or a subset of selected cells of the recipient. In a preferred embodiment, the recipient is a mammal. In a more preferred embodiment, the mammal is a human.

The nucleic acid molecule may be inserted into several commercially available vectors. Non-limiting examples include vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), E80-pSV2neo, p8PV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen), pCINeo (Promega), Okayama-Berg cDNA expression vector pcDV1 (Pharmacia), pRc/CMV, pcDNA1, pSPORT1 (GIBCO BRL), pGEMHE (Promega), pSVL and pMSG (Pharmacia, Uppsala, Sweden), pRSVcat (ATCC 37152), pSV2dhfr (ATCC 37146), pBC12MI (ATCC 67109) or pcDNA3.1 (Invitrogen), preferably pcDNA3.1/V5/His or pcDNA5/FRT/TO (Invitrogen).

The nucleic acid molecule referred to above may also be inserted into vectors such that a translational fusion with another nucleic acid molecule is generated. The vectors may also contain an additional expressible polynucleotide coding for one or more chaperones to facilitate correct protein folding.

For vector modification techniques, see Sambrook and Russel ("Molecular Cloning, A Laboratory Manual", Cold Spring Harbor Laboratory, N.Y. (2001)). Generally, vectors can contain one or more origin of replication (ori) and inheritance systems for cloning or expression, one or more markers for selection in the host, e. g., antibiotic resistance, and one or more expression cassettes.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in eukaryotic cells are well known to those skilled in the art. These elements comprise regulatory sequences ensuring the initiation of the transcription (e. g. translation initiation codon, promoters, enhancers, and/or insulators), internal ribosomal entry sites (IRES) (Owens, Proc. Natl. Acad. Sci. USA 2001, 98: 1471) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule is operatively linked to such expression control sequences allowing expression in eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1a-promoter, CMV enhancer, CaM-kinase promoter, the Autographa californica multiple nuclear polyhedrosis virus (AcMNPV) polyhedral promoter or the SV40-enhancer. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide. Moreover, elements such as origin of replication, drug resistance genes, regulators (as part of an inducible promoter) may also be included. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter).

The co-transfection with a selectable marker such as dihydrofolate reductase (dhfr), gpt, neomycin, hygromycin or G418 allows the identification and isolation of the transfected cells. The transfected nucleic acid can also be amplified to express large amounts of the encoded (poly)peptide. The DHFR marker is useful to develop cell lines that carry several hundred or even several thousand copies of the gene of interest. Another useful selection marker is the enzyme glutamine synthase (GS) (Murphy et al., Biochem J. 1991, 227:277; Bebbington et al., Bio/Technology 1992, 10:169). Using these markers, the mammalian cells are grown in selective medium and the cells with the highest resistance are selected. As indicated above, the expression vectors will preferably include at least one selectable marker.

The nucleic acid molecules as described herein may be designed for direct introduction or for introduction via liposomes, phage vectors or viral vectors (e.g. adenoviral, retroviral) into the cell. Additionally, baculoviral systems or systems based on vaccinia virus or Semliki Forest virus can be used as eukaryotic expression system for the nucleic acid molecules of the invention.

In a further preferred embodiment, the nucleic acid molecule of the invention, which is contained in a vector, is contained in a host cell.

The term "host cell" in accordance with the present invention refers to a host cell that may be produced by introducing said vector into a host cell, which upon its presence mediates the expression of the nucleic acid molecule of the invention.

The host cell may be any prokaryote or eukaryotic cell. Suitable prokaryotes/bacteria are those generally used for cloning like *E*. *coli* (e.g., *E coli* strains BL21(DE3), HB101, DH5α, XL1 Blue, Y1090 and JM101), *Salmonella typhimurium*, *Serratia marcescens, Pseudomonas putida*, *Pseudomonas fluorescens*, *Streptomyces lividans*, *Lactococcus lactis*, *Mycobacterium smegmatis* or *Bacillus subtilis.* A suitable eukaryotic host cell may be an animal cell such as a mammalian cell, an amphibian cell, a fish cell, an insect cell such as Drosophila S2 and Spodoptera Sf9 cells, a fungal cell or a plant cell. Mammalian host cells that could be used include, human Hela, HEK293, H9, Bowes melanoma cells and Jurkat cells, mouse NIH3T3 and C127 cells, Cos 1, Cos 7 and CV1, quail QC1-3 cells, mouse L cells and Chinese hamster ovary (CHO) cells. Also within the scope of the present invention are primary mammalian cells such as the immortalised keratinocyte cell line HaCaT, primary keratinocytes, primary hair folllicle cells or mouse embryonic fibroblasts (MEF). Appropriate culture mediums and conditions for the above-described host cells are well known in the art.

The present invention also relates to a polypeptide encoded by the nucleic acid molecule described above for the treatment and/or prevention of hair-loss.

As described above, the present inventors surprisingly found that the P2Y5 receptor piays a crucial role in maintenance of hair growth and that the dysfunction of this G protein-coupled receptor is one reason for hair-loss in humans. Thus, the polypeptide encoded by the above-described nucleic acid molecule may be used for the treatment and/or prevention of hair-loss. For example, administering the polypeptide of the invention as therapy to compensate for reduced or aberrant expression or activity of the protein may be useful in the treatment and/or prevention of hair-loss.

The invention further relates to a pharmaceutical composition comprising (i) a nucleic acid molecule encoding a polypeptide having P2Y5 receptor function wherein said nucleic acid molecule comprises (a) a nucleic acid molecule encoding a polypeptide having the amino acid sequence of SEQ ID NO:2; (b) a nucleic acid molecule having the DNA sequence of SEQ ID NO:1; (c) a nucleic acid molecule having the sequence of SEQ ID NO:1, wherein each thymidine is replaced by uridine; (d) a nucleic acid molecule that hybridizes under stringent conditions to the complementary strand of a nucleic acid molecule of (a), (b) or (c); (e) a nucleic acid molecule encoding a polypeptide that is at least 80% homologous to the polypeptide of (a); or (f) a nucleic acid molecule that is degenerate with respect to the nucleic acid molecule of (b), (c) or (d); (ii) a vector comprising the nucleic acid molecule of (i); (iii) a host cell comprising the vector of (ii); or (iv) a polypeptide encoded by the nucleic acid molecule of (i).

In accordance with the present invention, the term "pharmaceutical composition" relates to a composition for administration to a patient, preferably a human patient. The pharmaceutical composition of the invention comprises at least one of the compounds recited above. It may, optionally, comprise further molecules capable of altering the characteristics of the compounds of the invention thereby, for example, suppressing, stabilizing, blocking, modulating and/or activating their function. The composition may be in solid, liquid or gaseous form and may be, inter alia, in the form of (a) powder(s), (a) tablet(s), (a) solution(s) or (an) aerosol(s).The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g per day. However, a more preferred dosage might be in the range of 0.01 mg to 100 mg, even more preferably 0.01 mg to 50 mg and most preferably 0.01 mg to 10 mg per day.
The components of the pharmaceutical composition to be used for therapeutic administration must be sterile. Sterility is readily accomplished, e.g. by filtration through sterile filtration membranes (e.g., 0.2 micron membranes).
The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.
Preservatives and other additives may also be present such as, for example, antimicrobials, antioxidants, chelating agents, and inert gases and the like. Furthermore, the pharmaceutical composition may comprise further agents depending on the intended use of the pharmaceutical composition.

The components of the pharmaceutical composition as outlined in (i) to (iv) are as defined above.
The pharmaceutical composition may be particularly useful for the treatment or prevention of diseases associated with a dysfunction of the P2Y5 receptor, preferably diseases selected from those described herein above. As described hereinabove, P2Y5 receptor protein, or its corresponding nucleic acid molecule, plays a crucial role in the maintenance of hair growth while dysfunction of this G protein-coupled receptor gene is one reason for hair-loss in humans. Therefore, the pharmaceutical compositions of the invention offer the possibility to specifically target hair-loss in individuals suffering from these conditions. For example, overexpression of the nucleic acid molecules of the invention may be useful for ,,gene targeting" and/or "gene replacement" for restoring the mutant gene in affected individuals while the provision of the polypeptide of the invention may be used to compensate for reduced or aberrant expression or activity of the protein.

The invention further relates to a method of treating and/or preventing hair-loss comprising administering the pharmaceutical composition described above to a subject in need thereof.

In another embodiment, the present invention relates to the use of (i) a nucleic acid molecule encoding a polypeptide having P2Y5 receptor function wherein said nucleic acid molecule comprises (a) a nucleic acid molecule encoding a polypeptide having the amino acid sequence of SEQ ID NO:2; (b) a nucleic acid molecule having the DNA sequence of SEQ ID NO:1; (c) a nucleic acid molecule having the sequence of SEQ ID NO:1, wherein each thymidine is replaced by uridine; (d) a nucleic acid molecule that hybridizes under stringent conditions to the complementary strand of a nucleic acid molecule of (a), (b) or (c); (e) a nucleic acid molecule encoding a polypeptide that is at least 80% homologous to the polypeptide of (a); or (f) a nucleic acid molecule that is degenerate with respect to the nucleic acid molecule of (b), (c) or (d); (ii) a vector comprising the nucleic acid molecule of (i); (iii) a host cell comprising the vector of (ii); or (iv) a polypeptide encoded by the nucleic acid molecule of (i) for the manufacture of a pharmaceutical composition for treating and/or preventing hair-loss.
The components for use for the manufacture of a pharmaceutical composition for treating and/or preventing hair-loss are as defined above.

The present invention also relates to a method for the identification of a compound useful in the treatment of hair-loss or as a lead compound for the development of an agent for treating hair-loss comprising the steps: (i) determining the level of P2Y5 receptor protein or *P2RY5* transcript in a cell wherein said cell comprises *P2RY5* DNA in expressible form; (ii) contacting said cell with a test compound; (iii) determining the level of P2Y5 receptor protein or *P2RY5* transcript in said cell after contacting with the test compound; and (iv) comparing the P2Y5 receptor protein or *P2RY5* transcript level determined in step (iii) with the P2Y5 receptor protein or *P2RY5* transcript level determined in step (i), wherein an increase of P2Y5 receptor protein or *P2RY5* transcript level in step (iii) as compared to step (i) indicates that the test compound is a compound useful in the treatment of hair-loss or as a lead compound for the development of an agent for treating hair-loss.

A "compound" in accordance with the present invention is, for example, a small molecule. Such a small molecule may be, for example, an organic molecule. Organic molecules relate to or belong to the class of chemical compounds having a carbon basis, the carbon atoms linked together by carbon-carbon bonds. The original definition of the term organic related to the source of chemical compounds, with organic compounds being those carbon-containing compounds obtained from plant or animal or microbial sources, whereas inorganic compounds were obtained from mineral sources. Organic compounds can be natural or synthetic. Alternatively the compound may be an inorganic compound. Inorganic compounds are derived from mineral sources and include all compounds without carbon atoms (except carbon dioxide, carbon monoxide and carbonates).
Test compounds include but are not limited to, for example, purine analogs, peptides such as soluble peptides, including Ig-tailed fusion peptides and members of random peptide libraries (see, e.g., Lam et al. (1991) Nature 354: 82-84; Houghten et al. (1991) Nature 354: 84-86) and combinatorial chemistry-derived molecular libraries made of D-and/or L-configuration amino acids; phosphopeptides (e.g., members of random and partially degenerate, directed phosphopeptide libraries, see, e.g., Songyang et al. (1993) Cell 72: 767-778); antibodies (e.g., polyclonal, monoclonal, humanized, anti-idiotypic, chimeric, and single chain antibodies as well as Fab, F (ab') 2, Fab expression library fragments, and epitope-binding fragments of antibodies).
One example for a test compound is a soluble full-length receptor or fragment that competes for ligand binding. Other test compounds include mutant receptors or appropriate fragments containing mutations that affect receptor function and thus compete for ligand. Accordingly, a fragment that competes for a ligand, for example with a higher affinity, or a fragment that binds a ligand but does not allow release, is encompassed by the invention.

The term *"P2RY5* DNA in expressible form" refers to *P2RY5* DNA under the control of its naturally occurring regulatory sequences. For example, the *P2RY5* DNA may be under the control of its natural promoter. Furthermore, *P2RY5* DNA under the control of the fragments constituting the minimal promoter of *P2RY5* is also envisaged by the invention.

As described hereinabove, the P2Y5 receptor is involved in processes that regulate the maintenance of hair growth, while dysfunction of this G protein-coupled receptor is one reason for hair-loss in humans. Therefore, the use of P2Y5 receptor as a target for the discovery of compounds that modulate processes of hair-loss is also encompassed by the present invention. It is envisaged that an increase of expression levels of *P2RY5* conferred by a compound as described above may contribute to the maintenance of hair growth and may ameliorate conditions associated with dysfunction of this receptor, such as hair-loss. Accordingly, measurement of the P2Y5 receptor protein or *P2RY5* transcript level may be used to determine the readout of the above-described assay.

The level of P2Y5 receptor protein or *P2RY5* transcript may e.g. be undetectable before contacting the above-mentioned cell with the test compound and it may be clearly detectable after contacting the cell with the test compound indicating a compound suitable for the treatment of hair-loss or as a lead compound for the development of a compound for the treatment of hair-loss. Alternatively, the above-mentioned cell may exhibit a detectable level of P2Y5 receptor protein or *P2RY5* transcript before contacting with the test compound and the level of P2Y5 receptor protein or *P2RY5* transcript may be higher after contacting the cell with the test compound. Preferably, the level of P2Y5 receptor protein or *P2RY5* transcript is for example at least 5, 10, 20, 30, 40 or 50% higher after contacting the cell with the test compound. More preferably, the level of P2Y5 receptor protein or *P2RY5* transcript is for example at least 100, 200, 300 or 400% higher after contacting the cell with the test compound. Most preferably, the level of P2Y5 receptor protein or *P2RY5* transcript is for example at least 500% higher after contacting the cell with the test compound.

Measurements of protein levels can be accomplished in several ways. Western blotting using specific antibodies or polyacrylamide gel electrophoresis (PAGE) in conjunction with protein staining techniques such as, but not limited to, Coomassie Brilliant blue or silver-staining may be used. Also of use in protein quantification is the Agilent Bioanalyzer technique.

Techniques for the determination of the transcript level include, but are not limited to RT-PCR and its various modifications such as qRT-PCR (also referred to as Real Time RT-PCR). PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and stalls all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl₂, 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus, Amplitaq, Pfu and KOD, some of which may exhibit proof-reading function and/or different temperature optima. The person skilled in the art knows how to optimize PCR conditions for the amplification of specific nucleic acid molecules with primers of different length and/or composition or to scale down or increase the volume of the reaction mix.
The "reverse transcriptase polymerase chain reaction" (RT-PCR) is used when the nucleic acid to be amplified consists of RNA. The term "reverse transcriptase" refers to an enzyme that catalyzes the polymerization of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus (AMV) reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. High-temperature RT provides greater primer specificity and improved efficiency. U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20µl reaction mix containing: 4 µl of 5x AMV-RT buffer, 2 µl of Oligo dT (100 µg/ml), 2µl of 10 mM dNTPs, 1µl total RNA, 10 Units of AMV reverse transcriptase, and H₂O to 20µl final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample. Any of the above mentioned reaction conditions may be scaled up according to the needs of the particular case.

Real-time PCR employs a specific probe, in the art also referred to as TaqMan probe, which has a reporter dye covalently attached at the 5' end and a quencher at the 3' end. After the TaqMan probe has been hybridized in the annealing step of the PCR reaction to the complementary site of the polynucleotide being amplified, the 5' fluorophore is cleaved by the 5' nuclease activity of Taq polymerase in the extension phase of the PCR reaction. This enhances the fluorescence of the 5' donor which was formerly quenched due to the close proximity to the 3' acceptor in the TaqMan probe sequence. Thereby, the process of amplification can be monitored directly and in real time, which permits a significantly more precise determination of expression levels than conventional end-point PCR. Also of use in Real time RT-PCR experiments is a DNA intercalating dye such as SybrGreen for monitoring the de novo synthesis of double stranded DNA molecules.

In a preferred embodiment, the method is carried out in vitro.

In vitro methods offer the possibility of establishing high-throughput assays which are capable of screening up to several thousand compounds in parallel. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to said activity.

The identified so-called lead compounds may be optimized to arrive at a compound which may be used in a pharmaceutical composition. Methods for the optimization of the pharmacological properties of compounds identified in screens, the lead compounds, are known in the art and comprise methods of codifying a compound identified as a lead compound to achieve: (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmacokinetic parameters (resorption, distribution, metabolism and excretion), and/or (vii) modified physicochemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carboxylic acids, or (iii) esterification of hydroxyl groups to, e.g. phosphates, pyrophosphates or sulfates or hemi-succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or (xvi) transformation of ketones or aldehydes to Schiff's bases, oximes, acetales, ketales, enolesters, oxazolidines, thiazolidines or combinations thereof.

The various steps recited above are generally known in the art. They include or rely on quantitative structure-activity relationship (QSAR) analyses (Kubinyi, "Hausch-Analysis and Related Approaches", VCH Verlag, Weinheim, 1992), combinatorial biochemistry, classical chemistry and others (see, for example, Holzgrabe and Bechtold, Deutsche Apotheker Zeitung 2000, 140(8): 813).

Any of the cells described herein above may be used for the method of the invention. Preferably, said cell is a HaCaT cell (immortalized keratinocyte cell line), primary keratinocyte or primary hair folllicle cell.

In another preferred embodiment of the method of the invention said cell comprises a nucleic acid molecule encoding a protein having P2Y5 receptor function fused to a reporter gene, wherein said nucleic acid molecule comprises (a) a nucleic acid molecule encoding a polypeptide having the amino acid sequence of SEQ ID NO:2; (b) a nucleic acid molecule having the DNA sequence of SEQ ID NO:1; (c) a nucleic acid molecule having the sequence of SEQ ID NO:1, wherein each thymidine is replaced by uridine; (d) a nucleic acid molecule that hybridizes under stringent conditions to the complementary strand of a nucleic acid molecule of (a), (b) or (c); (e) a nucleic acid molecule encoding a polypeptide having at least 80% sequence identity to the polypeptide of (a); or (f) a nucleic acid molecule that is degenerate with respect to the nucleic acid molecule of (b), (c) or (d).

The fusion to a reporter gene allows the indirect measurement of protein level by measuring the level of the reporter gene attached to the protein of interest. Examples of reporter genes include, but are not limited to, green fluorescent protein (GFP), yellow fluorescent protein (YFP), red fluorescent protein (RFP), cyan fluorescent protein (CFP), luciferase or p-galactosidase.

In an alternative embodiment, the present invention relates to a method for the identification of a compound useful in the treatment of hair-loss or as a lead compound for the development of an agent for treating hair-loss comprising the steps: (i) contacting a cell containing P2Y5 receptor protein and a P2Y5 target molecule with a test compound; and (ii) determining the level of activity of the P2Y5 target molecule before contacting the P2Y5 protein with the test compound and after contacting the P2Y5 protein with the test compound, wherein a modulation of activity of the target molecule after contacting the P2Y5 protein with the test compound as compared to the level before contacting the P2Y5 protein with the test compound indicates that the test compound is a compound useful in the treatment of hair-loss or as a lead compound for the development of an agent for treating hair-loss.

In a preferred embodiment of the method of the invention, the compound modulates P2Y5 receptor activity.

Such compounds may, for example, alter the affinity or rate of binding to a known ligand, compete with a ligand for binding to the receptor, or displace a ligand bound to the receptor.

The term "P2Y5 target molecule" refers to molecules that are affected by P2Y5 receptor activity as a result of the downstream signalling of this G-protein coupled receptor. Downstream signalling refers to the modulation (e.g., stimulation or inhibition) of a cellular function/activity upon binding of a ligand to the GPCR. Examples of such functions include mobilization of intracellular molecules that participate in a signal transduction pathway, for example cAMP or adenylate cyclase or that result in a change of intracellular molecules, for example release of calcium, polarisation of the plasma membrane, production or secretion of molecules, alteration in the structure of a cellular component, cell proliferation, cell differentiation and cell survival. The response mediated by the receptor protein is cell type dependent and may, for example, stimulate an activity such as release of compounds, gating of a channel, cellular adhesion, migration, differentiation, etc., through cyclic AMP metabolism and turnover while in other cells, the binding of the ligand will produce a different result.
The P2Y5 target molecule can be a ligand or a component of the signal pathway with which the receptor protein normally interacts, for example, a G-protein or other interactor involved in cAMP or phosphatidylinositol turnover and/or adenylate cyclase, or phospholipase C activation.
The assay includes the steps of combining the receptor protein with a test compound under conditions that allow the receptor protein to interact with the P2Y5 target molecule, and to detect the formation of a complex between the protein and the target or to detect the biochemical consequence of the interaction with the receptor protein and the target, such as any of the associated effects of signal transduction such as G-protein phosphorylation, cyclic AMP or phosphatidylinositol turnover, and adenylate cyclase or phospholipase C activation.
Furthermore, assays determining the expression of genes that are up- or downregulated in response to the receptor protein dependent signal cascade can be employed. For example, the regulatory region of such genes may be operably linked to a marker that is easily detectable, such as for example luciferase, green fluorescent protein (GFP), yellow fluorescent protein (YFP), red fluorescent protein (RFP), cyan fluorescent protein (CFP) or β-gaiactosidase. Alternatively, phosphorylation of the receptor protein, or a receptor protein target, may also be measured.

In a preferred embodiment of the method of the invention, the P2Y5 target molecule is cAMP.

Intracellular levels of cAMP can be determined using methods well known in the art, for example using Flash-plate kits (Dupont) or the Biotrak cAMP SPA screening assay system (Amersham) according to the manufacturer's instructions (George et al., Journal of Biomolecular Screening 2, 4:235 (1997)).

Binding and/or activating compounds can also be screened by using chimeric receptor proteins in which the amino terminal extracellular domain, or parts thereof, the entire transmembrane domain or subregions, such as any of the seven transmembrane segments or any of the intracellular or extracellular loops and the carboxy terminal intracellular domain, or parts thereof, can be replaced by heterologous domains or subregions. For example, a G protein-binding region can be used that interacts with a different G protein than that which is recognized by the native receptor. Accordingly, a different set of signal transduction components is available as an end-point assay for activation. Alternatively, the entire transmembrane portion or subregions (such as transmembrane segments or intracellular or extracellular loops) can be replaced with the entire transmembrane portion or subregions specific to a host cell that is different from the host cell from which the amino terminal extracellular domain and/or the G protein-binding region are derived. This allows for assays to be performed in other than the specific host cell from which the receptor is derived. Alternatively, the amino terminal extracellular domain (and/or other ligand-binding regions) could be replaced by a domain (and/or other binding region) binding a different ligand, thus, providing an assay for test compounds that interact with the heterologous amino terminal extracellular domain (or region) but still cause signal transduction. Finally, activation can be detected by a reporter gene containing an easily detectable coding region operably linked to a transcriptional regulatory sequence that is part of the native signal transduction pathway.

The receptor polypeptides are further useful in competition binding assays in methods designed to discover compounds that interact with the receptor. Thus, a compound is exposed to a receptor polypeptide under conditions that allow the compound to bind or to otherwise interact with the polypeptide. Soluble receptor polypeptide is also added to the mixture. If the test compound interacts with the soluble receptor polypeptide, it decreases the amount of complex formed or activity from the receptor target. This type of assay is particularly useful in cases in which compounds are sought that interact with specific regions of the receptor. Thus, the soluble polypeptide that competes with the target receptor region is designed to contain peptide sequences corresponding to the region of interest.

The P2Y5 receptor is, as described above, involved in processes that regulate the maintenance of hair growth and dysfunction of this G protein-coupled receptor is one reason for hair-loss in humans. Therefore, its use as a target for the discovery of compounds that increase the activity of P2Y5 is also envisaged. P2Y5 protein is useful, as has been surprisingly found in accordance with the present invention, for the prevention of hair-loss and therefore, increase of its activity by the use of compounds identified in the above-described screen will result in amelioration or even loss of symptoms associated with hair-loss.

In a further embodiment, the present invention relates to a nucleic acid molecule deviating from the nucleic acid molecules described above by at least one mutation, wherein said mutation results in a loss of function of the polypeptide encoded by the nucleic acid molecule described above and is selected from: (i) a substitution; (ii) a deletion; (iii) an inversion; and/or (iv) an insertion; and wherein said mutation is causative and/or indicative of hair-loss.

The term "mutation" in accordance with the invention refers to changes to the nucleotide sequence of the genetic material and includes deletion, insertion, or substitution of one or more nucleotides in the gene, chromosomal rearrangement, such as inversion or transposition, modification of genomic DNA, such as aberrant methylation patterns or changes in gene copy number, such as amplification.

The "nucleic acid molecule deviating from the nucleic acid molecules described above by at least one mutation" in accordance is also referred to herein as the "mutant nucleic acid molecule".

The term "substitution" in accordance with the present invention, refers to point mutations preferably resulting in an amino acid exchange.

The term "deletion" as used in accordance with the present invention refers to the loss of nucleotides.

The term "inversion" in accordance with the present invention refers to a kind of mutation in which the order of the nucleotides in a section of the nucleic acid molecule is reversed with respect to the remainder of the nucleic acid molecule.

The term "insertion" in accordance with the present invention refers to the addition of one or more nucleotides to a nucleic acid molecule, wherein the addition is not to the 5' or 3' end of the nucleic acid molecule.

All of the above mutations may lead to the creation of nonsense codons, i.e. stop codons, which will lead to premature termination of translation and, thus, to truncated forms of the polypeptide of the present invention. Furthermore, such mutations, in particular deletions or insertions, may lead to a frameshift mutation, causing all of the codons occurring after the mutation to be read incorrectly during translation. This frameshift may result in the production of a severely altered and potentially nonfunctional protein.
Especially with respect to substitutions and deletions, it could be shown in accordance with the present invention that such mutations may lead to a frame shift which in turn leads to the expression of a truncated form of the polypeptide of the present invention due to the generation of a premature stop codon.

Thus, detection of a mutated form of the *P2RY5* gene associated with a dysfunction of the P2Y5 receptor protein provides a diagnostic tool for diseases or susceptibilities to diseases related to P2Y5 receptor activity, in particular the diseases cited herein above. Furthermore, the nucleic acid molecule of the invention may also be used for testing an individual for a genotype that, while not necessarily causing the disease, nevertheless affects the treatment modality. Thus, the polynucleotides can be used to study the relationship between an individual's genotype and the individual's response to a compound used for treatment (pharmacogenomic relationship). In the present case, for example, a mutation in the receptor gene that results in altered affinity for ligand could result in an excessive or decreased drug effect with standard concentrations of ligand that activates the receptor. Accordingly, the nucleic acid molecules of the invention can be used to assess the mutation content of the receptor gene in an individual in order to select an appropriate compound or dosage regimen for treatment.

In a preferred embodiment of the present invention, said substitution is a cytosine to thymidine exchange at a nucleotide position corresponding to position 463 of the nucleotide sequence of SEQ ID NO:1. The nucleic acid molecule having this cytosine to thymidine exchange is shown in SEQ ID NO:5.

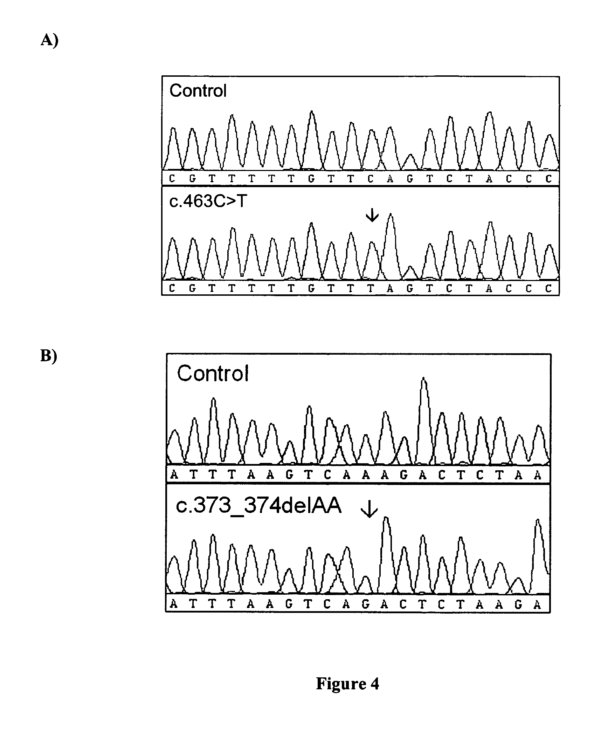

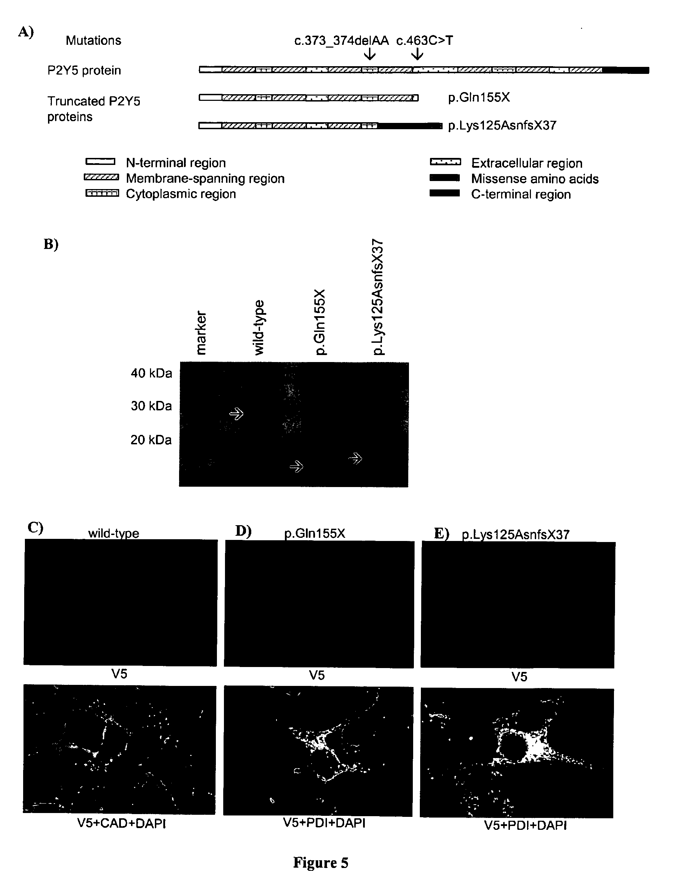

As shown in the examples, direct sequencing of *P2RY5* in individuals from a Saudi-Arabian family suffering from autosomal-recessive hypotrichosis simplex (family 1) revealed a substitution of a cytosine to thymidine at the nucleotide position 463 of *P2RY5* (c.463C>T; according to position 463 of SEQ ID NO:1), which results in a nonsense mutation leading to premature termination of translation (p.Gln155X; SEQ ID NO:6) (Figs. 4A and 5A). This mutation was not detected in 506 control chromosomes which included 138 chromosomes of Arabian origin.

In another preferred embodiment of the present invention, said deletion is a deletion of the adenosine at a nucleotide position corresponding to position 373 of the nucleotide sequence of SEQ ID NO:1 and/or a deletion of the adenosine at a nucleotide position corresponding to position 374 of the nucleotide sequence of SEQ ID NO:1. The nucleic acid molecule having this deletion at position 373 and 374 is shown in SEQ ID NO:7.

Sequencing of *P2RY5* in two additional HS-families from Saudi-Arabia (families 2 and 3) revealed the deletion of two adenosines from position 373 and 374 of *P2RY5* (c.373_374delAA; corresponding to the positions in SEQ ID NO:1) leading to a frame-shift and premature termination of translation of the P2Y5 receptor protein (p.Lys125AsnfsX37; SEQ ID NO:8) (Figs. 4B and 5A). Also this mutation was not detected in 506 control chromosomes which included 138 chromosomes of Arabian origin.

In a further embodiment, the invention also relates to a vector comprising the nucleic acid molecule of the invention as described above.

The term "vector" in accordance with the present invention has been described above.

In a further embodiment, the present invention also relates to a host genetically engineered with the nucleic acid molecule of the invention or with the vector comprising the nucleic acid molecule of the invention as described above. Said host may be produced by introducing said vector into a host which upon its presence mediates the expression of the polypeptide.

The host may be any prokaryote or eukaryotic cell. Suitable prokaryotes/bacteria are those generally used for cloning like *E*. *coli* (e.g., *E coli* strains BL21(DE3), HB101, DH5α, XL1 Blue, Y1090 and JM101), *Salmonella typhimurium*, *Serratia marcescens, Pseudomonas putida*, *Pseudomonas fluorescens, Streptomyces lividans, Lactococcus lactis*, *Mycobacterium smegmatis* or *Bacillus subtilis.* As described above, suitable eukaryotic hosts may be animal cells such as mammalian cells, amphibian cells, fish cells, insect cells such as Drosophila S2 and Spodoptera Sf9 cells, fungal cells, plant cells, transgenic non-human animals or transgenic plants.

A method for the production of a transgenic non-human animal, for example transgenic mouse, comprises introduction of the aforementioned nucleic acid molecule or targeting vector into a germ cell, an embryonic cell, stem cell or an egg or a cell derived thereof. The non-human animal can be used in accordance with the invention in a method for identification of compounds, described herein above in connection with cells. Production of transgenic embryos and screening of those can be performed, e.g., as described by A. L. Joyner Ed., Gene Targeting, A Practical Approach (1993), Oxford University Press. The DNA of the embryonic membranes of embryos can be analyzed using, e.g., Southern blots with an appropriate probe; see supra. A general method for making transgenic non-human animals is described in the art, see for example WO 94/24274. For making transgenic non-human organisms (which include homologously targeted non-human animals), embryonal stem cells (ES cells) are preferred. Murine ES cells, such as AB-1 line grown on mitotically inactive SNL76/7 cell feeder layers (McMahon and Bradley, Cell 62:1073-1085 (1990)) essentially as described (Robertson, E. J. (1987) in Teratocarcinomas and Embryonic Stem Cells: A Practical Approach. E. J. Robertson, ed. (Oxford: IRL Press), p. 71-112) may be used for homologous gene targeting. Other suitable ES lines include, but are not limited to, the E14 line (Hooper et al., Nature 326:292-295 (1987)), the D3 line (Doetschman et al., J. Embryol. Exp. Morph. 87:27-45 (1985)), the CCE line (Robertson et al., Nature 323:445-448 (1986)), and the AK-7 line (Zhuang et al., Cell 77:875-884 (1994)). The success of generating a mouse line from ES cells bearing a specific targeted mutation depends on the pluripotence of the ES cells (i.e., their ability, once injected into a host developing embryo, such as a blastocyst or morula, to participate in embryogenesis and contribute to the germ cells of the resulting animal). The blastocysts containing the injected ES cells are allowed to develop in the uteri of pseudopregnant non-human females and are born, e.g. as chimeric mice. The resultant transgenic mice are chimeric for cells having either the recombinase or reporter loci and are backcrossed and screened for the presence of the correctly targeted transgene(s), usually by PCR or Southern blot analysis on tail biopsy DNA of offspring so as to identify transgenic mice heterozygous for either the recombinase or reporter locus/loci.

The transgenic non-human animals may, for example, be transgenic mice, rats, hamsters, dogs, monkeys, rabbits, pigs, or cows. Preferably, said transgenic non-human animal is a mouse.

In an additional embodiment, the present invention relates to a method of producing a poiypeptide of the invention comprising culturing a host according to the invention under suitable conditions and isolating the polypeptide of the invention produced from said host or culture.

A large number of suitable methods exist in the art to produce polypeptides in appropriate hosts. If the host is a unicellular organism such as a prokaryote, a mammalian or insect cell, the person skilled in the art can revert to a variety of culture conditions. Conveniently, the produced protein is harvested from the culture medium, lysates of the cultured organisms or from isolated (biological) membranes by established techniques. In the case of a multicellular organism, the host may be a cell which is part of or derived from a part of the organism, for example said host cell may be the harvestable part of a plant. A preferred method involves the recombinant production of protein in hosts as indicated above. For example, nucleic acid sequences comprising the nucleic acid molecule according to the invention can be synthesized by PCR and inserted into an expression vector. Subsequently a suitable host may be transformed with the expression vector. Thereafter, the host is cultured to produce the desired polypeptide, which is isolated and purified. Such methods are well known in the art (see, e.g., Sambrook and Russel "Molecular Cloning, A Laboratory Manual", Cold Spring Harbor Laboratory, N.Y. (2001)).
An alternative method for producing the polypeptide of the invention is in vitro translation of mRNA. Suitable cell-free expression systems for use in accordance with the present invention include rabbit reticulocyte lysate, wheat germ extract, canine pancreatic microsomal membranes, *E*. *coli* S30 extract, and coupled transcription/translation systems such as the TNT-system (Promega). Theses systems allow the expression of recombinant polypeptides upon the addition of cloning vectors, DNA fragments, or RNA sequences containing coding regions and appropriate promoter elements.

In addition to recombinant production, fragments of the protein or the fusion protein of the invention may e.g. be produced by direct peptide synthesis using solid-phase techniques (cf Stewart et al. (1969) Solid Phase Peptide Synthesis; Freeman Co, San Francisco; Merrifield, J. Am. Chem. Soc. 85 (1963), 2149-2154).

Synthetic protein synthesis may be performed using manual techniques or by automation. Automated synthesis may be achieved, for example, using the Applied Biosystems 431A Peptide Synthesizer (Perkin Elmer, Foster City CA) in accordance with the instructions provided by the manufacturer. Various fragments may be chemically synthesized separately and combined using chemical methods to produce the full length molecule. As indicated above, chemical synthesis, such as the solid phase procedure described by Houghton (Proc. Natl. Acad. Sci., 1985, 82: 5131) can be used.
Protein isolation and purification can be achieved by any one of several known techniques; for example and without limitation, ion exchange chromatography, gel filtration chromatography, affinity chromatography, high pressure liquid chromatography (HPLC), reversed phase HPLC, hydrophobic interaction chromatography and preparative disc gel electrophoresis. Protein isolation/purification techniques may require modification of the polypeptides of the present invention using conventional methods. For example, a histidine tag can be further added to the protein to allow purification on a nickel column. Other modifications may cause higher or lower activity, permit higher levels of protein production, or simplify purification of the protein.

In an alternative embodiment the invention provides a polypeptide encoded by the nucleic acid molecule of the invention or produced by the method of the invention. The polypeptide encoded by the mutant nucleic acid molecule of the invention is also referred to herein as the "mutant polypeptide".

In a further embodiment the invention provides an antibody, aptamer or phage that specifically binds to the nucleic acid molecule or the polypeptide of the invention.

Said antibody may be, inter alia, a monoclonal or a polyclonal antibody.
The term "antibody" includes monoclonal antibodies, polyclonal antibodies, single chain antibodies, or fragments thereof that specifically bind said peptide or polypeptide, also including bispecific antibodies, synthetic antibodies, antibody fragments, such as Fab, a F(ab₂)', Fv or scFv fragments etc., or a chemically modified derivative of any of these. Monoclonal antibodies can be prepared, for example, by the techniques as originally described in Köhler and Milstein, Nature 256 (1975), 495, and Galfré, Meth. Enzymol 73 (1981), 3, which comprise the fusion of mouse myeloma cells to spleen cells derived from immunized mammals with modifications developed by the art. Furthermore, antibodies or fragments thereof to the aforementioned peptides can be obtained by using methods which are described, e.g., in Harlow and Lane "Antibodies, A Laboratory Manual", CSH Press, Cold Spring Harbor, 1988. When derivatives of said antibodies are obtained by the phage display technique, surface plasmon resonance as employed in the BIAcore system can be used to increase the efficiency of phage antibodies which bind to an epitope of the peptide or polypeptide of the invention (Schier, Human Antibodies Hybridomas 7 (1996), 97-105; Malmborg, J. Immunol. Methods 183 (1995), 7-13). The production of chimeric antibodies is described, for example, in WO89/09622 A further source of antibodies to be utilized in accordance with the present invention are so-called xenogenic antibodies. The general principle for the production of xenogenic antibodies such as human antibodies in mice is described in; e.g., WO 91/10741, WO 94/02602, WO 96/34096 and WO 96/33735. Antibodies to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning: A Laboratory Manual, Cold Spring Harbor Laboratory, Cold Spring Harbor, NY, 1989. The term "monoclonal" or "polyclonal antibody" (see Harlow and Lane, (1988), loc. cit.) also relates to derivatives of said antibodies which retain or essentially retain their binding specificity. Preferred derivatives of such antibodies are chimeric antibodies comprising, for example, a mouse or rat variable region and a human constant region.
The term "scFv fragment" (single-chain Fv fragment) is well understood in the art and preferred due to its small size and the possibility to recombinantly produce such fragments.
Preferably, the antibody, aptamer, fragment or derivative thereof according to the invention specifically binds the target protein, polypeptide or fragment or epitope thereof whose presence or absence is to be monitored.

The term "specifically binds" used in accordance with the present invention means that the antibody etc. does not or essentially does not cross-react with polypeptides of similar structures, such as wild type P2Y5, the sequence of which is shown in SEQ ID NO:2. Cross-reactivity of a panel of antibodies etc. under investigation may be tested, for example, by assessing binding of said panel of antibodies etc. under conventional conditions (see, e.g., Harlow and Lane, (1988), loc. cit.) to the polypeptide of interest as well as to a number of more or less (structurally and/or functionally) closely related polypeptides. Only those antibodies that bind to the polypeptide of interest but do not or do not essentially bind to any of the other polypeptides which are preferably expressed by the same tissue as the polypeptide of interest, are considered specific for the polypeptide of interest and selected for further studies in accordance with the method of the invention.

Aptamers are DNA or RNA molecules that bind other molecules, such as nucleic acids, proteins, small organic compounds, and even entire organisms. A database of aptamers is maintained at http://aptamer.icmb.utexas.edu/.
More specifically, aptamers can be classified as DNA or RNA aptamers or peptide aptamers. Whereas the former consist of (usually short) strands of oligonucleotides the latter consist of a short variable peptide domain, attached at both ends to a protein scaffold.
Nucleic acid aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms.
Peptide aptamers are proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which have good solubility properties.

Currently, the bacterial protein Thioredoxin-A is the most used scaffold protein, the variable loop being inserted within the reducing active site, which is a -Cys-Gly-Pro-Cys- loop in the wild protein, the two cysteins lateral chains being able to form a disulfide bridge. Peptide aptamer selection can be made using different systems, but the most used is currently the yeast two-hybrid system.
Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, etc. are available to scientists with which the half-life of aptamers easily can be increased to the day or even week time scale.

Phages in accordance with the present invention refer to recombinant phages and are well known in the art and are described, for example, in Griffiths, A.D. et al.: EMBO J. 1994, 13:3245. The phage may carry immunoglobulin fragments or derivatives with a desired binding specificity for the polypeptide of the invention as a fusion protein on their surface, wherein the fusion partner is a surface molecule of the phage.

In a preferred embodiment of the method of the invention said antibody or aptamer or phage is detectably labeled. Whereas the aptamers are preferably radioactively labeled with ³H or ³²P or with a fluorescent marker such as described above, the phage or antibody may e.g. be labeled in a corresponding manner (with ¹³¹I as the preferred radioactive label) or be labeled with a tag such as His-tag, FLAG-tag or myc-tag.

In another aspect, the present invention provides an oligo- or polynucleotide comprising or consisting of an oligo- or polynucleotide selected from the group consisting of: (a) an oligo- or polynucleotide consisting of at least 10 consecutive nucleotides of SEQ ID NO:5 or 7; wherein the at least 10 consecutive nucleotides contain a T at position 463 of SEQ ID NO:5, or a G at position 373 of SEQ ID NO:7; (b) an oligo- or polynucleotide hybridizing under stringent conditions to at least a portion of the oligo- or polynucleotide of (a), wherein said portion comprises the nucleotide in position 463 of SEQ ID NOs:5 or the nucleotide in position 373 of SEQ ID NO:7, wherein said oligo- or polynucleotide contains a T at position 463 of SEQ ID NO:5 or a G at position 373 of SEQ ID NO:7; and (c) an oligo- or polynucleotide identical to the oligo- or polynucleotide of (a) or (b) with the exception that T is replaced by U.

In the context of the present invention the term "oligo- or polynucleotide" refers to nucleic acid molecules of different length: an oligonucleotide is a nucleic acid molecule consisting of up to 30 bp, a polynucleotide is a nucleic acid molecule consisting of more than 30 bp. The oligo- or polynucleotides comprise most preferably at least 17 nucleotides, but may also comprise at least 19, 25, 50, 100, 150, 200 or more nucleotides, whereas oligonucleotides of 10, 11, 12, 13, 14, 15 or 16 nucleotides are also envisaged. The oligo- or polynucleotides of the invention contain either a T at the nucleotide position corresponding to position 463 of SEQ ID NO:5 or a G at the nucleotide position corresponding to position 373 of SEQ ID NO:7, wherein the said nucleotides may be located at any position within the oligo-or polynucleotides. The oligo- or polynucleotides of the present invention are characterized as being associated with P2Y5-mediated diseases, in particular the diseases recited herein above.

Said complementary oligo- or polynucleotides of item (b) refer to oligo- or polynucleotides the sequence of which is uniquely fitting to (hybridizing to/complementary to preferably 100%) the sequences of the oligo- or polynucleotides described in (a), but not to wild type sequences, which contain a C at the position corresponding to position 463 of SEX ID NO:5 and an A at the position corresponding to position 373 of SEQ ID NO:5 (as shown in SEQ ID NO:1). Upon hybridization of the oligo- or polynucleotides under stringent conditions to at least a portion of the oligo- or polynucleotide of (a), the nucleotides reflecting the mutations in the *P2RY5* gene, i.e. the T at position 463 of SEQ ID NO:5 and/or the G at position 373 of SEQ ID NO:7, have to be contained in the resulting double-strand nucleic acid strand.
These oligo- or polynucleotides of the invention may, for example, be useful as probes in Northern or Southern Blot analysis of RNA or DNA preparations, respectively, or can be used as oligonucleotide primers in PCR analysis dependent on their respective size. Also comprised by the invention are hybridizing oligo- or polynucleotides which are useful for analysing DNA-Protein interactions via, e.g., electrophoretic mobility shift analysis (EMSA). Preferably, said hybridizing oligo- or polynucleotides comprise at least 10, more preferably at least 15 nucleotides in length while a hybridizing oligo- or polynucleotide of the present invention to be used as a probe preferably comprises at least 100, more preferably at least 200, or most preferably at least 500 nucleotides in length, wherein the mutations of the present invention preferably have a central location. "Central" meaning most preferably that an equal number of nucleotides is located in the 5'- and 3' -direction adjacent to the position of the mutation. In a different preferred embodiment 60, 70, 80 or 90 percent of nucleotides are located in the 5'- or 3'-direction adjacent to the position of the mutation and the remaining nucleotides are located in the opposite direction.
The person skilled in the art is familiar with the preparation and the use of said probes (see, e.g., Sambrook and Russel "Molecular Cloning, A Laboratory Manual", Cold Spring Harbor Laboratory, N.Y. (2001)). Said nucleic acid molecules may be chemically synthesized or transcribed by an appropriate vector containing a chimeric gene which allows for the transcription of said nucleic acid molecule in the cell.

Such oligo-or polynucleotides may be used in any of the above described methods. In particular, they may be used in distinguishing nudeic acid molecules carrying the mutations resulting in P2Y5 receptor truncation from nucleic acid molecule encoding the wild-type P2Y5 receptor. Therefore, these oligo-or polynucleotides are of relevance in the diagnosis of diseases associated with P2Y5 receptor mutation, such as hair-loss.

The invention further relates to a diagnostic composition comprising the nucleic acid molecule or the polypeptide according to the invention or the antibody, aptamer or phage of the invention.

The term "diagnostic composition" relates to compositions for diagnosing individual patients for their potential response to or curability by the pharmaceutical compositions of the invention and for diagnosing a predisposition of individual patients for a disease involving P2Y5 receptor dysfunction, such as hair-loss. The diagnostic composition of the invention comprises at least one of the compounds recited above. Said composition may further comprise appropriate buffer(s), and enzymes such as reverse transcriptase, thermostable polymerases etc. The diagnostic composition of the invention may be used to test for the presence of the nucleic acid molecule of the invention using methods well known in the art. Such methods are described, e. g. in Sambrook and Russel "Molecular Cloning, A Laboratory Manual", Cold Spring Harbor Laboratory, N.Y. (2001).

Methods for testing a sample for the presence of the nucleic acid molecule of the invention include, but are not limited to, nucleic acid amplification, sequencing or hybridization assays.

Examples for nucleic acid amplification assays and means to perform such include without limitation PCR, (including nested PCR, RT-PCR, PCR extension assays, Nucleic Acid Sequence Base Amplification (NASBA), single-strand confirmation polymorphism (SSCP) PCR), amplification refractory mutation systems (ARMSTM) and amplification refractory mutation system linear extension (ALEXTM) assays. Details of such methods can be found in art, see, for example, Newton et al., Nucleic Acids Res. 17 (1989) 2503-2516; Agrawal (Ed.), "Protocols for Oligonucleotides and Analogs: Synthesis and Properties (Methods in Molecular Biology, 20)", Humana Press, 1993; Haque et al., Diagn. Maul. Pathol. 7 (1998) 248-252; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002; Pissardet al., Clin. Chem. 48 (2002) 769-772; Steemers et al., Nature Meth. 3 (2006) 31-33; Kakavas et al., J. Clin. Lab. Anal. 20 (2006) 1-7.

Examples for sequencing assays comprise without limitation approaches of sequence analysis by direct sequencing, fluorescent SSCP in an automated DNA sequencer and Pyrosequencing. These procedures are common in the art, see e.g. Adams et al. (Ed.), "Automated DNA Sequencing and Analysis", Academic Press, 1994; Alphey, "DNA Sequencing: From Experimental Methods to Bioinformatics", Springer Verlag Publishing, 1997; Ramon et al., J. Transl. Med. 1 (2003) 9; Meng et al., J. Clin. Endocrinol. Metab. 90 (2005) 3419-3422.

Examples for hybridization assays comprise without limitation Northern and Southern blot assays, heteroduplex analysis, detection of mutations by sequence specific oligonucleotide hybridization, allele-specific oligonucleotide hybridization on DNA chips, assays based on Illumina's^{®} technology, assays based on the BeadArray^{®} technology, see, for example, Barnes et al., Nucleic Acids Res. 33 (2005) 5914-5923; Fan et al., Biotechniques 39 (2005) 583-588; Shen et al., Mutat. Res.-Fund. Mol. M. 573 (2005) 70-82; Steemers and Gunderson, Pharmacogenomics, 6 (2005) 777-782.

Examples for assays based on protein detection include without limitation method steps such as ion exchange chromatography, gel filtration chromatography, affinity chromatography, hydrophobic interaction chromatography, reversed phase HPLC, disc gel electrophoresis, capillary electrophoresis, Western blot analysis, immunoprecipitation, immuno-blotting, ELISA, RIA, indirect immunofluorescence experiments, amino acid sequencing, spectroscopic methods (UV, CD; IR, Fluoreszenz) and mass spectrometry (e.g. MS-QTOF), see, for example, Soejima and Koda, Transfusion 45 (2005) 1934-1939; Yeh et al., Anesth. Analg. 101 (2005) 1401-1406; Chou et ai., Am. J. Clin. Pathol.. 124 (2005) 330-338.

The above described assays are known in the art, e.g. from standard text books such as Lottspeich, Engel "Bioanalytik" Spektrum Akademischer Verlag (2006); Sambrook" Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989); Higgins and Hames (Eds.) "Nucleic acid hybridization, a practical approach" IRL Press Oxford, Washington DC, (1985); Nollau et al, Clin. Chem. 43 (1997), 1114-1128; The use of some of the recited assays is described in the appended examples.

The diagnostic composition may be particularly useful for determining expression of P2Y5 receptor and *P2RY5* gene product in a biological sample. As the above described mutations within the *P2RY5* gene result in the expression of truncated, non-functional P2Y5 receptor protein, decreased expression levels of full length protein as well as detection of mutations may be used to ascertain that an individual does not have a disease or disorder, or is at risk of developing diseases or disorders associated with aberrant P2Y5 receptor or *P2RY5* expression, preferably diseases selected from those described herein above.

In an alternative embodiment the invention provides a method for testing for the presence of the mutant nucleic acid molecule or the mutant polypeptide of the invention comprising assaying a sample obtained from a subject for the presence of said mutant nucleic acid molecule or mutant polypeptide, wherein the presence of the mutant nucleic acid molecule or the mutant polypeptide of the invention is indicative for hair-loss.

Methods for testing a sample for the presence of the mutant nucleic acid molecule of the invention include, but are not limited to, nucleic acid amplification, sequencing or hybridization assays, as described herein above in connection with diagnostic methods

In another preferred embodiment of the method of the invention said sample is blood, serum, plasma, fetal tissue, saiiva, urine, mucosal tissue, mucus, vaginal tissue, fetal tissue obtained from the vagina, skin, hair, hair follicle or another human tissue.
Preferably, the sample is blood, serum, plasma, saliva, urine, mucosal tissue or mucus.

The invention also relates to a Kit comprising at least one of the mutant nucleic acid molecule, the vector containing the mutant nucleic acid molecule, the host, the mutant polypeptide, the antibody, aptamer and/or phage and/or the anti-sense oligo-or polynucleotide, the fragment of the nucleic acid molecule or the primer pair of the invention.
The various components of the kit may be packaged in one or more containers such as one or more vials. The vials may, in addition to the components, comprise preservatives or buffers for storage.

### The Figures show:

### Figure 1.

Multi-point LOD score for the linkage of Hypotrichosis to STR (short tandem repeat) markers covering chromosomes 1 to 22.

### Figure 2.

Pedigree of family 1, homozygosity mapping. Marker haplotypes on chromosome 13q that are linked to HS are indicated by black bars. Affected family members are shown in black, circles and squares denote females and males respectively. Microsatellite markers are given on the left, and the location of *P2RY5* is indicated by an arrow. The homozygous region of the affected individuals is boxed, confined by a recombination in II:4 at the centromeric site and the observation of heterozygosity at the telomeric site.

### Figure 3.

Pedigrees of HS-families 2 **(A)** and 3 **(B)** showing high resolution microsatellite genotyping in the region surrounding the *P2RY5* gene. The chromosomal region carrying the disease mutation shared between the two families is boxed. This indicates a founder effect for the c.373_374delAA mutation observed in families 2 and 3. SB, still birth.

### Figure 4.

Aligned sequences of mutation c.463C>T and a control **(A)** and of mutation c.373_374delAA and a control **(B).**

### Figure 5.

Structure and biochemical analysis of P2Y5 wild-type and mutant proteins.
**(A)** Domain structure of wild-type and mutant proteins of P2Y5. The positions of the mutations c.463C>T and c.373_374delAA are indicated by arrows.
**(B)** Western Blot analysis of COS7 cells transiently expressing wild-type and mutant P2Y5. The wild-type P2Y5 protein has a size of around 30 kDa, whereas the two truncated proteins have the anticipated sizes of 15 kDa and 17 kDa respectively. The proteins display additional bands, probably resulting from oligomerisation.
(**C** to **E**) Immunofluorescence analysis of COS7 cells transiently expressing wild-type and mutant P2Y5. Cells were stained with 4,6-diamidino-2-phenylindole-dihydrochloride (DAPI) and antibodies against V5 (P2Y5), and protein disulfide isomerase (PDI, mutant) or pan-cadherin (CAD, wild-type) respectively. P2Y5 wild-type is located in the cell membrane **(C)**, the mutant P2Y5 proteins are located predominantly in the endoplasmic reticulum (**D** and **E**). Scale bars 10 µm.

### Figure 6.

**(A)** Expression of *P2RY5* and *GAPDH* in various normal human tissues and cells. RT-PCR analysis was performed using the Human MTC Panel I and II (BD Biosciences, Franklin Lakes, NJ) and a human keratinocyte cDNA library (Clontech, Mountain View, CA). The last lane is a negative (no template) control. *P2RY5* is expressed in all investigated tissues.
**(B)** DNA contamination of RNA-samples was prevented by *DIVase* I digestion after isolation of RNA. Shown are PCR-products of *P2RY5* obtained with primers P2RY5 1.1F and P2RY5 1R.

### Figure 7.

BD MTE Multiple Tissue Expression Human Array 3 probed with a *P2Y5*-probe.

### Figure 8.

Northern blot analysis of *P2RY5* expression in mouse tissues. A DIG labelled cRNA probe corresponding to nucleotides 82 to 812 of mouse *P2RY5* cDNA sequence was used for hybridisation. A single transcript of 2.4 kb is detected in all tissues. Reference markers of 2.6 kb and 1.8 kb are indicated. Below is shown an ethidium bromide stain of ribosomal RNA as a loading control.

### Figure 9.

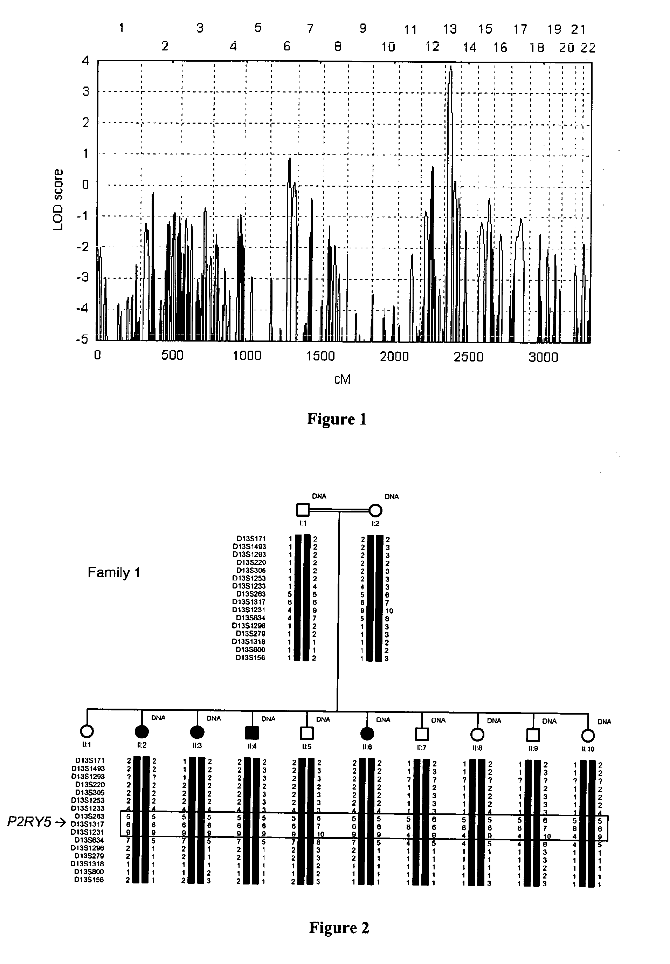

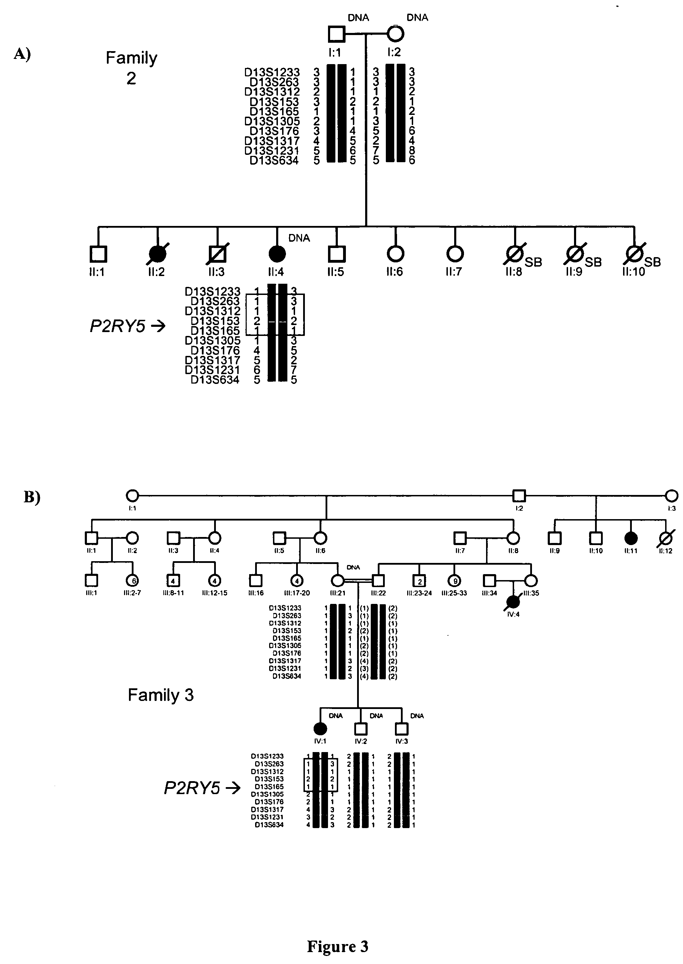

Protein truncation test (PTT) performed with the TNT^{®} T7 Quick Coupled Transcription/Translation System (Promega, San Luis Obispo, CA) according to manufacturer's instructions. The PTT was performed with RNA from immortalized lymphocytes of a homozygous and a heterozygous member of family 3 carrying the c.373_374AA deletion (fig. 3B, III:21 and IV:1) and genomic DNA of a homozygous and a heterozygous member of family 1 carrying the c.463C>T transition (Fig. 2, II:2 and II:7) and two control individuals (wild-type).
The Examples illustrate the invention:

### Example 1: Materials and Methods

### i) Linkage Analysis

Blood samples from eleven members of a consanguineous Saudi-Arabian family (K. Al Aboud, K. Al Hawsawi, D. Al Aboud, A. Al Githami, Clin Exp Dermatol 27, 654 (2002)) (the parents are third cousins, family 1, Fig. 2) were collected after obtaining informed consent. Samples from the two smaller families (D. Al Aboud, K. Al Aboud, K. Al Hawsawi, A. Al Aboud, Sudan J Dermatol 3, 128 (2005)) (families 2 and 3, Fig. 3)) were collected independently at a later date. Genomic DNA was extracted from families 1-3 according to standard methods. In addition, EBV- (Ebstein Barr Virus) transformed lymphoblastoid cell lines were created from samples of family 3 according to standard protocols (Current Protocols in Molecular Biology", Volume 5, John Whey and Sons, 2005).

A total genome scan was performed using 320 highly polymorphic microsatellite markers (Généthon) at an average density of 10-cM on an ABI 3100 Sequencer (Applied Biosystems, Foster City, CA). Mendelian incompatibilities were detected with the program PedCheck and erroneous genotypes deleted in the whole family (J. R. O'Connell, D. E. Weeks, Am J Hum Genet 63, 259 (1998)). An autosomal-recessive model with full penetrance was assumed and a trait locus mutant allele frequency of 0.0001. The genetic marker map from Généthon with equal male and female recombination rates was used. Two-point LOD scores were calculated between each marker locus and HSS, with the LINKAGE version 5.21 software (G. M. Lathrop, J. M. Lalouel, C. Julier, J. Ott, Proc Natl Acad Sci U S A 81, 3443 (1984)). For multi-point LOD score calculation and haplotyping Merlin and Simwalk2. were used (G. R. Abecasis, S. S. Cherny, W. O. Cookson, L. R. Cardon, Nat Genet 30, 97 (2002); E. Sobel, K. Lange, Am J Hum Genet 58, 1323 (1996)). The results of the multi-point LOD score calculation are shown in Figure 1.

### ii) Mutation Screening

39 genes (including *P2RY5,* table 1) were screened by amplifying the coding regions and splice sites by PCR and direct sequencing of patient 11:2 (family 1) and her parents.
PCR was performed in a total volume of 25 µl with 40 ng of genomic DNA using the REDTaq^{™} ReadyMix^{™} PCR Reaction Mix with MgCl₂ (Sigma-Aldrich, St. Louis, MO), under the following conditions: initial denaturation 95°C 5 min, 1 st cycle: 95°C 30 s, 63°C 30 s, 72°C 30-80 s, 2nd cycle 95°C 30 s, 60°C 30 s, 72°C 30-80 s, 3rd - 34th cycle: 95°C 30 s, 57°C 30 s 72°C 30-80 s, final extension 72°C 7 min.
The PCR products were purified with the GFX PCR DNA Purification Kit (Amersham Biosciences, Buckinghamshire, UK) and were directly sequenced on an ABI 3100 genetic analyzer (Applied Biosystems, Foster City, CA) using the BigDye Terminator v1.1 Cycle Sequencing Kit (Applied Biosystems, Foster City, CA) and the DyeEx 2.0 Spin Kit (Qiagen, Hilden, Germany).
Primers for amplification of PCR-products for sequencing of *P2RY5* and genotyping of microsatellites shown in Fig. 2 and Fig. 3 are given in tables 2, 3 and 4.

**Table 1: Exclusion of positional candidate genes in chromosomal region 13q14.11-13q21.33**

| Gene abbreviation | Positional candidate gene |
|---|---|
| FOXO1A | Forkhead box protein O1A |
| ELF1 | E74-like factor 1 (Ets domain transcription factor) |
| KBTBD6 | Kelch repeat and BTB (POZ) domain-containing 6 |
| KBTBD7 | Kelch repeat and BTB (POZ) domain containing 7 |
| RGC32 | Response gene to complement 32 |
| DGKH | Homo sapiens DGKH-1 mRNA for diacylglycerol kinase eta1 A-kinase anchor protein 11 |
| AKAP 11 | |
| TNFSF11 | Tumor necrosis factor ligand superfamily member 11 |
| EPSTI1 | Epithelial stromal interaction 1 |
| DNAJC15 | DnaJ homolog subfamily C member 15 |
| ENOX1 | Ecto-NOX disulfide-thiol exchanger 1 |
| TSC22D1 | TSC22 domain family protein 1 |
| GTF2F2 | General transcription factor IIF, polypeptide 2 |
| KCTD4 | Potassium channel tetramerisation domain |
| TPT1 | Tumor protein, translationally-controlled 1 |
| weakly similar to TRICHOHYALIN | AK057244 |
| LCP1 | L-plastin |
| HTR2A | 5-hydroxytryptamine (serotonin) receptor 2A |
| MED4,DRIP36 | Mediator of RNA polymerase II transcription subunit 4 (Vitamin D3 receptor-interacting protein complex 36 kDa component) |
| RCBTB2 | Regulator of chromosome condensation and BTB |
| FNDC3A | Fibronectin type-III domain-containing protein 3a |
| MLNR | Motilin receptor (G protein-coupled receptor 38) |
| PHF11 | PHD finger protein 11 |
| ARL11 | ADP-ribosylation factor-like protein 11 |
| EBPL | Emopamil binding related protein, delta8-delta7 |
| TRIM13 | Tripartite motif-containing protein 13 (Ret finger protein 2) |
| KCNRG | Putative potassium channel regulatory protein |
| INTS6 | Integrator complex subunit 6 isoform b |
| NEK3 | NIMA-related kinase 3 |
| THSD1 | Thrombospondin type I domain-containing 1 |
| CKAP2 | Cytoskeleton-associated protein 2 |
| SUGT1 | Suppressor of G2 allele of SKP1 homolog |
| PCDH8 | Protocadherin-8 isoform 1 precursor |
| PCDH17 | Protocadherin-17 precursor |
| DIAPH3 | Diaphanous homolog 3 isoform a |
| PCDH20 | Protocadherin-20 precursor |
| PCDH9 | Protocadherin-9 precursor |

**Table 2: Primers used for linkage analysis on chromosome 13. Fam 1 = Family 1; Fam 2+3 = Family 2 and 3**

| Primer denomination | sequence | Fam 1 | Fam 2+3 | SEQ ID NO: |
|---|---|---|---|---|
| D13S171F | 5'-CCTACCATTGACACTCTCAG-3' | x | | 26 |
| D13S171R | 5'-TAGGGCCATCCATTCT-3' | x | | 27 |
| D13S1493F | 5'-ACCTGTTGTATGGCAGCAGT-3' | x | | 28 |
| D13S1493R | 5'-GGTTGACTCTTTCCCCAACT-3' | x | | 29 |
| D13S1293F | 5'-TGCAGGTGGGAGTCAA-3' | x | | 30 |
| D13S1293R | 5'-AAATAACAAGAAGTGACCTTCCTA-3' | x | | 31 |
| D13S220F | 5'-CCAACATCGGGAACTG-3' | x | | 32 |
| D13S220R | 5'-TGCATTCTTTAAGTCCATGTC-3' | x | | 33 |
| D13S305F | 5'-TTGAGGACCTGTCGTTACG-3' | x | | 34 |
| D13S305R | 5'-TTATAGAGCAGTTAAGGCACA-3' | x | | 35 |
| D13S1253F | 5'-CCTGCATTTGTGTACGTGT-3' | x | | 36 |
| D13S1253R | 5'-CAGAGCCGTGGTAGTATATTTTT-3' | x | | 37 |
| D13S1233F | 5'-AGGACTANAGATGAATGCTC-3' | x | x | 38 |
| D13S1233R | 5'-GACATGACTCCATGTTTGGT-3' | x | x | 39 |
| D13S263F | 5'-CCTGGCCTGTTAGTTTTTATTGTTA-3' | x | x | 40 |
| D13S263R | 5'-CCCAGTCTTGGGTATGTTTTTA-3' | x | x | 41 |
| D13S1312F | 5'-TCTTCCCAGAATATATGGGA-3' | | x | 64 |
| D13S1312R | 5'-AGCTGTAAAAGTGTTTGTTTGATGT-3' | | x | 65 |
| D13S153F | 5'-AGCATTGTTTCATGTTGGTG-3' | | x | 66 |
| D13S153R | 5'-CAGCAGTGAAGGTCTAAGCC-3' | | x | 67 |
| D13S165F | 5'-GTTTCGCCAAGCCTGTT-3' | | x | 68 |
| D13S165R | 5'-GTTGACAATAAAATACGCCACA-3' | | x | 69 |
| D13S1305F | 5'-GATGGCACCATTGCAC-3' | | x | 70 |
| D13S1305R | 5'-CAGCACATCCAAACAAGG-3' | | x | 71 |
| D13S176F | 5'-CTGTGGGATTCCTTAGTGATAC-3' | | x | 72 |
| D13S176R | 5'-ATATTCAGACAAAAGCCAAGTTA-3' | | x | 73 |
| D13S1317F | 5'-CTTGGAAACCAACAAGTCAC-3' | x | x | 42 |
| D13S1317R | 5'-ATTTTGCCACCTAGAACGG-3' | x | x | 43 |
| D13S1231F | 5'-ACAGTTTTTCGAGGCCATATC-3' | x | x | 44 |
| D13S1231R | 5'-GGTTAAATAAATCTCCATCCAGAAG-3' | x | x | 45 |
| D13S634F | 5'-TCCAGATAGGCAGATTCAAT-3' | x | x | 46 |
| D13S634R | 5'-CCTTCTTCTTCCCATTGATA-3' | x | x | 47 |
| D13S1296F | 5'-TGCAGAAATGTGAGCC-3' | x | | 48 |
| D13S1296R | 5'-TCCACCTAGAGCAACTACC-3' | x | | 49 |
| D13S279F | 5'-TGGTTTGTTGCAGAAAGCACAC-3' | x | | 50 |
| D13S279R | 5'-TTGGGCCTTGTCAACCTTCATA-3' | x | | 51 |
| D13S1318F | 5'-GGCAAAGCCTTGCTCTTAAT-3' | x | | 52 |
| D13S1318R | 5'-GGCTGTGCTCTTCCAAAATA-3' | x | | 53 |
| D13S800F | 5'-AGGGATCTTCAGAGAAACAGG-3' | x | | 54 |
| D13S800R | 5'-TGACACTATCAGCTCTCTGGC-3' | x | | 55 |
| D13S156F | 5'-ATTAGCCCAGGTATGGTGAC-3' | x | | 56 |
| D13S156R | 5'-GCTGTGGTATGAGTTACTTAAACAC-3' | x | | 57 |

**Table 3: Primers for amplification and sequencing of the coding region of P2RY5**

| Primer | sequence | SEQ ID NO: |
|---|---|---|
| denomination | | |
| P2RY5 Ex1.1F | 5'-TGGAGGTTATAGAGGTTATAATC-3' | 11 |
| P2RY5 Ex1.2F | 5'-TTATACCAACATGTACGGAAGC-3' | 12 |
| P2RY5 Ex1R | 5'-TGTTAATTTCTTTTGGAGGTGG-3' | 13 |

**Table 4: Primers for amplification and sequencing of P2RY5 UTR**

| Primer | sequence | SEQ ID NO: |
|---|---|---|
| denomination | | |
| P2RY5 5'UTR 7F | 5'-TTGTGTGTCTTTAAGACCCTCC-3' | 14 |
| P2RY5 5'UTR 7R | 5'-TCCTGCCTGGGCCGCAGAGC-3' | 15 |
| P2RY5 5'UTR 6F | 5'-AAATGATACCGATAGACTTATGG-3' | 16 |
| P2RY5 5'UTR 6R | 5'-TCTTTGATGACACCTATGAATAGC-3' | 17 |
| P2RY5 5'UTR 5F | 5'-TTATAGGTGTGAACCACAGATCC-3' | 18 |
| P2RY5 5'UTR 5R | 5'-TGTTGCCATAATGACCTTGTAG-3' | 19 |
| P2RY5 5'UTR 4F | 5'-TGGGGTTTCTGTGTGGAAGTCC-3' | 20 |
| P2RY5 5'UTR 3R | 5'-TCTCTATTTCCAACTGAGGTACCC-3' | 21 |
| P2RY5 5'UTR 2R | 5'-AATGGATAGATCACTATTTGTTG-3' | 22 |
| P2RY5 5'UTR 1 F | 5'-AGGAAGTGCAAACAAACTGGG-3' | 23 |
| P2RY5 5'UTR 1 R | 5'-AGCAGTGGGAGCTGTTAACG-3' | 24 |
| P2RY5 3'UTR R | 5'-AAAGGAATTCAAAGACATTACAG-3' | 25 |

### iii) Structure and Expression of Human P2RY5

Human occipital hair follicles from various individuals were obtained by plucking and were pooled and immediately stored in RNA*later*^{™} Stabilization Solution (Ambion, Austin, TX). We isolated the RNA using the RNeasy Micro Kit (Qiagen, Hilden, Germany), followed by a RT-PCR using the SuperScript^{™} II RNase H⁻ Reverse Transcriptase (Invitrogen, Carlsbad, CA) and random hexamers under standard conditions (25°C 10 min, 42°C 50 min, 70°C 15 min).
Since alternative transcripts of *P2RY5* differing in the length of the 5'UTR are given in the database (UCSC, NCBI Build 36.1), we analysed the structure of *P2RY5* expressed in hair follicle cells. Primers were created for different parts of the UTR (Table 5) and hair follicle cDNA was amplified by PCR using the REDTaq^{™} ReadyMix^{™} PCR Reaction Mix with MgCl₂ (Sigma-Aldrich, St. Louis, MO) under the following conditions: initial denaturation 95°C 5 min, 1 st cycle: 95°C 30 s, 63°C 30 s, 72°C 80 s, 2nd cycle 95°C 30 s, 60°C 30 s, 72°C 80 s, 3rd - 37th cycle: 95°C 30 s, 57°C 30 s, 72°C 80 s, final extension 72°C 7 min. Samples were subjected to gel electrophoresis on 1.5% agarose gels. Only one transcript was found to be expressed in hair follicle cells consisting of the one coding exon, and resulting in a short 5'UTR. To determine the exact sequence of the 5'UTR, a RACE analysis with the 5'/3' RACE Kit, 2^{nd} Generation (Roche Applied Science, Indianapolis, IN) was performed according to the manufacturer's instructions using the gene-specific primer 5'-AGCAGTGGGAGCTGTTAACG-3' (SEQ ID NO:9). The sequence thus obtained is: 5'-NNTTTTTTTTTTTTTTTCACAGCAACAAATTTCATGTTTCTTTTGG GTATTTCTGAGAAAAAGGAAATATTTATAAAACCATCCAAAGATCCAGATAATTT GCAAATAAATTGGAGGTTATAGAGGTTATAATCTGAATCCCAAAGGAGACTGCA GCTGATGAAAGTGCTTCCAAACTGAAAATTGGACGTGCCTTTACGATGGTAAG CGTTAACAGCTCCCACTGCT-3' (SEQ ID NO:58).

**Table 5: Primers for expression analysis of P2RY5 UTR in human hair follicle cells (each forward-primer was combined with the P2RY5 5'UTR1 R Expr reverse-primer).**

| Primer denomination | Sequence | SEQ ID NO: |
|---|---|---|
| P2RY5 5'UTR7F Expr | 5'-TGGATTAGGAGAGCTTCTGG-3' | 80 |
| P2RY5 5'UTR6F Expr | 5'-TGGAAGTCACATTTCACCATGGC-3' | 81 |
| P2RY5 5'UTR3F Expr | 5'-TCTAAGGGTTGTGAAGATCACG-3' | 82 |
| P2RY5 5'UTR2F Expr | 5'-TCTTCCTCAGTAGAAACAACTGG-3' | 83 |
| P2RY5 5'UTR1 F Expr | 5'-AAGATACAACTGGCAACTGAGG-3' | 84 |
| P2RY5 5'UTR1R Expr | 5'-AGCAGTGGGAGCTGTTAACG-3' | 85 |

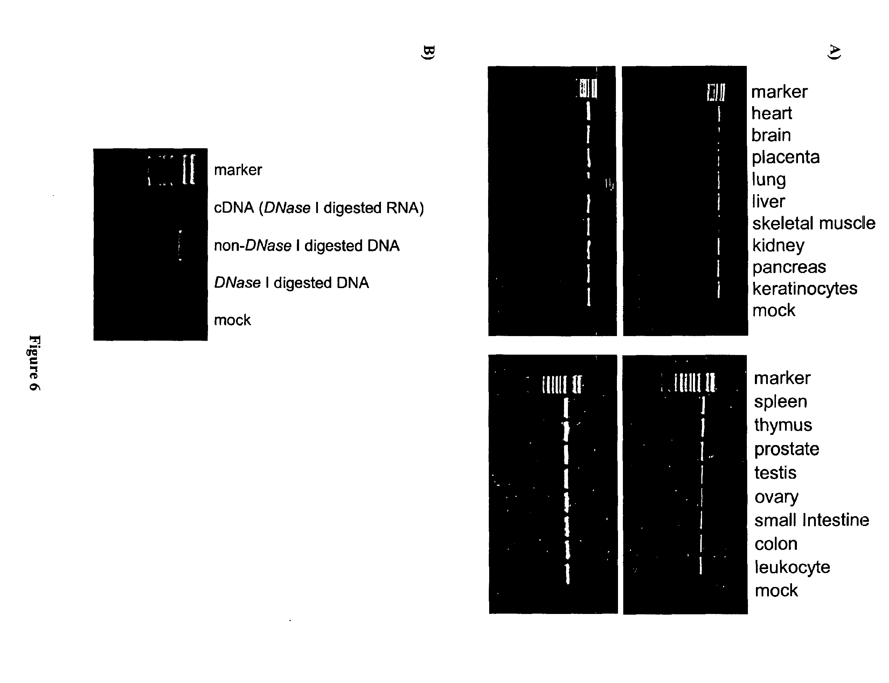

To study the expression of *P2RY5*-mRNA in different tissues, the Human MTC Panel I and II (BD Biosciences, Rockville, MD) were used (Fig. 6A), as well as a human keratinocyte cDNA library (Clontech, Mountain View, CA). The cDNA was amplified by PCR using the P2RY5 Ex1.1F and the P2RY5 Ex1 R primers (Table 3).. As an internal standard the glyceraldehyde-3-phosphate dehydrogenase (*GAPDH*) was amplified using the primers 5'-AGCCACATCGCTCAGACACC-3' (SEQ ID NO:62) and 5'-TCCTCTTGTGCTCTTGCTGGG-3' (SEQ ID NO:63).
To prevent genomic DNA contamination, total RNA was treated with *DNase* I after isolation (Fig. 6B).

### iv) Northern Blot Analysis of Mouse P2RY5 in Various Tissues

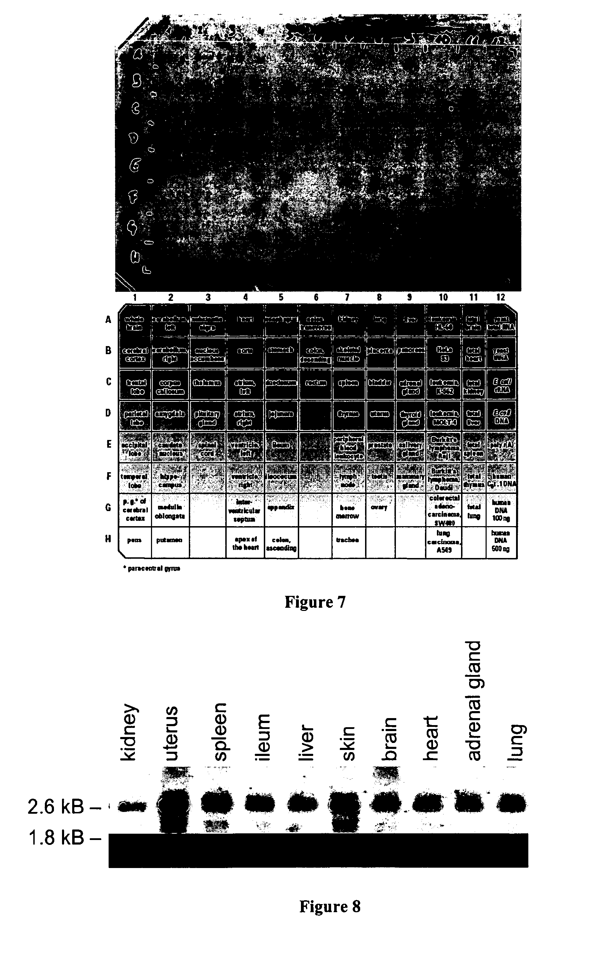

Northern Blot analysis (Fig. 8) was performed using 14 µg of mouse total RNA prepared from tissues using RNeasy kits (Qiagen, Hilden, Germany). Samples were subjected to gel electrophoresis on 1.2% agarose gels in the presence of 0.7% formaldehyde. The RNA was transferred to a positively charged Nylon-membrane (Roche Applied Science, Indianapolis, IN) by vacuum blotting using a Model 785 Vacuum Blotter (BIORAD, Hercules, CA). Hybridisation was carried out at 68°C in Dig Easy Hyb (Roche Applied Science, Indianapolis, IN). Stringency washes were performed at RT in 2xSSC/1% SDS and at 68°C in 0.2xSSC/0.1% SDS. Bound digoxygenin (DIG) was visualised using alkaline phosphatase-conjugated anti-DIG antibody and CPD Star according to the manufacturer's recommendations (Roche Applied Science, Indianapolis, IN).
A 730 bp cDNA fragment corresponding to nucleotides 82 to 812 of mouse *P2RY5* cDNA sequence (mRNA, gi|31341175) was amplified by PCR using primers 5'-CTGGGAAGGCTTTCTTGCTCACTTCAG-3' (SEQ ID NO:74) and 5'-GTTGGATATCAGCCCAAGCACGAAGAC-3' (SEQ ID NO:75) from a mouse skin cDNA preparation. The PCR product was re-amplified using the forward primer and a modified reverse primer to which the T7 RNA polymerase binding site sequence GTAATACGACTCACTATAGGG had been added to the 5' end. The identity of the PCR product was verified by sequencing. DIG-labelled cRNA probes were synthesized from the PCR products with T7 RNA polymerase in the presence of DIG RNA labeling mix (Roche Applied Science, Indianapolis, IN). Ethidium bromide stained ribosomal RNA was used as loading control.

### v) Cloning, Cell Culture and Protein Analysis

*Taq* polymerase-amplified PCR products (*P2RY5* mutations and wild-type-sequence) were cloned in a eukaryotic expression vector with the pcDNA3.1/V5-His TOPO TA Expression Kit (Invitrogen, Carlsbad, CA) using the forward primer 5'-CGAAGCTTCCGCCGCCATGGGTAAGCCTATCCCTAACCCTCTCCTCGGTCTCG ATTCTACGATGGTAAGCGTTAACAGCTCCC-3' (SEQ ID NO:76) and the reverse primer 5'- GCCTCGAGTCAGGCAGCAGATTCATTGTC-3' (wild-type; SEQ ID NO:77), 5'- GCCTCGAGCTAAACAAAAACGGCGGGTGC-3' (c.463C>T; SEQ ID NO:78) and 5'- GCCTCGAGTTACCCTGAGAGTGGGTAGAC-3' (c.373_374delAA; SEQ ID NO:79), respectively.
After reproduction in host cells (E. coli) and isolation of the vector, COS7 cells were transfected transiently. COS7 cells were cultured in Dulbecco's Modified Eagle's Medium with 4,5g/L glucose and L-glutamine (Cambrex, East Rutherford, NJ) supplemented with 10% foetal calf serum (FCS), Penicillin/Streptomycin and Amphotericin B (PAA, Pasching, Austria) at 37°C in an atmosphere of 5% CO₂/95% air.

For immunofluorescence analysis (Fig. 5C to E), COS7 cells were seeded on glass coverslips in 15-mm Petri dishes and kept in medium without antibiotics 24 h before transfection. Cells were transiently transfected using FuGENE HD transfection reagent (Roche Applied Science, Indianapolis, IN) according to manufacturer's instructions. Cells were fixed two days post transfection with ice-cold methanol:acetone (1:1) for 5 min at room temperature (RT) and permeabilized with 1% Triton X-100 in phosphate buffer saline (PBS) for 10 min at RT. After blacking with 3% BSA in PBS for 30 min at RT, we detected the P2Y5 proteins with a monoclonal antibody to V5 (Invitrogen, Carlsbad, CA) at a dilution of 1:500. We visualized the endoplasmic reticulum and the cell membrane through anti-PDI (Stressgen Biotechnologies, Victoria, BC, Canada) and anti-pan Cadherin (United States Biological Swampscott, MA) polyclonal antibodies respectively at a dilution of 1:250. After being washed in PBS, cells were incubated for 1 h at RT with Cy3-conjugated AffiniPure Goat Anti-Mouse IgG (Jackson Immuno Research, West Grove, PA) and Fluorescein Conjugated Affinity Purified Anti-Rabbit IgG (Rockland, Gilbertsville, PA) secondary antibodies in a dilution of 1:1000 respectively. Coverslips were thoroughly washed in PBS three times for 5 min before mounting in antifade mounting medium containing 4,6-diamidino-2-phenylindole-dihydrochloride (DAPI; Vector Laboratories, Burlingame, CA). Cells were imaged with a Zeiss Axioplan2 microscope with appropriate excitation and emission filter pairs. We processed the images using the CytoVision 2.81 software.

For Western Blot analysis (Fig. 5B), COS7 cells were split in 60-mm Petri dishes 24 h before transfection, and kept in medium without antibiotics. Cells were transiently transfected using Lipofectamine 2000 (Invitrogen, Carlsbad, CA) according to manufacturer's instructions. Cells were harvested two days post transfection and centrifuged at 16.100 g for 10 min at 4°C. We resuspended the pellet in an appropriate amount of loading buffer (2xNuPAGE LDS Sample Buffer; Invitrogen, Carlsbad, CA) and 1xComplete Mini Protease Inhibitor Cocktail (Roche Applied Science, Indianapolis, IN) and lysed the cells using an ultrasonic bath. Equal amounts of proteins, including sample reducing agent (Invitrogen, Carlsbad, CA), were boiled at 95°C for 5 min and subjected to SDS-PAGE. After protein separation on 4-12% Bis-Tris gels at 200 V for 45 min, we blotted the proteins semi-wet onto a Invitrolon PVDF membrane (Invitrogen, Carlsbad, CA) for 2 h at 30 V. Western Blot analysis was carried out using the Western Breeze Kit (Invitrogen, Carlsbad, CA) according to manufacturer's instructions. For detection of the expressed proteins an anti-V5 antibody (Invitrogen, Carlsbad, CA) was used in a dilution of 1:5000. Membranes were then exposed to an X-ray film for 15-30 min.

To verify the results of the Western Blot analysis, a protein truncation test (PTT) was performed with the TNT^{®} T7 Quick Coupled Transcription/Translation System (Promega, San Luis Obispo, CA). RNA isolated from immortalized lymphocytes of a homozygous and a heterozygous person carrying the c.373_374delAA deletion in the *P2RY5* gene (Fig. 3B, III:21 and IV:1) and a control probe were used. In addition, a PTT using genomic DNA of homozygous and heterozygous persons carrying the c.463C>T transition (Fig. 2, II:2 and II:7) was performed, since *P2RY5* contains no introns (Fig.7).

### vi) Pharmacological Analysis of the activity of the P2Y5 Receptor

Experiments to identify compounds that activate the orphan P2Y5 receptor may be performed as follows.
A V5 epitope-tagged wild-type human P2Y5 receptor can be stably expressed in Flp-In-CHO cells (vector pcDNA5/FRT/V5-His; Invitrogen, Carlsbad, CA). The cells can be cultured in F12 medium in the presence of 10% FCS and hygromycin 500 µg/ml (Invitrogen, Carlsbad, CA). Control experiments should be performed in Flp-In-CHO cells stably expressing for example the human platelet P2Y12 receptor or the truncated P2Y5 protein p.Lys125AsnfsX37 (vector pcDNA5/FRT/V5-His).
A possible activation of the receptor and, thus, coupling of the receptor to changes in, for example, intracellular adenylate cyclase activity can be assessed by the cAMP response element (CRE)-directed luciferase reporter gene assay 48 h after transient transfection of the cells with a pCRE-luc vector (Stratagene, Amsterdam, Netherlands). The transient transfection is performed in Optimem medium (Invitrogen, Carlsbad, CA) containing 2% FCS using lipofectamine 2000 (Invitrogen, Carlsbad, CA) as transfection reagent. Cells are subsequently cultured on 24-well plates and treated with pertussis toxin 200 ng/ml (PTX; Sigma-Aldrich, St. Louis, MO) or its solvent 20 h before the experiment. At the start of the experiment the culture medium is replaced by Optimem medium without serum. Drugs or their solvents (water or 2-propanol or dimethylsulfoxide, DMSO) are added for 3 h (all drugs and their solvents were obtained from Sigma-Aldrich, St. Louis, MO). The reaction is stopped by addition of Bright-GLO luciferase assay solution (Promega, San Luis Obispo, CA) used for cell lysis and the analysis of luciferase activity. Relative light units are measured using a single photon luminometer (Berthold, Wildbad, Germany). The luciferase activity determined for each well with cells is calculated as percentage of control (average activity in cells treated with solvent only). For statistical comparison one-way analysis of variance (ANOVA) followed by the Dunnett's post test is performed using GraphPad Prism version 4.03 (GraphPad, San Diego, CA).

Radioligand binding studies using [³H]-labelled test compounds may also be performed on HEK Flp-In cells (Invitrogen, Carlsbad, CA) stably expressing the P2Y5 receptor (wild-type) or the two truncated proteins (p.Gln155X and p.Lys125AsnfsX37; vector pcDNA5/FRT/V5-His; for culture media see above). For control experiments HEK Flp-In cells transfected with the empty vector are used. The binding assay is performed on intact cells cultured on 12-well plates coated with poly-L-lysine (Sigma-Aldrich, St. Louis, MO) as described previously (G. J. Molderings *et al, in press* (doi:10.1016/j.neuint.2007.04.022)). After washing of the cells with PBS containing bovine serum albumin (0.25% Sigma-Aldrich, St. Louis, MO), the cells are incubated for 30 min at 4°C with 10 nM [³H]-labelled compound in the presence of the solvent used for the compound or in the presence of 50 µM not-labelled compound. The incubation is stopped by washing the cells with cold PBS (three times 0.5 ml). After cell lysis by NaOH 1 M and sodium dodecyl sulphate 0.1% (Sigma-Aldrich, St. Louis, MO), cellular radioactivity is measured by liquid scintillation counting. The values are normalized toward the protein contents of the cell lysates (dpm/mg protein). Specific binding of the [³H]-labelled compound is assessed by the differences in radioactivity measured in cells in the absence and in the presence of not-labelled compound. For statistical comparison one-way analysis of variance (ANOVA) followed by the Dunnett's post test is performed.

### Example 2: Identification of mutations in P2Y5

We performed a genome-wide linkage analysis using 320 highly polymorphic microsatellite markers (as described above), having first excluded a number of candidate loci in this family (table 7). Homozygosity mapping revealed a new gene locus for HS on chromosome 13q14.11-13q21.33, with a maximum LOD score of 3.9 (θ = 0.0) between recombinant markers D13S1233 and D13S634 (Fig. 2 and Fig. 1). Haplotype analysis defined a critical interval of around 28 Mb based on the smallest homozygous haplotype segment shared by the affected siblings (Fig. 2).

**Table 7: Initial exclusion of candidate loci in family 1**

| Disease gene | Chromosomal location | Literature |
|---|---|---|
| Corneodesmosin (CDSN) | 6p21.3 | E. Levy-Nissenbaum et al., Nat Genet 34, 151 (2003) |
| Hairless (HR) | 8p21 | W. Ahmad et al., Science 279, 720 (1998); S. Cichon et al., Hum Mol Genet 7, 1671 (1998) |
| Winged-helix-nude (WHN) | 17q11.2 | J. Frank et al., Nature 398, 473 (1999) |
| Cadherin 3 (CDH3) | 16q22.1 | E. Sprecher et al., Nat Genet 29, 134 (2001) |
| Gene not yet identified | 18p11 | A. Baumer et al., Eur J Hum Genet 8, 443 (2000) |
| Vitamin D receptor (VDR) | 12q13.11 | J. Miller et al., J Invest Dermatol 117, 612(2001) |
| Keratin (KRT) gene clusters | Chr. 12 and 17 | M. Hesse et al., Eur J Cell Biol 83, 19 (2004) |

The identified region of interest contains 61 known genes. After excluding 38 of these by direct sequencing (as described above; see Table 1), a nonsense mutation was identified in *P2RY5* which encodes the orphan G protein-coupled receptor P2Y5. The four affected siblings carry a homozygous C>T transition (c.463C>T) which results in a premature termination of translation (p.Gln155X) (Figs. 4A and 5A), both parents being heterozygous for the mutation. Sequencing of *P2RY5* in two additional HS-families from Saudi-Arabia (families 2 and 3) revealed a two basepair deletion (c.373_374delAA) leading to a frame-shift and a premature termination of translation (p.Lys125AsnfsX37) (Figs. 4B and 5A) in both families. Haplotype analysis using densely spaced genetic markers suggests an ancestor common to both families (Fig. 3A and B, Table 2). The families show a similar pattern of hair loss to family 1 and are described in detail elsewhere (D. Al Aboud, K. Al Aboud, K. Al Hawsawi, A. Al Aboud, Sudan J Dermatol 3, 128 (2005)). We did not detect either of the two mutations in 506 control chromosomes which included 138 chromosomes of Arabian origin.

### Example 3: Analysis of P2Y5

*P2RY5* consists of one coding exon with a putative open reading frame of 344 amino acids (H. Herzog, K. Darby, Y. J. Hort, J. Shine, Genome Res 6, 858 (1996)). Expression analysis in human tissues (as described above) demonstrated that the *P2RY5* mRNA is expressed in heart, brain, placenta, lung, liver, skeletal muscle, kidney, pancreas, spleen, thymus, prostate, testis, ovary, small intestine, colon and peripheral blood leukocytes (Figure 6A).

Furthermore expression of *P2Y5* was assessed using the BD MTE Multiple Tissue Expression Human Array 3. An almost ubiquitous expression of the mRNA was detected (Figure 7). A listing of *P2Y5*-RNA tissue distribution is provided in Table 8.

**Table 8: Tissue distribution of P2Y5 mRNA as determined using a BD MTE Multiple Tissue Expression Human Array 3**

| Tissue | Expression | Tissue | Expression |
|---|---|---|---|
| Whole Brain | + | Kidney | + |
| Cerebral cortex | + | Skeletal muscle | + |
| Frontal lobe | + | Spleen | + |
| Parietal lobe | + | Thymus | + |
| Occipital lobe | + | Peripheral blood leukocyte | + |
| Temporal lobe | + | Lymph node | + |
| Postcentral gyrus of cerebral cortex | + | trachea | + |
| Pons | + | Lung | + |
| Cerebellum, left | + | Placenta | + |
| Cerebellum, right | + | Bladder | + |
| Corpus callosum | + | Uterus | + |
| Amygdale | + | Prostate | + |
| Caudate nucleus | + | Testis | + |
| Hippocampus | + | Ovary | + |
| Medulla oblongata | + | Liver | + |
| Putamen | + | Pancreas | + |
| Substantia nigra | + | Adrenal gland | + |
| Accumbens nucleus | + | Thyroid gland | + |
| Thalamus | + | Salivary gland | + |
| Pituitary gland | + | Mammary gland | - |
| Spinal cord | + | Leukemia, HL-60 | - |
| Heart | + | HeLa S3 | + |
| Aorta | | Leukemia, K-562 | + |
| Atrium, left | + | Leukemia, MOLT-4 | + |
| Atrium, right | + | Burkitt's lymphoma, Raji | + |
| Ventricle, left | + | Burkitt's lymphoma, Daudi | |
| Ventricle, right | + | Colorectal adeno-carcinoma, SW480 | + |
| Interventricular septum | + | Lung carcinoma, A549 | - |
| Apex of the heart | + | Fetal brain | + |
| Esophagus | + | Fetal heart | + |
| Stomach | + | Fetal kidney | + |
| Duodenum | + | Fetal liver | + |
| Jejunum | + | Fetal spleen | + |
| Ileum | + | Fetal thymus | + |
| Ilocecum | + | Fetal lung | + |
| Appendix | + | Yeast total RNA | - |
| Colon, ascending | + | Yeast tRNA | - |
| Colon, transverse | + | E. coli rRNA | - |
| Colon, descending | | E. coli DNA | - |
| Rectum | | Poly r(A) | - |
| | | Human C₀t-1 DNA | + |
| | | Human DNA 100ng | + |
| | | Human DNA 500ng | + |

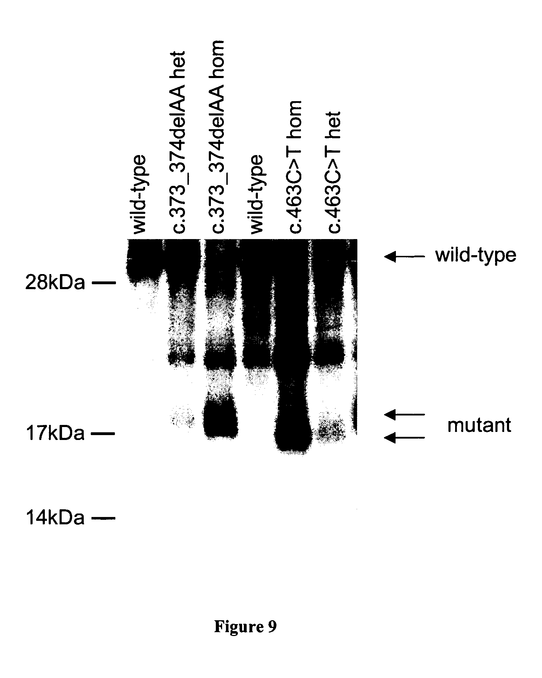

Western blotting of transiently transfected COS7 cell lysates (as described above) showed a signal at about 30 kDa, corresponding to the size anticipated for the wild-type protein. Both mutants also showed bands in their predicted size ranges (Fig. 5B, see also protein truncation test Fig. 9).
Subcellular localization of P2Y5 receptor proteins was studied using immunofluorescence analyses (as described above). We observed staining of the membrane for wild-type P2Y5 which co-localized with cadherins (Fig. 5C). In contrast, staining of the mutants revealed a network-like structure. Through costaining for the protein disulfide isomerase (PDI), a marker for the endoplasmic reticulum (ER), we demonstrated the accumulation of truncated P2Y5 in the ER (Fig. 5D and E).

## Claims

1. A nucleic acid molecule, encoding a polypeptide having P2Y5 receptor function wherein said nucleic acid molecule comprises
a) a nucleic acid molecule encoding a polypeptide having the amino acid sequence of SEQ ID NO:2;
b) a nucleic acid molecule having the DNA sequence of SEQ ID NO:1;
c) a nucleic acid molecule having the sequence of SEQ ID NO:1, wherein each thymidine is replaced by uridine;
d) a nucleic acid molecule that hybridizes under stringent conditions to the complementary strand of a nucleic acid molecule of (a), (b) or (c);
e) a nucleic acid molecule encoding a polypeptide having at least 80% sequence identity to the polypeptide of (a); or
f) a nucleic acid molecule that is degenerate with respect to the nucleic acid molecule of (b), (c) or (d);
for the diagnosis, treatment and/or prevention of hair-loss and the diagnosis of a predisposition for hair-loss.

2. The nucleic acid molecule of claim 1, which is contained in a vector.

3. The nucleic acid molecule of claim 2, wherein the vector is contained in a host cell.

4. A polypeptide encoded by the nucleic acid molecule of claim 1 for the treatment and/or prevention of hair-loss.

5. A pharmaceutical composition comprising
i) a nucleic acid molecule encoding a polypeptide having P2Y5 receptor function wherein said nucleic acid molecule comprises
a) a nucleic acid molecule encoding a polypeptide having the amino acid sequence of SEQ ID NO:2;
b) a nucleic acid molecule having the DNA sequence of SEQ ID NO:1;
c) a nucleic acid molecule having the sequence of SEQ ID NO:1, wherein each thymidine is replaced by uridine;
d) a nucleic acid molecule that hybridizes under stringent conditions to the complementary strand of a nucleic acid molecule of (a), (b) or (c);
e) a nucleic acid molecule encoding a polypeptide having at least 80% sequence identity to the polypeptide of (a); or
f) a nucleic acid molecule that is degenerate with respect to the nucleic acid molecule of (b), (c) or (d);
ii) a vector comprising the nucleic acid molecule of (i);
iii) a host cell comprising the vector of (ii); or
iv) a polypeptide encoded by the nucleic acid molecule of (i).

6. A method of treating and/or preventing hair-loss comprising administering the pharmaceutical composition of claim 5 to a subject in need thereof.

7. Use of
i) a nucleic acid molecule encoding a polypeptide having P2Y5 receptor function wherein said nucleic acid molecule comprises
a) a nucleic acid molecule encoding a polypeptide having the amino acid sequence of SEQ ID NO:2;
b) a nucleic acid molecule having the DNA sequence of SEQ ID NO:1;
c) a nucleic acid molecule having the sequence of SEQ ID NO:1, wherein each thymidine is replaced by uridine;
d) a nucleic acid molecule that hybridizes under stringent conditions to the complementary strand of a nucleic acid molecule of (a), (b) or (c);
e) a nucleic acid molecule encoding a polypeptide having at least 80% sequence identity to the polypeptide of (a); or
f) a nucleic acid molecule that is degenerate with respect to the nucleic acid molecule of (b), (c) or (d);
ii) a vector comprising the nucleic acid molecule of (i);
iii) a host cell comprising the vector of (ii); or
iv) a polypeptide encoded by the nucleic acid molecule of (i)
for the manufacture of a pharmaceutical composition for treating and/or preventing hair-loss.

8. A method for the identification of a compound useful in the treatment of hair-loss or as a lead compound for the development of an agent for treating hair-loss comprising the steps:
i) determining the level of P2Y5 receptor protein or *P2RY5* transcript in a cell wherein said cell comprises *P2RY5* DNA in expressible form;
ii) contacting said cell with a test compound;
iii) determining the level of P2Y5 receptor protein or *P2RY5* transcript in said cell after contacting with the test compound; and
iv) comparing the P2Y5 receptor protein or *P2RY5* transcript level determined in step (iii) with the P2Y5 receptor protein or *P2RY5* transcript level determined in step (i), wherein an increase of P2Y5 receptor protein or *P2RY5* transcript level in step (iii) as compared to step (i) indicates that the test compound is a compound useful in the treatment of hair-loss or as a lead compound for the development of an agent for treating hair-loss.

9. The method according to claim 8 wherein said cell comprises the nucleic acid molecule as defined in claim 1 fused to a reporter gene.

10. A method for the identification of a compound useful in the treatment of hair-loss or as a lead compound for the development of an agent for treating hair-loss comprising the steps:
i) contacting a cell containing P2Y5 receptor protein and a P2Y5 target molecule with a test compound; and
ii) determining the level of activity of the P2Y5 target molecule before contacting the P2Y5 protein with the test compound and after contacting the P2Y5 protein with the test compound, wherein an increased activity of the target molecule after contacting the P2Y5 protein with the test compound as compared to the level before contacting the P2Y5 protein with the test compound indicates that the test compound is a compound useful in the treatment of hair-loss or as a lead compound for the development of an agent for treating hair-loss.

11. A nucleic acid molecule deviating from the nucleic acid molecule of claim 1 by at least one mutation, wherein said mutation results in a loss of function of the polypeptide encoded by the nucleic acid molecule of claim 1 and is selected from:
i) a substitution;
ii) a deletion;
iii) an inversion; and/or
iv) an insertion;
and wherein said mutation is causative and/or indicative of hair-loss.

12. The nucleic acid molecule of claim 11, wherein said substitution is a cytosine to thymidine exchange at a nucleotide position corresponding to position 463 of the nucleotide sequence of SEQ ID NO:1.

13. The nucleic acid molecule of claim 11 or 12, wherein said deletion is a deletion of the adenosine at a nucleotide position corresponding to position 373 of the nucleotide sequence of SEQ ID NO:1 and/or a deletion of the adenosine at a nucleotide position corresponding to position 374 of the nucleotide sequence of SEQ ID NO:1.

14. A vector comprising the nucleic acid molecule according to any one of claims 11 to 13.

15. A host transformed with the vector of claim 14.

16. A method of producing a polypeptide comprising culturing the host of claim 15 under suitable conditions and isolating the polypeptide produced.

17. A polypeptide encoded by the nucleic acid molecule according to any one of claims 11 to 13 or produced by the method of claim 16.

18. An antibody, aptamer or phage that specifically binds to the nucleic acid molecule according to any one of claims 11 to 13 or the polypeptide of claim 17.

19. An oligo- or polynucleotide comprising or consisting of an oligo- or polynucleotide selected from the group consisting of:
(a) an oligo- or polynucleotide consisting of at least 10 consecutive nucleotides of SEQ ID NO:5 or 7; wherein the at least 10 consecutive nucleotides contain a T at position 463 of SEQ ID NO:5, or a G at position 373 of SEQ ID NO:7;
(b) an oligo- or polynucleotide hybridizing under stringent conditions to at least a portion of the oligo- or polynucleotide of (a), wherein said portion comprises the nucleotide in position 463 of SEQ ID NOs:5 or the nucleotide in position 373 of SEQ ID NO:7, wherein said oligo- or polynucleotide contains a T at position 463 of SEQ ID NO:5 or a G at position 373 of SEQ ID NO:7; and
(c) an oligo- or polynucleotide identical to the oligo- or polynucleotide of (a) or (b) with the exception that T is replaced by U.

20. A diagnostic composition comprising the nucleic acid molecule according to any one of claims 1 or 13 to 15, the polypeptide of claim 4 or 19, the antibody, aptamer or phage of claim 20 or the oligo- or polynucleotide of claim 21.

21. A method for testing for the presence of the nucleic acid molecule according to any one of claims 11 to 13 or the polypeptide of claim 17 comprising assaying a sample obtained from a subject for the presence of said nucleic acid molecule or polypeptide, wherein the presence of the nucleic acid molecule according to any one of claims 11 to 13 or the polypeptide of claim 17 is indicative for hair-loss.

22. The method of claim 21, wherein said sample is blood, serum, plasma, saliva, urine, mucosal tissue or mucus.

23. Kit comprising at least one of
a) the nucleic acid molecule according to any one of claims 11 to 13;
b) the vector of claim 14;
c) the host of claim 15;
d) the polypeptide of claim 17;
e) the antibody, aptamer or phage of claim 18; and/or
f) the oligo- or polynucleotide of claim 19;

24. The nucleic acid molecule according to any one of claims 1 or 11 to 13, or the polypeptide of claim 4, or the method according to any one of claims 6, 8 or 10 or the use of claim 7, wherein the hair-loss is selected from the group consisting of hypotrichosis simplex of the scalp (HSS), hypotrichosis simplex, generalised form (HSG) ), alopecia universalis congenitalis, papular atrichia, hypotrichosis Marie Unna and monilethrix.


SEQUENCE LISTING

<110>  LIFE & BRAIN GmbH

<120>  Maintenance of hair growth and treatment of hair-loss

<130>  N2080 EP

<160>  97

<170>  PatentIn version 3.4

<210>  1
<211>  1035
<212>  DNA
<213>  homo sapiens

<400>  1
atggtaagcg ttaacagctc ccactgcttc tataatgact cctttaagta cactttgtat      60

gggtgcatgt tcagcatggt gtttgtgctt gggttaatat ccaattgtgt tgccatatac     120

attttcatct gcgtcctcaa agtccgaaat gaaactacaa cttacatgat taacttggca     180

atgtcagact tgctttttgt ttttacttta cccttcagga ttttttactt cacaacacgg      240

aattggccat ttggagattt actttgtaag atttctgtga tgctgtttta taccaacatg     300

tacggaagca ttctgttctt aacctgtatt agtgtagatc gatttctggc aattgtctac     360

ccatttaagt caaagactct aagaaccaaa agaaatgcaa agattgtttg cactggcgtg     420

tggttaactg tgatcggagg aagtgcaccc gccgtttttg ttcagtctac ccactctcag     480

ggtaacaatg cctcagaagc ctgctttgaa aattttccag aagccacatg gaaaacatat     540

ctctcaagga ttgtaatttt catcgaaata gtgggatttt ttattcctct aattttaaat     600

gtaacttgtt ctagtatggt gctaaaaact ttaaccaaac ctgttacatt aagtagaagc     660

aaaataaaca aaactaaggt tttaaaaatg atttttgtac atttgatcat attctgtttc     720

tgttttgttc cttacaatat caatcttatt ttatattctc ttgtgagaac acaaacattt     780

gttaattgct cagtagtggc agcagtaagg acaatgtacc caatcactct ctgtattgct     840

gtttccaact gttgttttga ccctatagtt tactacttta catcggacac aattcagaat     900

tcaataaaaa tgaaaaactg gtctgtcagg agaagtgact tcagattctc tgaagttcat     960

ggtgcagaga attttattca gcataaccta cagaccttaa aaagtaagat atttgacaat    1020

gaatctgctg cctga                                                     1035

<210>  2
<211>  344
<212>  PRT
<213>  homo sapiens

<400>  2

Met Val Ser Val Asn Ser Ser His Cys Phe Tyr Asn Asp Ser Phe Lys
1               5                   10                  15

Tyr Thr Leu Tyr Gly Cys Met Phe Ser Met Val Phe Val Leu Gly Leu
            20                  25                  30

Ile Ser Asn Cys Val Ala Ile Tyr Ile Phe Ile Cys Val Leu Lys Val
        35                  40                  45

Arg Asn Glu Thr Thr Thr Tyr Met Ile Asn Leu Ala Met Ser Asp Leu
    50                  55                  60

Leu Phe Val Phe Thr Leu Pro Phe Arg Ile Phe Tyr Phe Thr Thr Arg
65                  70                  75                  80

Asn Trp Pro Phe Gly Asp Leu Leu Cys Lys Ile Ser Val Met Leu Phe
                85                  90                  95

Tyr Thr Asn Met Tyr Gly Ser Ile Leu Phe Leu Thr Cys Ile Ser Val
            100                 105                 110

Asp Arg Phe Leu Ala Ile Val Tyr Pro Phe Lys Ser Lys Thr Leu Arg
        115                 120                 125

Thr Lys Arg Asn Ala Lys Ile Val Cys Thr Gly Val Trp Leu Thr Val
    130                 135                 140

Ile Gly Gly Ser Ala Pro Ala Val Phe Val Gln Ser Thr His Ser Gln
145                 150                 155                 160

Gly Asn Asn Ala Ser Glu Ala Cys Phe Glu Asn Phe Pro Glu Ala Thr
                165                 170                 175

Trp Lys Thr Tyr Leu Ser Arg Ile Val Ile Phe Ile Glu Ile Val Gly
            180                 185                 190

Phe Phe Ile Pro Leu Ile Leu Asn Val Thr Cys Ser Ser Met Val Leu
        195                 200                 205

Lys Thr Leu Thr Lys Pro Val Thr Leu Ser Arg Ser Lys Ile Asn Lys
    210                 215                 220

Thr Lys Val Leu Lys Met Ile Phe Val His Leu Ile Ile Phe Cys Phe
225                 230                 235                 240

Cys Phe Val Pro Tyr Asn Ile Asn Leu Ile Leu Tyr Ser Leu Val Arg
                245                 250                 255

Thr Gln Thr Phe Val Asn Cys Ser Val Val Ala Ala Val Arg Thr Met
            260                 265                 270

Tyr Pro Ile Thr Leu Cys Ile Ala Val Ser Asn Cys Cys Phe Asp Pro
        275                 280                 285

Ile Val Tyr Tyr Phe Thr Ser Asp Thr Ile Gln Asn Ser Ile Lys Met
    290                 295                 300

Lys Asn Trp Ser Val Arg Arg Ser Asp Phe Arg Phe Ser Glu Val His
305                 310                 315                 320

Gly Ala Glu Asn Phe Ile Gln His Asn Leu Gln Thr Leu Lys Ser Lys
                325                 330                 335

Ile Phe Asp Asn Glu Ser Ala Ala
            340

<210> 3
<211> 3003
<212> DNA
<213> homo sapiens

<400> 3
tggattagga gagcttctgg atagctgaac acatggaggt ttctggaggg tgtggccccc 60

agagggcatg gaagctctgc acccctgctc ccatacctca ccctacgcat ctcctcatct 120

gtatcctttg caatatcctt tgtaataaac cgaaaatttt atcataatga gcatcaaaag 180

aatcccaaat gagaaaactg ctttttgcat aagtgtaaat taagagatgg aagtcacatt 240

tcaccatggc aaacatttgc tgcatgcaaa atgactgaaa caaggtcttg atctgtcacc 300

aggctgaagt gcagtggtac aatcaaagct cactgcagtc tcaacctcct aggctcaaac 360

aatcctccca cttcagcctc ccaaccagct gggaccacag gttgacatca ccacacccag 420

gtctgctgga atttgctgga ggtccactcc agaccctgtt tgtctggata tcaccagcag 480

aggttgcaga acaacaaaga ttgctgcctg ttccttcctc tggaagcttt gtcccagagg 540

ggcacccacc agacggcagc tggagctctc ctgtgtgcca ctggagtacg aaaaagaaaa 600

aatcctgcag ctagctcggt gtctgcccaa acggctgccc agttttgtgc ttgaaaccca 660

gggcctgggt ggtgtaggca cccgagggaa ttgcctggtc taagggttgt gaagatcacg 720

gaaaaggcat agagtctggg ccggaatgca tcgttcctcg tggcacattc cctcatggct 780

tcccttagct aggggaggaa gttccctggc cccttgcact tcccgttccc tctgctatgg 840

ctcttcctca gtagaaacaa ctggcaacaa aattcaagtt tatgattcat tcatcagcaa 900

acatgtgaga atcatctaca aagaaccaag aattgtgaga aagcgacctc aagatacaac 960

tggcaactga ggaaaaggcc tcaattcaac aagagctaac aagcttggga gtttatttcg 1020

gaatctttaa aagactcttc tgcttaccca caatctggga tccactgcag gaaaacaaaa 1080

aaggaaaact tcatttaaaa gaagcaagaa gtaaaatggg acaaattggg aatgtttaag 1140
tctctgaaac tctgcactga aaagaaaata agattgataa cttaagctta acattctgag 1200
gcataaagaa acattaactt tggagtattc atcttgacta ctgaaataca agtttagaag 1260
acaagtggtt tcattctggt cacagatcac agcttttctt taaatttata atcctatggg 1320
ttggactcgt tgactgtatt ttttaaaggt tgctcgtcag ttaactgagc cttggaattc 1380
atggattttc taaagactaa caaatgaaaa tattttcctg ttgaagaacc cagcggaaat 1440
tttacagcaa caaatttcat gtttcttttg ggtatttctg agaaaaagga aatatttata 1500
aaaccatcca aagatccaga taatttgcaa ataaattgga ggttatagag gttataatct 1560
gaatcccaaa ggagactgca gctgatgaaa gtgcttccaa actgaaaatt ggacgtgcct 1620
ttacgatggt aagcgttaac agctcccact gcttctataa tgactccttt aagtacactt 1680
tgtatgggtg catgttcagc atggtgtttg tgcttgggtt aatatccaat tgtgttgcca 1740
tatacatttt catctgcgtc ctcaaagtcc gaaatgaaac tacaacttac atgattaact 1800
tggcaatgtc agacttgctt tttgttttta ctttaccctt caggattttt tacttcacaa 1860
cacggaattg gccatttgga gatttacttt gtaagatttc tgtgatgctg ttttatacca 1920
acatgtacgg aagcattctg ttcttaacct gtattagtgt agatcgattt ctggcaattg 1980
tctacccatt taagtcaaag actctaagaa ccaaaagaaa tgcaaagatt gtttgcactg 2040
gcgtgtggtt aactgtgatc ggaggaagtg cacccgccgt tttgttcagt tctacccact 2100
ctcagggtaa caatgcctca gaagcctgct ttgaaaattt tccagaagcc acatggaaaa 2160
catatctctc aaggattgta attttcatcg aaatagtggg atttttattt cctctaattt 2220
taaatgtaac ttgttctagt atggtgctaa aaactttaac caaacctgtt acattaagta 2280
gaagcaaaat aaacaaaact aaggttttaa aaatgatttt tgtacatttg atcatattct 2340
gtttctgttt tgttccttac aatatcaatc ttattttata ttctcttgtg agaacacaaa 2400
catttgttaa ttgctcagta gtggcagcag taaggacaat gtacccaatc actctctgta 2460
ttgctgtttc caactgttgt tttgacccta tagtttacta ctttacatcg gacacaattc 2520
agaattcaat aaaaatgaaa aactggtctg tcaggagaag tgacttcaga ttctctgaag 2580
ttcatggtgc agagaatttt attcagcata acctacagac cttaaaaagt aagatatttg 2640
acaatgaatc tgctgcctga aataaaacca ttaggactca ctgggacaga actttcaagt 2700
tccttcaact gtgaaaagtg tctttttgga caaactattt ttccacctcc aaaagaaatt 2760
aacacatgga cattttaaag tctttagtat aaagaaaatt tgtattcaat gtgttaagca 2820
ttaacatgta ttttatttgt gtatccactc catctgattt ttctgagcca ttttgatttg 2880
ttccttcatt aaaaaaaatc tcttaaagtt atttagtgtc taaaagtgac tgacttaaat 2940
tatgtggtgc caatctgtaa tgtctttgaa ttccttttta tattaaatga tttaatttac 3000

taa                                                                 3003

<210>  4
<211>  35659
<212>  DNA
<213>  homo sapiens

<400>  4
agaaaaatcc tatgaaagaa cagggttctc tcatgaagaa taaacaggga gatccaggtg      60

gatggggtgg gttcaaggaa ggctgatctg ggaaggtaac atggacgctg agtcttgaaa     120

tgtgagaagg aggctgtgct ttgaaaggag aaggggcaga gatcttccaa aagatgaaag     180

atcatatgct aagaaactga tgttctttca gaaaatgaaa gaggccacca gaactaaagt     240

acccgggtgg gatagtggag aggttgctaa agaagaggtt gctgtatgat cacgcagaat     300

ctgaaagacc atggtaaaac gtttaaatat tctaagagaa acagtaaacc aattacaaca     360

gaaactctgt tttaggataa gtcactaata tagagatagc tagttcaatt gtgtctggct     420

tcctatcaca tcactagcac ttagtacaga attggggtcc taaaaatatt tggcaatgat     480

gacctgtgtt gctttcagga aagtattcca agtgataggg tccaccataa tccatattgc     540

tttagctctg ttacaagtga caaaattttc tatcaatttc caacttccta gctccaatgt     600

ctcaagaata tgccaggcgg gtgtaggcac agctccagac agcaaacaac tttggggttg     660

tatgcatata actcaggagt taactctatt gatctgtgaa attaaaaaaa aaaaaactac     720

tatttactca atttacttaa gtctttaact tcatattatg caaaactgaa aatatataaa     780

aactgttgtg ttgttataac gtttggtgtg gtaggcctca ggggcaggcc tcggaccttc     840

tcttgccctc ctttaacctg ttccaaggca ggactctaat tttcccccca tcattgtaaa     900

ttgtgtgtct ttaagaccct ccccagagat ggtttttgcc ctatagtgta ggggaggaat     960

gctgacatca taaaaatccc aatgctgaca tcataaaaac tggattagga gagcttctgg    1020

atagctgaac acatggaggt ttctggaggg tgtggccccc agagggcatg gaagctctgc    1080

acccctgctc ccatacctca ccctacgcat ctcctcatct gtatcctttg caatatcctt    1140

tgtaataaac cggtaagtgt gtttcctttg attttttctt tttgtttttt cagacagtct    1200

tgctctgcgg cccaggcagg agtgcagtgg cacaatctca gttcactgca acctctgcct    1260

cctgggttta ggtgattctc gtgcctcagc ctctggagta gctgggacta caggggcatg    1320

ccaccacacc cagataattt ttgtattttt agtagagacg gggttttgcc atgttggcca    1380

ggctgttctt gaactcctgg cctcaagtga tctgcccacc tcgacctccc aaagtgctgg    1440

gattacaggc ttgagccacc gtgcccagta tccttgagtt ttgtgagcag ctccagcaaa    1500

ttaattgaac ctgaagagtg tggtgtgaga accccaattt gaagccagta ggtcagaagt    1560

tctggaggcc taggatggtg tctgggggta tgaggtatgg gcactgagac cccaaccagt    1620

gggatctgac actatctcca agtagacagt atcagaactg agttggagga tgcccagctg    1680

gtatctgctg cttagtgtgt ggggaaaaaa acccacacat tttgtcacag aagtcttttg 1740
tgttgacaac tgttgtggtg gtatgagagc agaagaaaaa catggtttga gagagttttt 1800
tcctacacat cactatgttc tgggttttta tttcagcaat agcatgtcat caacaaaaat 1860
gtttttcagc ttatcttttt tccaaaagaa aaaaaaatgt atataagcaa tataaaacat 1920
agccaggtct aaaagcaacg gaggtgccta agatggaaaa tttaaggagg catttactct 1980
cacttgcaca atcttagtat gaaatgtacc cttggtgccc tgaacactaa actgacaata 2040
aaagctagtt aatttaagat acactggcaa aatagaatga tgtgtctttt actttcatgg 2100
tttccttaca caagaatgaa agtactttca gtttaaaagg ttttatttga atgtattacc 2160
tcatttaatc ttcaaaataa gtcttaaaat taagtgttat tattcatttt atgaatcaga 2220
aaacttagct attttagtaa actgtgactt aatacattag aaatattttt tgagtgtgga 2280
ataacatttg aagtttatag atattaaaac taaaactctt ttaaacttct ttttaaaaat 2340
aaaaaagcaa atgaatagag aagcttccta gaattaaata ttaagatatt tatgactctt 2400
tctttaaaac atttatttaa tttcttattt ttcagcttta tccatttact gaaaaaggaa 2460
tctttttctt tagctaaaaa atagctaagc atttttttta agtctttcaa aacagtaatc 2520
ctttcgctaa attatagact aagaggagat atttttaaga acataacaat gtttaaagga 2580
atagtaatgt tatatcaatg acataatttt atacacatga acaaacacat tttaaaatgt 2640
gtttcaaatg gatttattaa ttaaatgtct taaaatgtct ttcaaagtgg atttattaat 2700
tgatatcttg gtcaaaattt tcaaagaaaa tgagagatat atcaatttga ttaggtctga 2760
tttcaaaaat ttttatttta ttttaaacta agttactttg tggtgttggg tattttttta 2820
aatatttaaa gtattttgaa aagtgcaata tgtaaaatat attttgtgat tgttctgtta 2880
attttaaaat attgtttaaa gaaaggactt cctaactcaa aattagccaa gatttctaaa 2940
attgggtcca ggtttacttg aggatgtctg atataccagc tctgggaggc agctccaagg 3000
gagatgacaa aattcatgta attaaactaa aatagtcact tatctgaatg aggtgttaac 3060
agaagtcaaa gtcctcaccc atcattacta tcttcaattc agtgagggtc ttcctccttt 3120
ctaatagcca ggaaaaaaac cttgttaaag agtttttgtg gaatttatcc taagtacagt 3180
aggaaagtta ccttctgaat ccaaagaaaa aaatcctgtt ctaactgaaa taagacaaaa 3240
atatctatga tgaatatttg caaaatgaat aaaaactcat actatatcca tcctggctaa 3300
cacggtgaaa ccccatctct acaaaaaata caaaaaatta gccgggcatg gttgcatgcg 3360
cctgtagtcc cagctactcg ggaggctgag gaaggagaat ggcgtgaacc caggaggcag 3420
agcttgcagt gagtggagat ggtgccactg cactccagcc tgggcaacag agcaagactc 3480
catttcaaaa aaaaaaaata aaattcatac tgtaggaaga attaatacta taaaaggaac 3540
taatacaatg tgagaaacag acattaaaag aggaatagga aagttcttga agctagcatg 3600

tctgaatatg atgaaagtca aagatgcctc accaaatttt gtagatgtag aaggagtggg 3660
ttacgactga atagaaaaac acaagaaggg tgaattcgag tttaaaagtc agaaataaac 3720
atgcttgtac tgctaagtga caaggcatca aattatctat taataatgct catgactaag 3780
atttgtgaat aagttaaaaa caagtgaata aataggtcct ataattaaat gatcaaaatt 3840
aaagtcattt ttaaataatt cctgcctctt aactaggatc tctataccac agaatgaaaa 3900
tcaccaggtt tttgccaatg ggaatctaac tggtaccctt taccatatat tataattcta 3960
tgatagcaaa ttgtgtcaag tttaacctat tcctagtgct aaactttttt gttaaaaatt 4020
gcttgatgat taaacacagt atagttttat tttctagtaa ataacactta aaaattaaac 4080
aacactatta aaaattgtat tggtttttgg ggttttgctg cttctacttc taggtggacc 4140
tactccaagt tcccagaatt tcataatcct taaagcttcc aattctgaaa cagaactaga 4200
tctaagacag taggagtagg cttattagct agtattgggt tcctaccaag tgatttttgt 4260
tgatatgcac atgttactac ttctatatgt ttggaaaccc gttggtcatt cttgccctag 4320
tgccttaaaa tcaaaggttg gccagataca atagaaattt gatatgtaaa ggtaacataa 4380
gggtcttcag gcacaattta gtttcaacac atccctcatg aaaaggatga ttagtaccct 4440
tataaaaggg accccagaga gctgccttgt cccttccacc atctgaggac atggtaagaa 4500
ggtgccatct atgaaccagg aaacaggcct ccgccagata tcagatctgc tggaaacttg 4560
atcttggact tcccagcttt cagaactgtg agaaatacat ttctgttgtt tataagccac 4620
caaatctatg gtattttgtt aaagcagact gaacagatcc agtgtaataa gagttcagaa 4680
aatctaatga ttaatttcaa gcttagacta tcaatggcct aggtcatcct tagaatgtct 4740
gcaggattct ttttttcttc cctacatttt aattataagc aaaccttgaa tattacaata 4800
tagggcattc ttagcaatac agatgagagt ttaaaatcat aattttaaaa ttgttttctc 4860
tgataactat gtgttcaatt tgctttttaa gctcctttca ttttctattt attatgaata 4920
cattaatttt ttaaatgaaa agttaagcaa gtataagaga tgtctctttt ttacctttta 4980
acaaagaaaa ccctttatct atgagtgcaa ctacaatttt aaatttaact aagttcttct 5040
tggttaaaac aaaatcatat aattaatact gactctagcc cttgatcata aattctttga 5100
cacagtagaa gaaaggcaag gcagatcgcc ttaccccaat ctttatatat atgaagccaa 5160
atactaaatt tcaaccaaaa aattaaactt tggatcatgt gcccatcagg ctatgcatgt 5220
aaataacaaa cattaaagtt aaaaaatatg cattagctat ttgtacataa gtaaatgaaa 5280
attaacaggg cccaacatag acaatatcat acataaaaga aagatgtctt catcaactct 5340
agcctccctc ctctttgcct cacagaacat agcaaaagtt atctcccaaa tttcaaccac 5400
ttctatctca ggaaaaatag ttaatatttt gtagaacatc tgactttagt tttcggttat 5460
attttatact gtaaactcct atttcctgat atggcttgta atttatttgg gaggcagcat 5520

atatcagagt ccccaaggaa gctttattaa acatgcatgc caggtttccc ttttactccc 5580

aagattttga tatacccaaa aagagagaat atctacatgt aagaaaagct cctaagcaga 5640

ttctaatgta atcctactat tctatctcct tcctacccta ttcctgatac ttctctcata 5700

tacatataaa aatatttaga cttatgtata tatgtatgca tgtatctatc taatctcctc 5760

caccacctcc ttaagaagta taaactatat ttctgcattt ttctaattct gatataaatt 5820

tctgaaaagt gacttaatat tctttcttaa atagcaatat tgtgataaca gttcaatttc 5880

ttgtcttctt aggttcaaaa aggccttctt tcaaaccttc cccaaagcct attttctgga 5940

acactaaaaa ctcaggcaaa tttgaggtct atcaaatgta aaaagtacag actctactgt 6000

gaatctcaga aattctctaa atcataacac attcaatagc tgacagaaga cttttgtttc 6060

tctttttccc agcatttggg ctaaagtgat gatacggtaa gaaggcagaa tattctgaac 6120

atttcattaa ctttagttta tgaggattaa tttttaactt tatcccagac tcagtgggca 6180

aaaatgttgt gtggactggt gaggatattg cactataagg aagattaaca accgtaatct 6240

ttctatcatg cctgatttcg ataactatga ttaatgattc aaagtcatta ccttatactt 6300

catgattcta ttttatttct ctcactacct cactaccaaa attctgtctt catgattatc 6360

ttaatttaat aatagtacta gttaatattt attgaatatc tactatatgc taggttctat 6420

gctaaatatt ttacatatat tatctcattt aatcttcaaa atacgtccat aaagtagata 6480

tgtgtatttt atagaaatta taaattttgc ccatattatt atggctgata agtgatggag 6540

cagtgattca aacccaagtc tacctgtttc taaaacttgt gccgttagct gctatactag 6600

tatgggagtc acgttccttt gcttactgcc tctttcactc tttctttgcc cattgtcatg 6660

cttttaacca tgtttctagg ttgctgttta cccctacaat tagttccact ggccacaatt 6720

ccatctatca ttcattcact gatccattca tctagagatg ctaagagaaa tagcacattc 6780

atagtagtac ccactacata gagttgttgt gaaaataaaa taacctatat aaaaagtttg 6840

gtatgataca tggcacatga taagcaatca ataattctac cctcaaagag cttaacagat 6900

aacatttatt gaatgccaga catagcacca accacattac ttatattatt taattctcac 6960

aacaaactct agaaagaagg taattatttt ttaaatgcca ttttataagt gaagaaaggt 7020

agacaaggag taaattgtcc agtcacatga ataagtggtg gatccaggac acagttccaa 7080

actgtttgat ttcagagcct ttgttttgaa cttttatgct atagtttaca gtctaatgaa 7140

aaaaaaaaca gataagtaaa taaaaaatag caatacaaag tgctaagtac cagaataaaa 7200

gaatacacag ggtgccatga aaatcagaag agacaactaa ctcagattag gagcattaga 7260

gaaaacttct tggaagagat tatgcttgag ttgaatccta aagaaagaac aaattatttt 7320

aagtaaaaag cagaagaata gtgtgctcag aagcaagaga agaaaaaata atatggccct 7380

ggggaaactt catgcagcta atctataaga taagaaaaat aatgcttact atagggctgt 7440

tttgacaact aagttagata ataaatgtaa agtgcttaga agggcacctg gcacacaagc 7500
ctttgagaca gaaatttttc tttccataat gctaaaatac agatttcata gcaaggaaat 7560
agcaaaatat aaactcgctg aaggatttta gacaggtgac taccagggtt cagatgggac 7620
tttcagggag ttcttaaaat aaaattccag tgccaagcat ataatggaca cttggtaaat 7680
atttctagaa tattgacata tgatgcagcc atatcatgag tcaaattaac tacagcaaag 7740
cacaaacaag aaaactgata ataggatttg ttttctaatt gacactaagt cataactatg 7800
gcagccagcc tccaagatca ccccaataat ctctgacact gggtatttat atccttgtgc 7860
agacagttcc taaactgtac cagggttagt ctgtgggcca atgacatagg gcagaagtga 7920
tggcatgtca cttctgagat gaatttataa aaatctgcag tttccatctt aggttctctc 7980
cctctctagg atctcttcca ctggggttga ggcagggagc tgctatggag tagccctacg 8040
gagaggctca catggcaaaa aaaatgaagc ccctggccat cagccagcaa agaactgagg 8100
cctgccaaca accacacgag tgaacttgga agtagactct ccaatcccag tgaagcctta 8160
aactagctgc agccttggtt gacaatttaa gaatctctca tgaggaaaca tgagtcaaaa 8220
tcacacagta aagccactcc cagtttcccg actttcagaa tttgtgtgag ataataaatg 8280
ttagttgttc taacagataa ttaatatagc aattgatctt ttttaactat aaagaaaata 8340
tttcatggaa tacttaactt ttgaaagtaa aatatgtata tttctttcta aagcattgtt 8400
tctatccagg acttgggcag tagttttctc ttgtactcaa ctcatgttat tacttatcta 8460
ctcaaattaa ctgtcagcct aattgctctc tgaacaacgc ttcactctgt tttgctactt 8520
agtcttgtga aggaaaccag gctctcactg tcagcaagat tagtataaat ttcactgggt 8580
aaataatatt gatagaaaaa gtagaagcta taagtctgtt ctacatgact tttcataact 8640
aacatacatt tctaaggcaa gcatgaactt gtctagaaag taaaggactc caaggctatt 8700
aagtcaaggt gctcctaaaa gagattctga ttatcaaagt aagtacaagt cagtagtgct 8760
agtagaaaaa ttaacaatag aggaagtgga aggcagaaat tgctattgtt ttctgggtat 8820
tttaaggaat attattagaa ctacattaca ggaaatgttt ttgtgccacc ctaagctaaa 8880
ttctaagact gttttgcctc gcaagaggaa actagaatag aaaatagaag aattttggca 8940
gaccttggcc tttctgaaga tcttttccat tttaccttgc tggtccttta atactaatat 9000
ttctgcagat ctgcttgcca atacatatgc tggctgagcc actatcatga tttttttttt 9060
taagagagag tcttgctctt gtcacccagg ctggagtgca gtggtgcaat cttggctcac 9120
tgcaacctcc gtctcccggg ttcaagcgat tctcctgcct cagcctcccg agtagctggg 9180
attacaggca tgtgccacca tactcagcta atttttgtat ttttagtaga gatgaggttt 9240
caccatgttg gccaggctgg tctcgaactt ctgaccgcag gtgatccgcc tgccttggcc 9300
tctcaaagtg ctgagattac aagcatgagc cacagcaatt ggcctcatga ttttaactat 9360

atctatatta ttcctaagtc tacatttctg actaaaacta tgctttcaag ttctagatcc 9420
atatttccaa ctggacacca aagatatcca catgaatatg tggcaaacat ctcaaatata 9480
ataggttcaa acattaacat gaactcattt ccttcatcac caaccagcta tgagatggtc 9540
tttcaatacc tactttggtt aattgtgtta tcaaccacct aatcacccat agtacaaatc 9600
ttaaaattat cctcagttaa cctcagttcc atccaggacc taatcttttc attcatcctt 9660
ttaacatatt cgtgtcttta tatttacaat agatttcttt tagatagcat agagttgggt 9720
tttgtttttc atccactctg ataatctctg ccttttaatt gggctgctta gagcactgac 9780
atttaatgca attattaata taattagttt aaattctaca tctatttgtc tcatcttttc 9840
tttgttccct ttttgacaaa gatacaaaag caaatcaata gaaaaaggat agcctttaaa 9900
ataaatggtg ctgaaacaac tgaacatcca tggtgcccaa ctcagcaccc cctaaaaaaa 9960
cctcaaccca aatctcaaac tttgtacaaa aataaactta aaatggatca tggacttaaa 10020
atgtgaaact ataatttttt aagtataagg gaaaatcttc aggatctgga gttcttagat 10080
ttgacaccaa aagcataatc cataaaagga aaaagtgata aactggactt tatgaaaatt 10140
aaaaactttt ggatgagaaa catgatgtga agaagatgaa gaaagaagct acaggccaag 10200
agaaaatatt tgctaaccac atatctaaca aaaaagacta gtattgagaa catataaaga 10260
accctcaaaa ctcaatggta aaaaacaaac aatccaatca gaaaatgagc aaagacatga 10320
agagaaattt caccagagag gaaatacagg tcacaaataa acaggaaaag gttaatcaat 10380
atcattagct atcatcctga tgggaaatgc aaattataac ctcactgaga tatccttaca 10440
ctatcagaat gactaaaata aaaaataaag acatcaccaa atgatggaaa gaatgcagag 10500
aaactggatc actcactcgt atattgctgg cggaaattta aaatgattca accactctgg 10560
aaaatacttt tgcagtcttt tatcaaagaa aatgcaaaat aaccatacaa ctcttcaatt 10620
ttactcatta ggcatttatc ccagagaaaa gaaaacccat actgatacta aacataaatg 10680
ttcatagcag ctttatttgt gatagccaaa aactagaacc tgtcctgata ttcttcaaca 10740
tgtgtctggc caaagaaact gtagtacatt catgctacaa aatactagtc agcaataaaa 10800
aggtatgaac tattgataca tgcaatatcg tgaatggatc tccagaaaat tatactgaat 10860
gagaaaaaaa actaatcctg tttgattcca cttacataac atttttgaaa tgacaaaatt 10920
tgagaattga agggcagatt agcagtggcc aggtgttagg gacaggggta gagaggctgg 10980
agggatatgg gtgcaattgc ttgtggtggt ggatacatgg acctacacat tttcaccagg 11040
tcaagctgcc tggtggaatg gtgtatagtg gaaacccctg aatagtgtct ctctacaggc 11100
ctggtgttcc tgggctgaga aagagtctcg gctgtagggt taaagagact tcctgggcca 11160
gggtgttttt tgtggctggg tgccttctgc tggtttcact tgcttgcctg gagcttccta 11220
atggggaagg gaatcttagg agtcttaggc ttgttggata aaagcacctg aagaaaaatg 11280

actaatgcta tttccagata ttgctgacag agcaatattg aaataatcta tctgttaaaa 11340

tttttttct ccgttttat tatcaagact gaatccaaaa acagattcac tgaagaataa 11400

ttttatttat ttatttatta aatggagaga gattctcact atgttgtcca ggccgttctc 11460

aaactactgg cctcaactga tcctcctgcc tcagcctccc aaagtgtaag gattacaggc 11520

atgagccacc aggcctggct cattgaagaa gaattttaaa acttcaatgt ttcaatttat 11580

aatataataa aatacaaagc tattatgatt ggagaaatta tttattttaa ttctcaacat 11640

tttaagaaca tatattcttg taacctttaa ataaatattg aggccaggca cagtggctca 11700

tgcctataat cccagcactt tgggaggcta aggcaggaga ataacttgag tccaggagtt 11760

caagaccagc ctgggcaaca tagcaagacc ctatctgaaa aaaagtatat atgtatatac 11820

atataaataa aacatattga ggtaataaag acaaaagttg taagatatgc ttcttctcaa 11880

gaagaaacta attcaaagat gatgaatgga ataaatctga ataagccaaa gtccttattt 11940

gtctagttta actgaaagtt ttccaaaaga tatcattgtt ttgtaataca atgcatacct 12000

actaaaggta aaaaaaaaaa aatgcatttt tgttaacagt aaaacaaata aaacagtgga 12060

acagttttat caagaaacaa ttatgctttt gtcataatat ttggtgtaag ttataaaaaa 12120

tggtttcatt taaatacagt ctatcctatg tggcatactt atttaagact tatttagtgg 12180

cagaaattct ctgaacaaaa atttttaca gattcatctc cagcaggtac tctgagggag 12240

tccctgaaaa agctctttta agttacaaag caaggcaaca gaatcccaat tctcttcttc 12300

aaatagtata catcttaaat aagaatttta atgcttctaa tcaaagataa ttttaaaatt 12360

tagagttagt ggtatattta aataatttta aatgttttaa aacatcaaag tacattttcc 12420

tatgttctat atttacccag gtatactcat aaatagaaaa gtacatatgt atctttttga 12480

aaaaaaaatt aacgtctaaa agaatctatt tctaataagt cacgttttag atatttcata 12540

atactgaggt ttagtagctt acatgtatgt tcatatcaga aattaattta tttctcatat 12600

tgtttaaaat gtacagtggt taaaatcact ttgagaaact tatggtatga cagagtttat 12660

aagacacagt agaaagagtt cttatctaag agcacataaa aagaaagagc agtatcccat 12720

tccaaatctt aaagatatta ttctctgatt tcaactatat aagcataata gcaaagactg 12780

aaattaaatc tggaaaatca taaacagcag atttattaga gttgtagggt tatgctatct 12840

tttttccttt taattttatt ctgtctctta taattgcttt tcaagagcct taatagtaat 12900

atctctccta tattataaat taaaatttta ttccaaggaa ataaaatttt atattcctgt 12960

acgcttactc aagcatagtt aggacaaata attgacagaa gtcaaggaag acagttgaca 13020

taggctgtca gatgtccctg ctatcaggga aattaaattc aggagaagaa agttcagtct 13080

cttgaggtaa acaggatgct aaccaaagca ttaaagagta aaagacatga acctaatcac 13140

cgaatggtga gaagagacaa gtcaggtggg aatgtgactg aaatggaact gttgatctgg 13200

ggctctttaa tgagagacag ctactctccg agaagtattt agtttcagaa cactggcaag 13260
tagcccaaaa aaaaaaacaa aaaaacaaaa catgcattcc tattttatga ggtgccatga 13320
aaaataagaa tcagaagcag aaatgtagaa tcttaccaag ttgaaatact gtttagaaat 13380
ttctatttgc cccaaagatc ttggggacat gaacatggtg agtgaatttc tgggaggtga 13440
gctggaaata cagattggag aaggcctttt aaagatgttg atcagttaaa ggttaattcc 13500
ctgagacaac cacatgattc attccttcac ttctttcaag tctctgttca aatgtcccct 13560
tattagcaag gccttctttg acctccctat gtagaacagt aaaagcccac cccctttttc 13620
ctcctaaatt atttttcttc agagaactta tcaccatatg accagttgta taattgttta 13680
tttattgatt gtcttctcca aataaaatat aagctcaggg attttgtttt attcactgct 13740
atattcccag cacctagaaa agcacctggt acacagttac actcaataaa tattttgcta 13800
ctggaaaaaa aaataagtat cagttgaccc agagatgaag acaacataag caggaaaaaa 13860
ttttaatatg cctgtcttaa tttcctattt atatggccag ggcaaaaatc cagcaatctt 13920
tttcggttag gttgaaatag tccacttctg tttaactaaa acaacaacaa acggctgggc 13980
tttaacaggg ggccttttgt agaagccgtc ctttggtctt ttgtttctaa cctttctgga 14040
gtagtgctcg tatcttctag tatagacagg aaaccctcaa attttccaaa catgtgaaaa 14100
cagttgatta aagcaagcac actagaaaac caataaaaaa aagtaaccac agaaattttg 14160
ctctgggaat agatgactag tggcttagaa tcataccaat aattcttcct tacctttgag 14220
tatcagtatc tgttaaagag ctagtctctt tgaattccaa gtgggttaag agcgaaacat 14280
ttatctgcat aaatgcctaa agtcaaaata tgtttttggt tttaagcata cttgctttaa 14340
tgttcatagc actagtcctt aaaaatcaat catctataat aaaaagaaaa gaaagaagtc 14400
taatcaaatg tatagtcatt tttattttga aggccaaaac ttaaaaaaaa tacaaatatt 14460
aaatttgggt acctaaaact gagaaccact tttaagttaa ggacaaaatg atatgcttgt 14520
ttagatgcat ctcccacctt tttgaatggg ttgttatttt ttgttagttt gttctaacag 14580
acttgtttcc ataatttgtc atttaactat tctgtaaatg ttataactca tatgattcat 14640
atgatggact gatgttttca ttccacttta tgttgttctt gcaacaaaat tatttagtca 14700
aacctatctt tttttaaaca ctagatttta tataatactt agaaagcctc tactgctatt 14760
tttaaatata ttattttatt ttaattgtta gagttgggct gtcacccagg ctgaagtaca 14820
gtggcatggc ctactgcagc cccaaactcc tgggctccag aaatcaatcc ttctgcttca 14880
gcctctttgc tgggattaca ggtgtgagcc acagcaggcc tctactgttt tttttttttg 14940
tttgtttgtt tgtttttttg tttttgagat ggagactcac tctgtcaccc aggctagagt 15000
gcagtggcac aatctcggct cactgcaacc tccgccccct gggttcaagc cattctcctg 15060
cctcagcctt ccgagtatct gggactacag gcacgtgcca ccatgcccag ctaatttttg 15120

tatttttagt agagacgggg tttcaccacg ttagctaggc tggtgtcaaa ctcctgacct 15180
cagatgaccc acctgtctcg cctcccaaag tgctgggatt ataggcatga gccacagtgc 15240
ctggcctcta ctgttatttt tctaacgtgc tcattttttt ccagaactta tatggagtca 15300
ttttttaaat ggagtcattt tttattgaga ttattgatct agctagaatt tattttggta 15360
taaagaataa atcaaagtct acctcttttt ttcaaattgc tatcctgata actcatgagc 15420
atttactgaa ttatcctgaa ttttctccac tgatttgaag tggataaatt atcatacatt 15480
aaattcccct gggtatggga ttatttctgg actccttata actaaagaaa tgcaaactaa 15540
acaagatact actttgacct gttaaaagtc taataataat ctggtttggg tgaaggtgtg 15600
gagaatgcaa attaggaggg gatttttttc ttagaaatga aactgctagg tgatataacc 15660
cacagtatct acaggtgaac aaacttgtaa aaacatgcac tgaaaacata ctgagatttt 15720
tactggattg gcattaaatg tatagatttg tcccagaaaa actgcctggc acatgttcaa 15780
gtattctctt atggccttta ttaaagtttt gtagtctttt gctttgtttt catctacatc 15840
ctgcactttg cctattaagc ttattcctaa ggtattttgt actatttgtt attgctgtaa 15900
attgaatatt tttcattaca ttttccagtt gattactgct ggtacataaa ttgtcttatt 15960
tgagagattg gcaccagtta atttaaaaag ctaagcagga aatcatttta aaataatata 16020
tataattcca tataacattt taattttaaa aacacaaaaa gtgtattcat ttgaaaatct 16080
ctttacgtaa ggtcaaggtt ttttgttttt tttttagttc taagttgttt ttcttctata 16140
aaccacacca ataatgtggg tttatgattt tcactatgtg cagggtgagg acagaaaaat 16200
gcaaaggagt ctgaggccta gagacacaat ccttctactt tccgacctgg gtgggttcgc 16260
ccctccttgg gcaacctggt agtcccctgc tcccaggagg tcaccatatt gatgcagaac 16320
ttagtgtgga cacctaatca gcatagcgca cagcccagaa ctcctgggct ccagtgatct 16380
tcccacctca gcctacagag tagctgggac tacaggcatg tgtcactgca cctagcaatc 16440
ctttctagtt tcttacgttg ccccctcata tcttttctac atctaagtct acaatatttt 16500
ctgcaacttg cctctgagtc atttcaaagc accgaacttg gtttgactaa aacaaccctt 16560
ttttgcactt ccattttata acattacaat aatatgaaaa atcagcaaat ataaatcagg 16620
gagtaaacaa tgcttaaact tttaatacac acacaatttc actactggga gcaaaagaat 16680
tcaggtatat cagagaatag ctttatagcc acaagcatgc agcttccagg agcatcctga 16740
gagaccaatc ctttttaact tctacccctc agtaataggt aaaagataca agtcaataag 16800
tgtttacttt aactgaagag tacttggatt tctgataata atctatgctg gaaggtgtta 16860
aatttactat tcacagaaaa cccttgttct aattatttga aagcagcaga gatatttcct 16920
gcttttgtat aaaacttggc acattactat gtacaaatgc aaacttttta aataaataaa 16980
gatgaagcta aaaatgaagg atggtcatca tacgatttta atctacagaa ggatgaaaat 17040

gtctaaaatc tcttaatctt aaatttctat cataatgtta gaatttagtg ttttaaaaag 17100
tctgcagagc ttactcattt aaagacaaag ttctttgctt aatatttacc taagctatgg 17160
accaagtttc ctattttatg tctgatatgg tttcaatgtt tgtcccttcc aaatctcgtg 17220
ttgaaatgtg atccccaatg ctggaggtga ggtttggtag aagatgactc gtgttgaaat 17280
gtgctcccca atgctggagg tgaggtttgg tagaagatga ctcgtgttga aatgtgctcc 17340
ccaatgctgg aggtgaggtt tggtagaaga tgactcgtgt tgaaatgtgc tccccaatgc 17400
tggaggtgag gtttggtaga agatgactcg tgttgaaatg tgctccccaa tgctggaggt 17460
gaggtttggt agaagatgac tcgtgttgaa atgtgctccc caatgctgga ggtgaggttt 17520
ggtagaagat gactcgtgtt gaaatgtgct ccccaatgct ggaggtgagg tttggtagaa 17580
gatgactcgt gttgaaatgt gctccccaat gctggaggtg aggtttggta gaagatgact 17640
cgtgttgaaa tgtgctcccc aatgctggag gtgaggtttg gtagaagatg actcgtgttg 17700
aaatgtgctc cccagtgctg gaggtgaggt ttggtagaag atgactcgtg ttgaagtgtg 17760
ctccccagtg ctggaggtga ggtttggtag aagatgactc gtgttgaagt gtgctcccca 17820
gtgctggagg tgaggtttgg tagaagatga ctcgtgttga agtgtgctcc ccagtgctgg 17880
aggtgaggtt tggtagaaga tgactcgtgt tgaagtgtgc tccccagtgc tggaggtgag 17940
gtttggtaga agatgactcg tgttgaagtg tgctccccaa tgctggaggt gaggtttggt 18000
agaagatgac tcgtgttgaa gtgtgctccc caatgctgga ggtgaggttt ggtagaagat 18060
gactcgtgtt gaagtgtgct ccccagtgct ggaggagagg tttggtagaa gatgactcgt 18120
gttgaagtgt gctccccaat gctggaggtg aggtttggta gaagatgact cgtgttgaag 18180
tgtgctcccc aatgctggag gtgaggtttg gtagaagatg actcgtgttg aagtgtgctc 18240
cccaatgctg gaggtgaggt ttggtagaag atgactcgtg ttgaagtgtg ctccccaatg 18300
ctggaggtga ggtttggtag aagatgactc gtgttgaagt gtgctcccca atgctggagg 18360
tgaggtttgg tagaagatga ctcgtgttga agtgtgctcc ccaatgctgg aggtgaggtt 18420
tggtagaaga tgactcgtgt tgaagtgtgc tccccaatgc tggaggtgag gtttggtaga 18480
agatgactcg tgttgaagtg tgctccccaa tgctggaggt gaggtttggt agatgactcg 18540
tgttgaagtg tgctccccaa tgctggaggt gaggtttggt agaagatgac tacatcaggg 18600
gggtggatcc ctcatgaatg gtttagcacc acccccttgg tgataaccga gctctcgctc 18660
agttagttca caagagatct gcttgtttaa aaagggcatg ggacctcctc cttcctcttg 18720
ctcctattgt caccacgtga catgcctgtt tcccctccga cttctgccat taatataaac 18780
ttcctcagga ctcaccagaa gctgaggaga agctgatgcc atgcttcctg tacagcctga 18840
agaaccgtga gccaactaaa ccctactctt ttctttacaa attacccagt ctcaggtatt 18900
tctttacagc aatgcaagaa cgaactagca caacatcagt aagaaaaaaa gggtgaaagc 18960

tacctgttac tacgagaaca tttgactaga agtcaagaaa ctaggattcg agttcctgct 19020
ccagcaataa tcagctatgg ttttagtttc ctaagttgta cttgagtgac tggaaggaga 19080
gctaaactat ataccaaaga agatgaatgg ctagttgtaa caggggtaag gtggactttt 19140
attctgaatt tctaggatcc agtgctagct ccaacattag aggatattaa gactaatgca 19200
atatgcaatg tgttaaccac caagcaaact aacttagaaa tatatgtggt ttttataagc 19260
aaagcctatg tctatttgat ttaaattggc tgtacattag cagtgctaaa aaaggcatac 19320
caaatgcatc acatgatcac ttctttatct gacagagcac aggcatccta tctggtcaag 19380
aaaacaaaac aaatcttatt gttccatgat ttcccacaac ataaccttta tttctataaa 19440
gttaagatat tataggtgat gtcatctata atagtatctt tttagaggtt tacattcaga 19500
atatgttttc atttcatttt attctgaaaa ctcttcataa ggtcaaaagg gcatgtcata 19560
tccatcttaa agttcagggc agagtttgcc caaggtaagg tcaaaattta gaaatcaagg 19620
attctggatc tctaggtcac agtgctttct attatccttt atgcatgcac tcactaagtt 19680
gtattctttc attatacaaa catgtataag gaactgggtt gggtacttct tgtgaggacc 19740
cagtttttag ttttgacttc gtcattctcc accaataatc actttgtaat cctaagcaag 19800
tcacaacttc tctgaatttt agtttcttgg tctgtaaaat tgaaacaata atatatgttc 19860
tttcttgtca ttgaaaatta gtggggagac tgaattagat agaaagctaa gagctgaaaa 19920
gaattctata ggtctaattt aatactctcc ttttacaaat atacaataga gaagctaaat 19980
tatttgctca aggtcatgtc attttttttt tcctttaaga gttgaggact ctctatattg 20040
cccaggctaa actggaattc ctgggctcaa gagatcctcc tacctctgcc tcctgagtag 20100
gtggggctca ggagacgcac attaattttt agtaacttgg attagaatcc aagtcactta 20160
acacctagtg aaattggtcc tttcattaca ctttgcagcg tctaaactag gtgaaaaatt 20220
tttgaaagct acaacccacc tagccttacc caactaaggt tttatgacca aattcagcct 20280
ttatgccaca aggcatatat tgtatgtgaa gaattaacat cttccaggaa ttcagagtag 20340
gactaggtta catagcatcc ttgagagaat ggagaaaata gtcactcaaa tagttttctg 20400
tgtttttgga tgagaaaggc agcatgacta tattaatggg acattattac caggaatggc 20460
tctagccttc aagacctgaa atttaaaatt tgtattcaac tgtaaaggct agccttcatt 20520
acacatcatc tcctcaagaa gctctttact aaattcgtag attctcatgt ctatgtgtta 20580
ctaatagctg ttgttatatc ttgtattgca attatttctt tgttttgtat tcttttattt 20640
ttcttttttt ttgagacaga gtcttgctct gtcacccagg ctggagtgca gtggcatgat 20700
ctccgtcact gcaacctctg cttcccaggt tcaagtgatt ctcctgcctt agccttctga 20760
gtagctggga ttacaggtgt gagccaccac acctggataa cattttgtat ttttagtaga 20820
gacagagttt caccatgttg gccaggctgg tttcaaactc ctgacctcaa gaggtctgcc 20880

cgccttggcc tcccaaagtg ctaggattac atgcatgagc caccacacat ggccttgttt 20940
ttcattctta attgaattgc acgtatatgt cttaaaaggc aaactaagta ctgtggaagt 21000
acagaaaagg aactgatgag attaaggaga ctccatagaa aagacgtgat atgattctaa 21060
gagttggaga tgaagaagca acaagagagt tcggaaaaaa agataaggaa gaagggacaa 21120
ctcaagtgta tgcacagata aatatgctta ttcaagtttt aaatcttacc acaataacat 21180
tttcactcct cttgatttcc tttccctact tgtttgaacc tgcttcaacc atcattccca 21240
tcaactttta ctttgctcta atcatcattc atcacccttt aaatctttcc tcttctactc 21300
ccatccaact ctataaaagt tggttcaact ctttgaactt gggtagatga ctcacattcc 21360
ggtaaaaagt aacataaagt attaagtgtt tacaataatg aaaggaaggt aaatcacttc 21420
gaaatgatac cgatagactt atggtaatac tagtctagaa tttaagattt tttctcctta 21480
ggctaaaaga tatttttata gaaaataaat atattctgtc aacataaatt acaaatgttc 21540
atttctcaaa ttgaaaaagg tttatttgtt tctataccta aatagaaaat tttatcataa 21600
tgagcatcaa aagaatccca aatgagaaaa ctgctttttg cataagtgta aattaagaga 21660
tggaagtcac atttcaccat ggcaaacatt tgctgcatgc aaaatgactg aagtaagttt 21720
agaataattc tttttaaatt ttaaaatatc aatgtcccat actttttct ttctttcttc 21780
aagagacagt gtctcgttat gttgcccagg ctggagtgct gtggctattc ataggtgtca 21840
tcaaagagca atgaagcccc aaactcctat ggccttaagt gatcctcttg tctcagcctc 21900
tagagtagct tcgactacag gagtgtacca ctatgcctgg ctcacacttt cttttgaact 21960
taaatcaatt tatttctgtt ttaagtctca catactctct catttgattt ttttcttttt 22020
tctttttgca tatcccctgt tctcaatgtt atgtacaatt tcaattatgg ccttcgaaac 22080
ttttaatgag agaattcttt aaagttttaa tcgattcctc tttgatcaat tttatagtaa 22140
aagaacacgg tgtcttattg tggaaaaaaa taggtctttt aaagaagaat gctattgaaa 22200
cataaaggga agccttttac agaatattta atcttataca tgtttcttgt gcaacctgtt 22260
tcaaaaatca gaaatcctta aacataaaga gtgtaaggtg actccttgat ctattatacc 22320
tgagagtgaa aaaacccaac ctgcggaggt tgcagtgagc cgagatcgcg ccactgcact 22380
ccagcctgga tgacagagca agactccgtc tcaaaaacac aaaaacaaaa acaaaaacaa 22440
aaacccaacc cagcagctta agcagtgaaa gtattcaaag aatcagaatc agatgtcatt 22500
aaattttaaa aaccgatgca ttacataatg aaattcacta tatttcattc tttttattta 22560
tttacttatt tatttgagaa caggcctctc tctgtcactc aggatggagt aaaatggtac 22620
aatcatagct caatgcagcc tcaatctccc gggcccaagt gatccaccca cctcagcctc 22680
cctagtaacc aggactacag gtgcccacca ccacacttgg ctaatttttg gaaacagggt 22740
gtcactatgt tgcctaggct ggtcttgaaa tcctggtctc aagtgatcct ctcatctcag 22800

cctcccaaag tgttgggatt ataggtgtga accacagatc ctggccaaga ttcactatat 22860
tttgaattgg ttgtatatgt aggctatatt ctttcctttt tgtttttttt ttagacaagg 22920
tcttgatctg tcaccaggct gaagtgcagt ggtacaatca aagctcactg cagtctcaac 22980
ctcctaggct caaacaatcc tcccacttca gcctcccaac cagctgggac cacaggttga 23040
catcaccaca cccaggtaat ttttaaattt tttatggaga cagggtttcc ctatgttgcc 23100
aaagctgata cactatattt caaatcagtc tacaaggtca ttatggcaac agtcctaaaa 23160
aaaggcttgt cttcaccatt ttttaaaagt attatacttt aagtattggg atacatgtgc 23220
agaacgtgca ggtttgttac ataggtatac acgtgccatg gtggtttgct gcacccatca 23280
acccatcatc tatgttaagt atttctctta atgctatccc tcctctaacc cccaaccccg 23340
acaggccccg atgtgtgatg ttcccctccc tgtgtccatg tgttctcatt gttcagttcc 23400
cacttatgag tgagaacatg cggtgtttgg ttttctgttc ctgtgttagt ttgctgaaag 23460
tgatggtttc cagcttcatc catgtccctg caaaggacat gaactcatcc ttgttcatgg 23520
ctgcatagta ttcgatggta tatatgtgcc acattttctt tatcctgtct atcattgatg 23580
cgcatttggg tgggttccaa gtctttgcta ttgtaaatac tgctgcaata agcatatgtg 23640
tgcatgtgtc tttatattag aattatttat aatcctttgg gtatataccc agtaatgggc 23700
ttgctgggtc aaatggtatt tctggttcta gatccttgag gaatcgccac actgtcttcc 23760
acaatggttg aactaattta cactcccacc aacagtgtaa aagcattcct attctccaca 23820
tcctctccag catctgttgt ttcctgacgt tttaataatc gccattctaa cggccatgag 23880
atggtatctc attgtggttt tgatttgcat ttcattaatg accgctgatg atgagctttt 23940
gttcatatgc ttgttggcca cataaatgtc ttcttttgag aagtgtcagt tcatatcctt 24000
cacccacttt ttgatggagt tgtttgcttt tttcctgtaa atttgtttaa gttccttgta 24060
gattctggac tttagccctt tgtcagatgg atagatcgca aaaattttct cccattctgt 24120
aggttgcctg ttcactctga tgatagtttc ttttgctgtg cagaagctct ttagtttaat 24180
tagatcccat ttgtcaattt tggcttttgt taccattgct tttggtgttt tggtcaggaa 24240
gtctttgccc atgcctatgt cctgaatggt attgcctagg ttttcttcta gggttttcat 24300
ggctttaggt cttacattta actctttaat ccatcttgag ttaatttttg tataaggtgt 24360
aagaaagggg tccagtttca gttttctgca catggctagc cagttttccc aacaccattt 24420
attaaacgga atcctttgtc tcatctttgt caaagatgag atggttgtag atgtgtggca 24480
ttatttctga ggcctctctt ctgttccatt ggtcaatata tctgttttgg taccaatacc 24540
atgctgtttt ggttattgta gccttgtagt atagtttgaa gtcaggtagc atgatgcctc 24600
cagctttctt ctttttgctt aggattgtct tggctatatg ggcttttttg gttccatatg 24660
aaatttaaag tagttttttc taattttata aaggaagtca atggtagctc gatagggata 24720

gcattgaatc tataaattac tttgagtggt atggccattt tcacaagatt gattcttcct 24780

atccatgaac atggaatgtt cttccatttg tttgtgtcct ctttatttcc ttgagcagtg 24840

aagtttgtag ttcttgaaga tgtccttcac atcccttgtg tgttgtatgc ctaggtattt 24900

tattctcttt gtagcatttg tgaatgggag ttcactcatg atttggctct ctgtttgtct 24960

attattgttg tataggaata cttgtgattt ctgcacatta attttgtatc ctgagacttt 25020

gctgaagttg cttatcagct taaggagatt ttgggctgag ataatggggt tttctaaata 25080

gacaatcatt gtctctgcaa acagagacaa tttaatttcc tctcttccta cttgaatacg 25140

ctttatttct tcctcttttc taactgccct gaacagaact tccaatacta tgttgaatag 25200

gagtggtgag agagggcatc cttgtcttgt gccagttttc aaagggaatg cttccagctt 25260

ttgctgattc agtatgatat tggctgtggg tctgccataa atagctctta ttattttgag 25320

atacattcca tcaatatgaa gtttattgag agtttttagc atgaagcagt gttgaatttc 25380

atcaatggcc ttttctgcat ctattgacat aatcatgtgg tttttatctt tggttctgtt 25440

tatgtgatgg attacgttta ttgatttgca tatgttgaac cagccttgca tcccagggat 25500

gaagccgact tgtttgtggt ggataagctt tttgatgtgc tatggattca gtttgccagt 25560

attttattga gggttttcac gtcgatattc atcaaggata ttggccgaaa ttttcttttt 25620

ttgttgtgtc tctggcaggt tatggtatca ggatgattct tggcttataa aatgagttaa 25680

ggaggactct cttttcttct attgcttgga atagtttcag aaggaatagt accagctcct 25740

ccttgtacct ctggcagaat tcagctgtga atccatctgc tcctgggatt tttttggttg 25800

gtacgctatt aattactgcc tcaatttcag aacttgttat tggtctattc agggattcga 25860

cttcttcctg gtttactctt gggagggtgt atgtgtccag gaatttatcc atttcttcta 25920

gattttctag attatttgca tagagatgtt tacagtattc tctgatggta gtttgtattt 25980

ctgtgggatc agtggtgata tcccctttag cattttttta ttgtgtctat ttgattcttc 26040

tctcttttct tctttattag cctggctagt ggtctatcta ttttgttaat cttttcaaaa 26100

aaccagctcc tggattcatt gattttttga agggtttttc gtgtttctct ctccttcagt 26160

tcttctctga ccttggttat ttcttatctt ctgctagctt ttgaattagt ttgctcttgc 26220

ttctctagtt cttttaattg tgatgttagg gtgtcaattt tacatctttt ctgctttctc 26280

ctgtgggcat ttagtgctat aaacttccct ctaaacattg ctttagctgt gtcccagaga 26340

tttgggtatg ttgtgtcttg gttctcattg gtttcaaaga acttctttgt ttctgcctta 26400

atttcattat tttcccagta ttcattcagg aacaggttgt tcagtttcca tgcagttgtg 26460

caggtttgag tgagtttctt aatcctgagt tctaatttga ttgcactgag gtctcagaga 26520

ctgtgttatg atttccattg ttttgcattt gttgaggagt gttttactcc caatcatgtg 26580

gttagtttta gaataagtgt gatgtggtgc taagaagaat gtctattctg ttgatttggg 26640

gtggagagtt ctgtagatgt ctattaggtc tgcttggtgc agagctgagt tcaagtcctg 26700
aatatccttg ttaattttct gtctcattga tctgtcaaat attgacagtg gggtgttaaa 26760
gtctcccact attattgtgt gggagtctaa gtctctttgt aggtctccaa ggacttgctt 26820
tatgaatctg ggtgctcctg tattgggtgc atacatactt aggatagtta gctcttcttg 26880
ttgcattgat ccctttacca ttatgtaatg cccttcttgt cttttttgat cttcgttggt 26940
ttaaagtctg ttttatcaga gactaggatt gcaacccctg cttttttttt tttttgcttt 27000
ccatttgctt ggtaaatctt cctccattcc cttatttaga gcacgtgtct ttgcatgtga 27060
gatgagtctc ctgaatacag cacactgatg ggtcttgact ctttatccaa cttgccagtc 27120
tgtgtctttt aattgtggca cttagcccgt ttacttttaa ggttaatatt gttatatgtg 27180
aatctgatcc tgtcattatg atgctagcca gttagtttgc ccattagttg atgcccatta 27240
gtttcttcat agtgtcaatg gtctttacaa tttggtatgt ttttgcagtg gctgggacca 27300
atttttcctt tccatattta gtgcttcctt caggagctct tgtaatgcag gcctggtggt 27360
gacaaaatct ctcaacattt gcttatctgt gaaggatttt atttctcatt cacttatgaa 27420
gcttagtttg gctggataca aaattctggg ttgaaaattc ctttctttaa gaatgttgaa 27480
tattggcccc cactcttttc tggcctgtag ggtttctgca gagagatcca ctattagtct 27540
gatgggcttc cctttgtagg taacccgacc tttctctctg gctgctttta acattttttc 27600
cttcatttca accttggtga atctgacgat tatgtgtctt ggggttgctc ttcttgagga 27660
gtatctttgt ggtgttctct gtatttcctg aattggaatg ttggcctgtc gtgctaggtt 27720
ggggaaattc tcctggataa tatcctgaag agtgttttcc aacttggttc aattctccct 27780
gtcactttca ggtacaggga caatcaaaca taggtttggt cttttcacat agtcccctat 27840
ttcttggagg ctttgttcgt ttcttttcat tctttttcct ctaatcttgt cttcacactt 27900
tatttcataa aggtgatctt caatctctga tatcctttct tctgctttgg ctgttgatac 27960
ttgtgtatgc ttcacgaagt tcttgtgctg tgtttttcag ctccatcagg tcatttatgt 28020
tcttctctaa actgcttatt ttaggtagca attcctctaa ccttttttca aggtttttag 28080
cttccttgca ttgggttaga acatggtcct ttggcttgga ggagtttatt attctctgtc 28140
cagttttgtt cccttgctgg cgaggagttg tgattctttg gaagagaaaa ggcattctgg 28200
tttttggaat tgtcagcctt tttgcgctgg tttttcctca tcttcatgga tttatctacc 28260
tttggtcttc gatgttggtg accttcaaat ggggtttctg tgtggaagtc ctttttgttg 28320
atattgatgc tattcatttc tgtttgttag ttctccttct aacagtcagg cccctctgct 28380
gcaggtctgc tggaatttgc tggaggtcca ctccagaccc tgtttgtctg gatatcacca 28440
gcagaggttg cagaacaaca aagattgctg cctgttcctt cctctggaag ctttgtccca 28500
gaggggcacc caccagacgg cagctggagc tctcctgtgt gagttgtctg tcgattcctg 28560

ctgggaggtg tttcccagtc aggagacatg ggggtcagag acccacttga ggaggcagtc 28620

tgtcccttag cagagctcga gcaatgtcct gggagatctg ctgctctctt cagagccagc 28680

aggcaggaac gtttaagtct gctgaagctg caccaatagc cacccatagc acccttcccc 28740

caggtgctct gtcccaggga gatggcagtt ttatctataa gcccctgact ggggctgctg 28800

cctttttttc agagatgccc tgcccagaga ggaggactct agagaagcag ttgggctaca 28860

gtgggctcca cccagttcaa acttcccggt ggctttgctt acactgtgag gtgaaaacca 28920

cctactcaag cctcagtaat ggcagaagcc cctcctccca ccaagctcca gcatcccagg 28980

tcgacttcag actcctgtgc tggcagtaag agtttcaagc cagtggatct tagcttgctg 29040

ggctctgtgg gggtgggatc cactgagcta gaccacttgg ctccctggct tcagcccctg 29100

ttccagggga gttaacagtt ctgtctcact ggtgtttcag gtgccactgg agtacgaaaa 29160

agaaaaaatc ctgcagctag ctcggtgtct gcccaaacgg ctgcccagtt ttgtgcttga 29220

aacccagggc ctgggtggtg taggcacccg agggaattgc ctggtctaag ggttgtgaag 29280

atcacggaaa aggcatagag tctgggccgg aatgcatcgt tcctcgtggc acattccctc 29340

atggcttccc ttagctaggg gaggaagttc cctggcccct tgcacttccc gtgtaaggcg 29400

atgccccacc ctgctttggc tcgccttcca tgggctgcac cccctgtctg accagtctca 29460

atgagatgag ctgggtacct cagttggaaa tagagaaatc acccaccttc tgcgttgatt 29520

tcgctgggag ctgcagactc cagctgttcc tgtgttcacc attttaacat ctatctggct 29580

ctgtttaatt ttcgctcccc ccaaaaaatc catataagaa ataagttgat tctcagagtt 29640

aagttccaga agtgtagaga tctcacatta aaataacagt ttgcctgagt ttatcaaata 29700

caaaagtatc tattaaatat aattgattat atggattcat tacctccctt tgcaagtagt 29760

tactctgtat ttttccaatt ctaacctaag aatttttcaa agtttctaaa ttcccctttg 29820

gttccttaat tagaggccaa atttaatatc tggaaaaaat gcagtattca attatcttct 29880

tcctgttcaa acagtccctc tgctatggct cttcctcagt agaaacaact ggcaacaaaa 29940

ttcaagttta tgattcattc atcagcaaac atgtgagaat catctacaaa gaaccaagaa 30000

ttgtggtaag aattatacaa acatttacta tgatactgat aataatgaaa agcctaattc 30060

aacaaatagt gatctatcca tttttaagcc ccagtaactc aaacattaaa aaactataaa 30120

tgaacaggcc gggcacggtg gctcacacat ggaatcccag cacttgggga ggctgaagtg 30180

ggtggatgac ttgaggtcag gagttcaaga ccagcctggc cacatggtaa aaccctatct 30240

ctactaaaaa tacaaaaatt agctgggtgt ggtggcgggt gcccataatc ccagctactc 30300

gggaggctga ggtgggaaaa ttgctggaac ctgggaggca gaggttgcag taagacaaga 30360

tcgcaccact gcactccagc ctgggcggca gagtgagaaa aaaaaaagaa agaaagaaaa 30420

agaaaaagaa aagaaaagaa aaaactatag atgaatgact taagtgaata acaagtcaag 30480

20

tgagtaatga caatatgtag aaaaatattc caagtagaat taataaattt taattatttg 30540

aatgagaaaa tgccgccata ttcaaaatga aagtaaaagt aggatgagat cagcatatac 30600

aactgcatat atttcattaa ttaaagatga ctaaagagga gactatcaat ttgaaagaag 30660

attactaaga ttttgttttt ttaaaacata ctgaaccaaa taaaaaaaaa ataacagtaa 30720

gacataacta ctaacagaaa atttttccca atgagaatac aagcttatta accactcctt 30780

ttttcattaa tgctcactca gcttctacca gccttctaat aagaacacac acatacatct 30840

tttctatacc aaatgtgata taaaagatat tcaacccttc aaaattttaa gtattggtta 30900

taagtaatac tcatcttttt ttgtcatttg ataatagatg aattttctag tccagacagt 30960

agaaatagat tatagttcat caacaaatga caaaaattgc tgagtgtgaa agaatggtgc 31020

aaaacaacac aaaacatttt actgtaataa gttattttta agctagctaa gtaatttatg 31080

agaaaaaaat aaattgttct taaattactt aatacccaag aggaagagtt tcagcttaaa 31140

ctagaccaaa ttaaaaaaat aataacagta aggcataact agatctagcc ttttctgaag 31200

aaaatgatat ttaaaacaag aaaaacatgt gccatagagg ttgtgatatc ctcaatcttt 31260

gaggaaataa cacctacatt taagtaccct caccttacac acactttctt cttctgattt 31320

tggagtcttt aaaatataag tatacttttt tttttttttt ttgagatagt ctcgctgtcg 31380

cccaggctgg agtgcagtgg cttgatcttg gttcactgca acatctgcct cccaggttca 31440

agcgattctc ctgcctcagt ctcctgggta gctgggacta caggcacgca ctattgccct 31500

aggctagttt tgtatttttg gtagagatgg ggtttcgcca ttttggccag gctgctctca 31560

aactcctgat ctcaggtgat ccacctgcct cggccttcca aagtgctggg attataggcg 31620

tgagccactg cacctggccc aaaatatgag tatactttaa atataaacat ataaaccatt 31680

catagaaact tttagatgga gacaaaatct gaaatatatt tcaatatata gttaatgtca 31740

tattaagggg ttaagggaag agctgggagg aagaaggaag ataagactat gaacctaaaa 31800

ttttgccaag tagtaggatt tttattctct aaaccttatt tcaatcaatt taaggttaaa 31860

aaaattttgc ttaaaaagat gcaatttcat tttgtgctta gaaagtaaaa ttaacaccac 31920

accaaagata aacattcttg agtttgttct tttggagaaa tgagtaattt aaaaatttaa 31980

acagctctaa aatattaata caactgcata ggaaagagga aagaatgtta gaaaattgct 32040

tatcaaaaac taatgagacc caggataaga gctactcata atgataacaa ttaaatattg 32100

agtatttttc agaaaccaag agtcaaatgc ttgttatacc ttaaaggtta aaggcccctg 32160

tttcatagtc attaaacagt tctttgaaat gctatgttaa gatatcacta acaatttcaa 32220

tttataaata cacacccata cacattttct tttctaaatt gtcatatccc aaacacgaaa 32280

acaatcgaat aagagaacag gctgaatagt gatttttttc tttcaattat tactactgaa 32340

acaaatcaaa acaactctga gaggttgcca gcaccgcaga aggcatttcc acataaaaag 32400

agttaatgag cagaccacgc agttcaacag ccagtgacca tttgcaagcc tcctaggtat 32460
attcccagca gacacattat cagattaaag gaagtgcaaa caaactgggc tgaaagtttc 32520
tttgtccaaa cccagcctct tcttcctgtg atgtcatatt acaaatctag gaagcctttc 32580
tttttcactt cagagaaagc gacctcaaga tacaactggc aactgaggaa aaggcctcaa 32640
ttcaacaaga gctaacaagc ttgggagttt atttcggaat ctttaaaaga ctcttctgct 32700
tacccacaat ctgggatcca ctgcaggaaa acaaaaaagg aaaacttcat ttaaaagaag 32760
caagaagtaa aatgggacaa attgggaatg tttaagtctc tgaaactctg cactgaaaag 32820
aaaataagat tgataactta agcttaacat tctgaggcat aaagaaacat taactttgga 32880
gtattcatct tgactactga aatacaagtt tagaagacaa gtggtttcat tctggtcaca 32940
gatcacagct tttctttaaa tttataatcc tatgggttgg actcgttgac tgtatttttt 33000
aaaggttgct cgtcagttaa ctgagccttg gaattcatgg attttctaaa gactaacaaa 33060
tgaaaatatt ttcctgttga agaacccagc ggaaatttta cagcaacaaa tttcatgttt 33120
cttttgggta tttctgagaa aaaggaaata tttataaaac catccaaaga tccagataat 33180
ttgcaaataa attggaggtt atagaggtta taatctgaat cccaaaggag actgcagctg 33240
atgaaagtgc ttccaaactg aaaattggac gtgcctttac gatggtaagc gttaacagct 33300
cccactgctt ctataatgac tcctttaagt acactttgta tgggtgcatg ttcagcatgg 33360
tgtttgtgct tgggttaata tccaattgtg ttgccatata cattttcatc tgcgtcctca 33420
aagtccgaaa tgaaactaca acttacatga ttaacttggc aatgtcagac ttgctttttg 33480
tttttacttt acccttcagg atttttactt tcacaacacg gaattggcca tttggagatt 33540
tactttgtaa gatttctgtg atgctgtttt ataccaacat gtacggaagc attctgttct 33600
taacctgtat tagtgtagat cgatttctgg caattgtcta cccatttaag tcaaagactc 33660
taagaaccaa aagaaatgca aagattgttt gcactggcgt gtggttaact gtgatcggag 33720
gaagtgcacc cgccgttttt gttcagtcta cccactctca gggtaacaat gcctcagaag 33780
cctgctttga aaattttcca gaagccacat ggaaaacata tctctcaagg attgtaattt 33840
tcatcgaaat agtgggattt tttattcctc taattttaaa tgtaacttgt tctagtatgg 33900
tgctaaaaac tttaaccaaa cctgttacat taagtagaag caaaataaac aaaactaagg 33960
ttttaaaaat gatttttgta catttgatca tattctgttt ctgttttgtt ccttacaata 34020
tcaatcttat tttatattct cttgtgagaa cacaaacatt tgttaattgc tcagtagtgg 34080
cagcagtaag gacaatgtac ccaatcactc tctgtattgc tgtttccaac tgttgttttg 34140
accctatagt ttactacttt acatcggaca caattcagaa ttcaataaaa atgaaaaact 34200
ggtctgtcag gagaagtgac ttcagattct ctgaagttca tggtgcagag aattttattc 34260
agcataacct acagacctta aaaagtaaga tatttgacaa tgaatctgct gcctgaaata 34320

aaaccattag gactcactgg gacagaactt tcaagttcct tcaactgtga aaagtgtctt 34380

tttggacaaa ctatttttcc acctccaaaa gaaattaaca catggacatt ttaaagtctt 34440

tagtataaag aaaatttgta ttcaatgtgt taagcattaa catgtatttt atttgtgtat 34500

ccactccatc tgatttttct gagccatttt gatttgttcc ttcattaaaa aaaatctctt 34560

aaagttattt agtgtctaaa agtgactgac ttaaattatg tggtgccaat ctgtaatgtc 34620

tttgaattcc tttttatatt aaatgattta atttactaaa ttattttata ctaatttatg 34680

tttatgtaat attttattca acataccatt tttattttga attaaaacta ctaatcatat 34740

ttcttaaact atgattaaca aatgagaaag aagagtaatt tgaaataaat gttttgggag 34800

tattttaaaa tatatgtata actaaaaacg tagtcttctg tagaaatgat gacatattaa 34860

cattgttgga agatcttgag cagtaacaca ttgataacat tttgggagtt tggtctgtgt 34920

aggcaatttg tagaaagcag atgctatcta tatggtatgc tttgaagtta ctattttgat 34980

tttctgtata taatgcaccc aatttcaaaa tgatatatgc cacaaaaatt gagagggaga 35040

aaactaataa aatgtcattt tgctaagttt ttacttagca aaaaaataaa atactaccag 35100

aggtacattt taaaggtgca cttacatcac agtcaagaac ttatgtttta agtatgttag 35160

gtcggagaaa catcagagat agtgaaatat agtacttggc atttagaaag taatatactg 35220

tcacgtgttg cttatgatgc ttgcttaggt aattatgtcg tgcaaacatc acagtgtgta 35280

cttacacaac tctagaaggt atacactatt acacacctag gcttatatgg tacaccctac 35340

tgcttctagg ctacaaacct atacagcatg ctactgcaaa gaatactgta ggcaactgta 35400

aaataatggt acttgtgtat ctaaatacat ctcaacacag aaaaggtaca gtaaaaatat 35460

agtatgataa tcttatgaga ccaccatcgt ttatgcggtc attgaccaaa atgctattac 35520

gtgatgcatg actgtaaata ttttcttttc cttcactctt ctggccattt taagccaaat 35580

attgggaagt tcaaaacatt atttcaagta ggcaagatgt tattgtacgt caaaatgatt 35640

aattaaggac acagttcct 35659

<210> 5
<211> 465
<212> DNA
<213> homo sapiens

<400> 5
atggtaagcg ttaacagctc ccactgcttc tataatgact cctttaagta cactttgtat 60

gggtgcatgt tcagcatggt gtttgtgctt gggttaatat ccaattgtgt tgccatatac 120

attttcatct gcgtcctcaa agtccgaaat gaaactacaa cttacatgat taacttggca 180

atgtcagact tgctttttgt ttttacttta cccttcagga ttttttactt cacaacacgg 240

aattggccat ttggagattt actttgtaag atttctgtga tgctgtttta taccaacatg 300

tacggaagca ttctgttctt aacctgtatt agtgtagatc gatttctggc aattgtctac 360

ccatttaagt caaagactct aagaaccaaa agaaatgcaa agattgtttg cactggcgtg 420

tggttaactg tgatcggagg aagtgcaccc gccgtttttg tttag 465

<210> 6
<211> 154
<212> PRT
<213> homo sapiens

<400> 6

Met Val Ser Val Asn Ser Ser His Cys Phe Tyr Asn Asp Ser Phe Lys
1               5                   10                  15

Tyr Thr Leu Tyr Gly Cys Met Phe Ser Met Val Phe Val Leu Gly Leu
            20                  25                  30

Ile Ser Asn Cys Val Ala Ile Tyr Ile Phe Ile Cys Val Leu Lys Val
        35                  40                  45

Arg Asn Glu Thr Thr Thr Tyr Met Ile Asn Leu Ala Met Ser Asp Leu
    50                  55                  60

Leu Phe Val Phe Thr Leu Pro Phe Arg Ile Phe Tyr Phe Thr Thr Arg
65                  70                  75                  80

Asn Trp Pro Phe Gly Asp Leu Leu Cys Lys Ile Ser Val Met Leu Phe
                85                  90                  95

Tyr Thr Asn Met Tyr Gly Ser Ile Leu Phe Leu Thr Cys Ile Ser Val
            100                 105                 110

Asp Arg Phe Leu Ala Ile Val Tyr Pro Phe Lys Ser Lys Thr Leu Arg
        115                 120                 125

Thr Lys Arg Asn Ala Lys Ile Val Cys Thr Gly Val Trp Leu Thr Val
    130                 135                 140

Ile Gly Gly Ser Ala Pro Ala Val Phe Val
145                 150

<210> 7
<211> 483
<212> DNA
<213> homo sapiens

<400> 7
atggtaagcg ttaacagctc ccactgcttc tataatgact cctttaagta cactttgtat 60

gggtgcatgt tcagcatggt gtttgtgctt gggttaatat ccaattgtgt tgccatatac 120

attttcatct gcgtcctcaa agtccgaaat gaaactacaa cttacatgat taacttggca 180

atgtcagact tgctttttgt ttttacttta cccttcagga ttttttactt cacaacacgg 240

aattggccat ttggagattt actttgtaag atttctgtga tgctgtttta taccaacatg 300

tacggaagca ttctgttctt aacctgtatt agtgtagatc gatttctggc aattgtctac 360

ccatttaagt cagactctaa gaaccaaaag aaatgcaaag attgtttgca ctggcgtgtg 420

gttaactgtg atcggaggaa gtgcacccgc cgtttttgtt cagtctaccc actctcaggg 480

taa 483

<210> 8
<211> 160
<212> PRT
<213> homo sapiens

<400> 8

Met Val Ser Val Asn Ser Ser His Cys Phe Tyr Asn Asp Ser Phe Lys
1               5                   10                  15

Tyr Thr Leu Tyr Gly Cys Met Phe Ser Met Val Phe Val Leu Gly Leu
            20                  25                  30

Ile Ser Asn Cys Val Ala Ile Tyr Ile Phe Ile Cys Val Leu Lys Val
        35                  40                  45

Arg Asn Glu Thr Thr Thr Tyr Met Ile Asn Leu Ala Met Ser Asp Leu
    50                  55                  60

Leu Phe Val Phe Thr Leu Pro Phe Arg Ile Phe Tyr Phe Thr Thr Arg
65                  70                  75                  80

Asn Trp Pro Phe Gly Asp Leu Leu Cys Lys Ile Ser Val Met Leu Phe
                85                  90                  95

Tyr Thr Asn Met Tyr Gly Ser Ile Leu Phe Leu Thr Cys Ile Ser Val
            100                 105                 110

Asp Arg Phe Leu Ala Ile Val Tyr Pro Phe Lys Ser Asp Ser Lys Asn
        115                 120                 125

Gln Lys Lys Cys Lys Asp Cys Leu His Trp Arg Val Val Asn Cys Asp
    130                 135                 140

Arg Arg Lys Cys Thr Arg Arg Phe Cys Ser Val Tyr Pro Leu Ser Gly
145                 150                 155                 160

<210> 9
<211> 20

<212>  DNA
<213>  Artificial sequence

<220>
<221>  source
<223>  /note="Description of artificial sequence:Primer"

<400>  9
agcagtggga gctgttaacg                                                    20


<210>  10
<211>  374
<212>  DNA
<213>  Artificial sequence

<220>
<221>  source
<223>  /note="Description of artificial sequence:DNA-Probe"

<400>  10
ttataccaac atgtacggaa gcattctgtt cttaacctgt attagtgtag atcgatttct      60

ggcaattgtc tacccattta agtcaaagac tctaagaacc aaaagaaatg caaagattgt     120

ttgcactggc gtgtggttaa ctgtgatcgg aggaagtgca cccgccgttt ttgttcagtc     180

tacccactct cagggtaaca atgcctcaga agcctgcttt gaaaattttc cagaagccac     240

atggaaaaca tatctctcaa ggattgtaat tttcatcgaa atagtgggat tttttattcc     300

tctaatttta aatgtaactt gttctagtat ggtgctaaaa actttaacca aacctgttac     360

attaagtaga agca                                                       374


<210>  11
<211>  23
<212>  DNA
<213>  Artificial sequence

<220>
<221>  source
<223>  /note="Description of artificial sequence:Primer"

<400>  11
tggaggttat agaggttata atc                                                23


<210>  12
<211>  22
<212>  DNA
<213>  Artificial sequence

<220>
<221>  source
<223>  /note="Description of artificial sequence:Primer"

<400>  12
ttataccaac atgtacggaa gc                                                 22


<210>  13
<211>  22

<212>  DNA
<213>  Artificial sequence

<220>
<221>  source
<223>  /note="Description of artificial sequence:Primer"

<400>  13
tgttaatttc ttttggaggt gg                                                22


<210>  14
<211>  22
<212>  DNA
<213>  Artificial sequence

<220>
<221>  source
<223>  /note="Description of artificial sequence:Primer"

<400>  14
ttgtgtgtct ttaagaccct cc                                                22


<210>  15
<211>  20
<212>  DNA
<213>  Artificial sequence

<220>
<221>  source
<223>  /note="Description of artificial sequence:Primer"

<400>  15
tcctgcctgg gccgcagagc                                                   20


<210>  16
<211>  23
<212>  DNA
<213>  Artificial sequence

<220>
<221>  source
<223>  /note="Description of artificial sequence:Primer"

<400>  16
aaatgatacc gatagactta tgg                                               23


<210>  17
<211>  24
<212>  DNA
<213>  Artificial sequence

<220>
<221>  source
<223>  /note="Description of artificial sequence:Primer"

<400>  17
tctttgatga cacctatgaa tagc                                              24


<210>  18

<211>  23
<212>  DNA
<213>  Artificial sequence

<220>
<221>  source
<223>  /note="Description of artificial sequence:Primer"

<400>  18
ttataggtgt gaaccacaga tcc                                              23


<210>  19
<211>  22
<212>  DNA
<213>  Artificial sequence

<220>
<221>  source
<223>  /note="Description of artificial sequence:Primer"

<400>  19
tgttgccata atgaccttgt ag                                               22


<210>  20
<211>  22
<212>  DNA
<213>  Artificial sequence

<220>
<221>  source
<223>  /note="Description of artificial sequence:Primer"

<400>  20
tggggtttct gtgtggaagt cc                                               22


<210>  21
<211>  24
<212>  DNA
<213>  Artificial sequence

<220>
<221>  source
<223>  /note="Description of artificial sequence:Primer"

<400>  21
tctctatttc caactgaggt accc                                             24


<210>  22
<211>  23
<212>  DNA
<213>  Artificial sequence

<220>
<221>  source
<223>  /note="Description of artificial sequence:Primer"

<400>  22
aatggataga tcactatttg ttg                                              23

<210> 23
<211> 21
<212> DNA
<213> Artificial sequence

<220>
<221> source
<223> /note="Description of artificial sequence:Primer"

<400> 23
aggaagtgca aacaaactgg g 21


<210> 24
<211> 20
<212> DNA
<213> Artificial sequence

<220>
<221> source
<223> /note="Description of artificial sequence:Primer"

<400> 24
agcagtggga gctgttaacg 20


<210> 25
<211> 23
<212> DNA
<213> Artificial sequence

<220>
<221> source
<223> /note="Description of artificial sequence:Primer"

<400> 25
aaaggaattc aaagacatta cag 23


<210> 26
<211> 20
<212> DNA
<213> Artificial sequence

<220>
<221> source
<223> /note="Description of artificial sequence:Primer"

<400> 26
cctaccattg acactctcag 20


<210> 27
<211> 16
<212> DNA
<213> Artificial sequence

<220>
<221> source
<223> /note="Description of artificial sequence:Primer"

<400> 27
tagggccatc cattct 16

<210> 28
<211> 20
<212> DNA
<213> Artificial sequence

<220>
<221> source
<223> /note="Description of artificial sequence:Primer"

<400> 28
acctgttgta tggcagcagt 20


<210> 29
<211> 20
<212> DNA
<213> Artificial sequence

<220>
<221> source
<223> /note="Description of artificial sequence:Primer"

<400> 29
ggttgactct ttccccaact 20


<210> 30
<211> 16
<212> DNA
<213> Artificial sequence

<220>
<221> source
<223> /note="Description of artificial sequence:Primer"

<400> 30
tgcaggtggg agtcaa 16


<210> 31
<211> 24
<212> DNA
<213> Artificial sequence

<220>
<221> source
<223> /note="Description of artificial sequence:Primer"

<400> 31
aaataacaag aagtgacctt ccta 24


<210> 32
<211> 16
<212> DNA
<213> Artificial sequence

<220>
<221> source
<223> /note="Description of artificial sequence:Primer"

<400> 32
ccaacatcgg gaactg 16

<210>  33
<211>  21
<212>  DNA
<213>  Artificial sequence

<220>
<221>  source
<223>  /note="Description of artificial sequence:Primer"

<400>  33
tgcattcttt aagtccatgt c                                                21


<210>  34
<211>  19
<212>  DNA
<213>  Artificial sequence

<220>
<221>  source
<223>  /note="Description of artificial sequence:Primer"

<400>  34
ttgaggacct gtcgttacg                                                   19


<210>  35
<211>  21
<212>  DNA
<213>  Artificial sequence

<220>
<221>  source
<223>  /note="Description of artificial sequence:Primer"

<400>  35
ttatagagca gttaaggcac a                                                21


<210>  36
<211>  19
<212>  DNA
<213>  Artificial sequence

<220>
<221>  source
<223>  /note="Description of artificial sequence:Primer"

<400>  36
cctgcatttg tgtacgtgt                                                   19


<210>  37
<211>  23
<212>  DNA
<213>  Artificial sequence

<220>
<221>  source
<223>  /note="Description of artificial sequence:Primer"

<400>  37

cagagccgtg gtagtatatt ttt 23

<210> 38
<211> 20
<212> DNA
<213> Artificial sequence

<220>
<221> source
<223> /note="Description of artificial sequence:Primer"

<220>
<221> misc_feature
<222> (8)..(8)
<223> n is a, c, g, or t

<400> 38
aggactanag atgaatgctc 20

<210> 39
<211> 20
<212> DNA
<213> Artificial sequence

<220>
<221> source
<223> /note="Description of artificial sequence:Primer"

<400> 39
gacatgactc catgtttggt 20

<210> 40
<211> 25
<212> DNA
<213> Artificial sequence

<220>
<221> source
<223> /note="Description of artificial sequence:Primer"

<400> 40
cctggcctgt tagtttttat tgtta 25

<210> 41
<211> 22
<212> DNA
<213> Artificial sequence

<220>
<221> source
<223> /note="Description of artificial sequence:Primer"

<400> 41
cccagtcttg ggtatgtttt ta 22

<210> 42
<211> 20
<212> DNA

<213>  Artificial sequence

<220>
<221>  source
<223>  /note="Description of artificial sequence:Primer"

<400>  42
cttggaaacc aacaagtcac                                                    20


<210>  43
<211>  19
<212>  DNA
<213>  Artificial sequence

<220>
<221>  source
<223>  /note="Description of artificial sequence:Primer"

<400>  43
attttgccac ctagaacgg                                                     19


<210>  44
<211>  21
<212>  DNA
<213>  Artificial sequence

<220>
<221>  source
<223>  /note="Description of artificial sequence:Primer"

<400>  44
acagtttttc gaggccatat c                                                  21


<210>  45
<211>  25
<212>  DNA
<213>  Artificial sequence

<220>
<221>  source
<223>  /note="Description of artificial sequence:Primer"

<400>  45
ggttaaataa atctccatcc agaag                                              25


<210>  46
<211>  20
<212>  DNA
<213>  Artificial sequence

<220>
<221>  source
<223>  /note="Description of artificial sequence:Primer"

<400>  46
tccagatagg cagattcaat                                                    20


<210>  47
<211>  20

<212>  DNA
<213>  Artificial sequence

<220>
<221>  source
<223>  /note="Description of artificial sequence:Primer"

<400>  47
ccttcttctt cccattgata                                                    20


<210>  48
<211>  16
<212>  DNA
<213>  Artificial sequence

<220>
<221>  source
<223>  /note="Description of artificial sequence:Primer"

<400>  48
tgcagaaatg tgagcc                                                        16


<210>  49
<211>  19
<212>  DNA
<213>  Artificial sequence

<220>
<221>  source
<223>  /note="Description of artificial sequence:Primer"

<400>  49
tccacctaga gcaactacc                                                     19


<210>  50
<211>  22
<212>  DNA
<213>  Artificial sequence

<220>
<221>  source
<223>  /note="Description of artificial sequence:Primer"

<400>  50
tggtttgttg cagaaagcac ac                                                 22


<210>  51
<211>  22
<212>  DNA
<213>  Artificial sequence

<220>
<221>  source
<223>  /note="Description of artificial sequence:Primer"

<400>  51
ttgggccttg tcaaccttca ta                                                 22


<210>  52

<211>  20
<212>  DNA
<213>  Artificial sequence

<220>
<221>  source
<223>  /note="Description of artificial sequence:Primer"

<400>  52
ggcaaagcct tgctcttaat                                                    20


<210>  53
<211>  20
<212>  DNA
<213>  Artificial sequence

<220>
<221>  source
<223>  /note="Description of artificial sequence:Primer"

<400>  53
ggctgtgctc ttccaaaata                                                    20


<210>  54
<211>  21
<212>  DNA
<213>  Artificial sequence

<220>
<221>  source
<223>  /note="Description of artificial sequence:Primer"

<400>  54
agggatcttc agagaaacag g                                                  21


<210>  55
<211>  21
<212>  DNA
<213>  Artificial sequence

<220>
<221>  source
<223>  /note="Description of artificial sequence:Primer"

<400>  55
tgacactatc agctctctgg c                                                  21


<210>  56
<211>  20
<212>  DNA
<213>  Artificial sequence

<220>
<221>  source
<223>  /note="Description of artificial sequence:Primer"

<400>  56
attagcccag gtatggtgac                                                    20

<210> 57
<211> 25
<212> DNA
<213> Artificial sequence

<220>
<221> source
<223> /note="Description of artificial sequence:Primer"

<400> 57
gctgtggtat gagttactta aacac 25


<210> 58
<211> 228
<212> DNA
<213> homo sapiens


<220>
<221> misc_feature
<222> (1)..(2)
<223> n is a, c, g, or t

<400> 58
nntttttttt tttttttcac agcaacaaat ttcatgtttc ttttgggtat ttctgagaaa 60

aaggaaatat ttataaaacc atccaaagat ccagataatt tgcaaataaa ttggaggtta 120

tagaggttat aatctgaatc ccaaaggaga ctgcagctga tgaaagtgct tccaaactga 180

aaattggacg tgcctttacg atggtaagcg ttaacagctc ccactgct 228


<210> 59
<211> 20
<212> DNA
<213> artificial feature

<220>
<221> source
<223> /note="Description of artificial sequence:Primer"

<400> 59
agcagtggga gctgttaacg 20


<210> 60
<211> 21
<212> DNA
<213> Artificial sequence

<220>
<221> source
<223> /note="Description of artificial sequence:Primer"

<400> 60
gaaccagtga gtttttatta c 21


<210> 61
<211> 21
<212> DNA
<213> Artificial sequence

<220>
<221> source
<223> /note="Description of artificial sequence:Primer"

<400> 61
agacacagca tataatacat g 21

<210> 62
<211> 20
<212> DNA
<213> Artificial sequence

<220>
<221> source
<223> /note="Description of artificial sequence:Primer"

<400> 62
agccacatcg ctcagacacc 20

<210> 63
<211> 21
<212> DNA
<213> Artificial sequence

<220>
<221> source
<223> /note="Description of artificial sequence:Primer"

<400> 63
tcctcttgtg ctcttgctgg g 21

<210> 64
<211> 20
<212> DNA
<213> Artificial sequence

<220>
<221> source
<223> /note="Description of artificial sequence:Primer"

<400> 64
tcttcccaga atatatggga 20

<210> 65
<211> 25
<212> DNA
<213> Artificial sequence

<220>
<221> source
<223> /note="Description of artificial sequence:Primer"

<400> 65
agctgtaaaa gtgtttgttt gatgt 25

<210> 66
<211> 20
<212> DNA

<213>  Artificial sequence

<220>
<221>  source
<223>  /note="Description of artificial sequence:Primer"

<400>  66
agcattgttt catgttggtg                                                    20


<210>  67
<211>  20
<212>  DNA
<213>  Artificial sequence

<220>
<221>  source
<223>  /note="Description of artificial sequence:Primer"

<400>  67
cagcagtgaa ggtctaagcc                                                    20


<210>  68
<211>  17
<212>  DNA
<213>  Artificial sequence

<220>
<221>  source
<223>  /note="Description of artificial sequence:Primer"

<400>  68
gtttcgccaa gcctgtt                                                       17


<210>  69
<211>  22
<212>  DNA
<213>  Artificial sequence

<220>
<221>  source
<223>  /note="Description of artificial sequence:Primer"

<400>  69
gttgacaata aaatacgcca ca                                                 22


<210>  70
<211>  16
<212>  DNA
<213>  Artificial sequence

<220>
<221>  source
<223>  /note="Description of artificial sequence:Primer"

<400>  70
gatggcacca ttgcac                                                        16


<210>  71
<211>  18

<212>  DNA
<213>  Artificial sequence

<220>
<221>  source
<223>  /note="Description of artificial sequence:Primer"

<400>  71
cagcacatcc aaacaagg                                                    18


<210>  72
<211>  22
<212>  DNA
<213>  Artificial sequence

<220>
<221>  source
<223>  /note="Description of artificial sequence:Primer"

<400>  72
ctgtgggatt ccttagtgat ac                                               22


<210>  73
<211>  23
<212>  DNA
<213>  Artificial sequence

<220>
<221>  source
<223>  /note="Description of artificial sequence:Primer"

<400>  73
atattcagac aaaagccaag tta                                              23


<210>  74
<211>  27
<212>  DNA
<213>  Artificial sequence

<220>
<221>  source
<223>  /note="Description of artificial sequence:Primer"

<400>  74
ctgggaaggc tttcttgctc acttcag                                          27


<210>  75
<211>  27
<212>  DNA
<213>  Artificial sequence

<220>
<221>  source
<223>  /note="Description of artificial sequence:Primer"

<400>  75
gttggatatc agcccaagca cgaagac                                          27


<210>  76

<211>  83
<212>  DNA
<213>  Artificial sequence

<220>
<221>  source
<223>  /note="Description of artificial sequence:Primer"

<400>  76
cgaagcttcc gccgccatgg gtaagcctat ccctaaccct ctcctcggtc tcgattctac      60

gatggtaagc gttaacagct ccc                                              83


<210>  77
<211>  29
<212>  DNA
<213>  Artificial sequence

<220>
<221>  source
<223>  /note="Description of artificial sequence:Primer"

<400>  77
gcctcgagtc aggcagcaga ttcattgtc                                        29


<210>  78
<211>  29
<212>  DNA
<213>  Artificial sequence

<220>
<221>  source
<223>  /note="Description of artificial sequence:Primer"

<400>  78
gcctcgagct aaacaaaaac ggcgggtgc                                        29


<210>  79
<211>  29
<212>  DNA
<213>  Artificial sequence

<220>
<221>  source
<223>  /note="Description of artificial sequence:Primer"

<400>  79
gcctcgagtt accctgagag tgggtagac                                        29


<210>  80
<211>  20
<212>  DNA
<213>  Artificial sequence

<220>
<221>  source
<223>  /note="Description of artificial sequence:Primer"

<400>  80
tggattagga gagcttctgg                                                  20

<210> 81
<211> 23
<212> DNA
<213> Artificial sequence

<220>
<221> source
<223> /note="Description of artificial sequence:Primer"

<400> 81
tggaagtcac atttcaccat ggc 23

<210> 82
<211> 22
<212> DNA
<213> Artificial sequence

<220>
<221> source
<223> /note="Description of artificial sequence:Primer"

<400> 82
tctaagggtt gtgaagatca cg 22

<210> 83
<211> 23
<212> DNA
<213> Artificial sequence

<220>
<221> source
<223> /note="Description of artificial sequence:Primer"

<400> 83
tcttcctcag tagaaacaac tgg 23

<210> 84
<211> 22
<212> DNA
<213> Artificial sequence

<220>
<221> source
<223> /note="Description of artificial sequence:Primer"

<400> 84
aagatacaac tggcaactga gg 22

<210> 85
<211> 20
<212> DNA
<213> Artificial sequence

<220>
<221> source
<223> /note="Description of artificial sequence:Primer"

<400> 85

agcagtggga gctgttaacg                                                    20

<210>  86
<211>  23
<212>  DNA
<213>  artificial feature

<220>
<221>  source
<223>  /note="Description of artificial sequence:Primer"

<400>  86
agcatgactt cgatctgatc agc                                               23

<210>  87
<211>  22
<212>  DNA
<213>  artificial sequence

<220>
<221>  source
<223>  /note="Description of artificial sequence:Primer"

<400>  87
tgagatagaa gtaggccaac cc                                                22

<210>  88
<211>  21
<212>  DNA
<213>  artificial sequence

<220>
<221>  source
<223>  /note="Description of artificial sequence:Primer"

<400>  88
attttcttct acgtgcggcg g                                                 21

<210>  89
<211>  22
<212>  DNA
<213>  artificial sequence

<220>
<221>  source
<223>  /note="Description of artificial sequence:Primer"

<400>  89
tgtatagtgg acagactcgc gg                                                22

<210>  90
<211>  21
<212>  DNA
<213>  artificial sequence

<220>
<221>  source
<223>  /note="Description of artificial sequence:Primer"

<400>  90
atctacgtgt acgtcaagag g                                                21

<210>  91
<211>  21
<212>  DNA
<213>  artificial sequence

<220>
<221>  source
<223>  /note="Description of artificial sequence:Primer"

<400>  91
ttgtaggagt agatgatggg g                                                21

<210>  92
<211>  22
<212>  DNA
<213>  artificial sequence

<220>
<221>  source
<223>  /note="Description of artificial sequence:Primer"

<400>  92
tcgtactatt aggactagga gg                                               22

<210>  93
<211>  22
<212>  DNA
<213>  artificial sequence

<220>
<221>  source
<223>  /note="Description of artificial sequence:Primer"

<400>  93
acaacagttc agagttgcaa gg                                               22

<210>  94
<211>  22
<212>  DNA
<213>  artificial sequence

<220>
<221>  source
<223>  /note="Description of artificial sequence:Primer"

<400>  94
tgtactactt tagcgccgag gg                                               22

<210>  95
<211>  22
<212>  DNA
<213>  artificial sequence

<220>
<221>  source
<223>  /note="Description of artificial sequence:Primer"

<400>  95
agaacgagag gcgttgggag tc                                                22

<210>  96
<211>  23
<212>  DNA
<213>  artificial sequence

<220>
<221>  source
<223>  /note="Description of artificial sequence:Primer"

<400>  96
tggaggttat agaggttata atc                                               23

<210>  97
<211>  22
<212>  DNA
<213>  artificial sequence

<220>
<221>  source
<223>  /note="Description of artificial sequence:Primer"

<400>  97
tgttaatttc ttttggaggt gg                                                22